(19) Europäisches Patentamt
European Patent Office
Office européen des brevets

(11) EP 3 247 772 B1

(12) EUROPÄISCHE PATENTSCHRIFT

(45) Veröffentlichungstag und Bekanntmachung des Hinweises auf die Patenterteilung:
**16.10.2019 Patentblatt 2019/42**

(21) Anmeldenummer: **16703733.2**

(22) Anmeldetag: **20.01.2016**

(51) Int Cl.:
*C09K 11/06* (2006.01) *H01L 51/00* (2006.01)
*C07D 401/14* (2006.01) *C07D 403/14* (2006.01)
*C07D 409/14* (2006.01) *C07D 417/14* (2006.01)
*C07D 209/86* (2006.01) *C07D 209/88* (2006.01)
*C07F 5/02* (2006.01)

(86) Internationale Anmeldenummer:
**PCT/EP2016/051170**

(87) Internationale Veröffentlichungsnummer:
**WO 2016/116528 (28.07.2016 Gazette 2016/30)**

(54) **SYMMETRISCHE ORGANISCHE MOLEKÜLE ZUR VERWENDUNG IN OPTOELEKTRONISCHEN BAUELEMENTEN**

SYMMETRICAL ORGANIC MOLECULES FOR USE IN OPTOELECTRONIC COMPONENTS

MOLÉCULES ORGANIQUES SYMÉTRIQUES DESTINÉES À ÊTRE UTILISÉES DANS DES COMPOSANTS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2015 EP 15151870**
**20.05.2015 EP 15168394**

(43) Veröffentlichungstag der Anmeldung:
**29.11.2017 Patentblatt 2017/48**

(73) Patentinhaber: **cynora GmbH**
**76646 Bruchsal (DE)**

(72) Erfinder:
- **AMBROSEK, David**
**10437 Berlin (DE)**
- **DANZ, Michael**
**76344 Eggenstein-Leopoldshafen (DE)**
- **FLÜGGE, Harald**
**76135 Karlsruhe (DE)**
- **FRIEDRICHS, Jana**
**76137 Karlsruhe (DE)**
- **GRAB, Tobias**
**76133 Karlsruhe (DE)**
- **JACOB, Andreas**
**30449 Hannover (DE)**
- **SEIFERMANN, Stefan**
**77815 Brühl (DE)**
- **VOLZ, Daniel**
**76137 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**EP-A1- 2 966 145**
**WO-A1-2005/040117**
**WO-A1-2005/090512**
**CN-A- 105 198 792**
**US-A1- 2002 045 061**

- **KUNDU P ET AL: "HIGH-TG CARBAZOLE DERIVATIVES AS BLUE-EMITTING HOLE-TRANSPORTING MATERIALS FOR ELECTROLUMINESCENT DEVICES", ADVANCED FUNCTIONAL MATERIALS, WILEY - V C H VERLAG GMBH & CO. KGAA, DE, Bd. 13, Nr. 6, 1. Juni 2003 (2003-06-01), Seiten 445-452, XP001162507, ISSN: 1616-301X, DOI: 10.1002/ADFM.200304308**
- **XUDONG WU ET AL: "Acid-Promoted Cross-Dehydrative Aromatization for the Synthesis of Tetraaryl-Substituted Pyrroles", ORGANIC LETTERS, Bd. 18, Nr. 1, 18. Dezember 2015 (2015-12-18), Seiten 56-59, XP055272749, US ISSN: 1523-7060, DOI: 10.1021/acs.orglett.5b03240**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- KATSUAKI SUZUKI ET AL: "Triarylboron-Based Fluorescent Organic Light-Emitting Diodes with External Quantum Efficiencies Exceeding 20?%", ANGEWANDTE CHEMIE INTERNATIONAL EDITION, Bd. 54, Nr. 50, 13. November 2015 (2015-11-13), Seiten 15231-15235, XP055272750, DE ISSN: 1433-7851, DOI: 10.1002/anie.201508270
- THOMAS J. PENFOLD: "On Predicting the Excited-State Properties of Thermally Activated Delayed Fluorescence Emitters", JOURNAL OF PHYSICAL CHEMISTRY C, Bd. 119, Nr. 24, 19. Mai 2015 (2015-05-19) , Seiten 13535-13544, XP055272753, US ISSN: 1932-7447, DOI: 10.1021/acs.jpcc.5b03530
- SHEN J-Y ET AL: "AMBIPOLAR CONDUCTIVE 2,7-CARBAZOLE DERIVATIVES FOR ELECTROLUMINESCENT DEVICES", ADVANCED FUNCTIONAL MATERIALS, WILEY - V C H VERLAG GMBH & CO. KGAA, DE, Nr. 17, 16. April 2007 (2007-04-16), Seiten 983-995, XP001509462, ISSN: 1616-301X, DOI: 10.1002/ADFM.200600921
- THOMAS K R J ET AL: "Light-Emitting Carbazole Derivatives: Potential Electroluminescent Materials", JOURNAL OF THE AMERICAN CHEMICAL SOCIETY, AMERICAN CHEMICAL SOCIETY, US, Bd. 123, Nr. 38, 28. August 2001 (2001-08-28), Seiten 9404-9411, XP002367047, ISSN: 0002-7863, DOI: 10.1021/JA010819S
- AUSRA TOMKEVICIENE ET AL: "Diphenylamino-substituted derivatives of 9-phenylcarbazole as glass-forming hole-transporting materials for solid state dye sensitized solar cells", SYNTHETIC METALS, Bd. 162, Nr. 23, 1. Dezember 2012 (2012-12-01), Seiten 1997-2004, XP055167587, ISSN: 0379-6779, DOI: 10.1016/j.synthmet.2012.10.002
- TOMAS LEIJTENS ET AL: "Hole Transport Materials with Low Glass Transition Temperatures and High Solubility for Application in Solid-State Dye-Sensitized Solar Cells", ACS NANO, Bd. 6, Nr. 2, 28. Februar 2012 (2012-02-28), Seiten 1455-1462, XP055136015, ISSN: 1936-0851, DOI: 10.1021/nn204296b
- A. TOMKEVICIENE ET AL: "Dimethyldiphenylamino-substituted carbazoles as electronically active molecular materials", DYES AND PIGMENTS, Bd. 96, Nr. 2, 1. Februar 2013 (2013-02-01), Seiten 574-580, XP055167480, ISSN: 0143-7208, DOI: 10.1016/j.dyepig.2012.09.021
- ZE BIAO TANG ET AL: "Synthesis and Optical Properties of D-A Type Organic Molecules Based on Fluorene and Carbazole", ADVANCED MATERIALS RESEARCH, Bd. 1061-1062, 1. Dezember 2014 (2014-12-01), Seiten 291-295, XP055272754, CH ISSN: 1022-6680, DOI: 10.4028/www.scientific.net/AMR.1061-1062.2 91
- KATSUYUKI SHIZU ET AL: "Strategy for Designing Electron Donors for Thermally Activated Delayed Fluorescence Emitters", JOURNAL OF PHYSICAL CHEMISTRY C, Bd. 119, Nr. 3, 22. Dezember 2014 (2014-12-22), Seiten 1291-1297, XP055272755, US ISSN: 1932-7447, DOI: 10.1021/jp511061t
- ZE BIAO TANG ET AL: "Synthesis of D-A Type Organic Molecules Based on Carbazole and Phenothiazine for Organic Light-Emitting Materials", ADVANCED MATERIALS RESEARCH, Bd. 1061-1062, 1. Dezember 2014 (2014-12-01), Seiten 307-310, XP055272756, CH ISSN: 1022-6680, DOI: 10.4028/www.scientific.net/AMR.1061-1062.3 07
- IBRAHIM F. SENGUL ET AL: "Some electrophilic reactivity studies of di-(2-indolyl)dibenzofurans and di-(2-indolyl)carbazoles", TETRAHEDRON, Bd. 70, Nr. 51, 13. November 2014 (2014-11-13), Seiten 9601-9614, XP055272757, AMSTERDAM, NL ISSN: 0040-4020, DOI: 10.1016/j.tet.2014.11.014
- ZE BIAO TANG ET AL: "Synthesis of a Novel D-A Type Molecule Based on Carbazole Unit for Organic Light-Emitting Material Applications", ADVANCED MATERIALS RESEARCH, Bd. 989-994, 1. Juli 2014 (2014-07-01), Seiten 292-296, XP055272758, DOI: 10.4028/www.scientific.net/AMR.989-994.292
- ZE BIAO TANG ET AL: "Synthesis of a Novel Donor Unit for Organic Light-Emitting Materials: 10-octyl-3,7-di(thiophen-2-Yl)-10H-Phenoth iazine", ADVANCED MATERIALS RESEARCH, Bd. 989-994, 1. Juli 2014 (2014-07-01), Seiten 284-287, XP055272759, DOI: 10.4028/www.scientific.net/AMR.989-994.284
- K.S.V. GUPTA ET AL: "Carbazole based A-[pi]-D-[pi]-A dyes with double electron acceptor for dye-sensitized solar cell", ORGANIC ELECTRONICS., Bd. 15, Nr. 1, 1. Januar 2014 (2014-01-01) , Seiten 266-275, XP055272761, NL ISSN: 1566-1199, DOI: 10.1016/j.orgel.2013.11.020

## Beschreibung

Die Erfindung betrifft rein organische Moleküle (Farbstoffe) aufweisend eine 3,6-funktionalisierte Carbazol-Donoreinheit und deren Verwendung als Emitter in OLEDs (organic light emitting diodes) und in anderen optoelektronischen Bauelementen.

## Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(I)-Komplexe verwendet werden, die aufgrund eines geringen Energieabstandes zwischen dem untersten Triplett-Zustand $T_1$ und dem darüberliegenden Singulett-Zustand $S_1$ ($\Delta E(S_1$-$T_1$) Triplett-Exitonen thermisch in einen Singulett-Zustand rückbesetzen können. Neben der Verwendung von Übergangsmetallkomplexen können auch rein organische Moleküle diesen Effekt ausnutzen.

Einige solcher TADF-Materialien wurden bereits in ersten optoelektronischen Bauelementen eingesetzt. Die bisherigen Lösungen weisen jedoch Nachteile und Probleme auf: Die TADF-Materialien weisen in den optoelektronischen Bauelementen oftmals keine ausreichende Langzeitstabilität, keine ausreichende thermische oder keine ausreichende chemische Stabilität gegenüber Wasser und Sauerstoff auf. Außerdem sind nicht alle wichtigen Emissionsfarben verfügbar. Weiterhin sind einige TADF-Materialien nicht verdampfbar und dadurch für den Einsatz in kommerziellen optoelektronischen Bauteilen nicht geeignet. Auch weisen einige TADF-Materialien keine passenden Energielagen zu den weiteren im optoelektronischen Bauteil verwendeten Materialien (z.B. HOMO-Energien von TADF-Emittern von größer gleich -5,9 eV) auf. Nicht mit allen TADF-Materialien lassen sich ausreichend hohe Effizienzen der optoelektronischen Bauelemente bei hohen Stromdichten bzw. hohe Leuchtdichten erreichen. Weiterhin sind die Synthesen einiger TADF-Materialien aufwendig.

Kundu et al., Adv. Funct. Mater., 2003, 13, No. 6, 445 offenbart Carbazolderivate, die als LochTransportmaterialien in optoelektronischen Vorrichtungen eingesetzt werden können.

## Beschreibung

Die Erfindung betrifft in einem Aspekt die Bereitstellung von organischen Molekülen,
wobei gilt:
Im Gesamtmolekül sind mindestens zwei chemische Einheiten AF1 und eine davon verschiedene chemische Einheit AF2 enthalten.

AF1 ist eine erste chemische Einheit, aufweisend ein konjugiertes System, insbesondere mindestens sechs in Konjugation stehende $\pi$-Elektronen (z. B. in Form mindestens eines aromatischen Systems), wobei AF1 durch die in Unterformel 1.1 dargestellte allgemeine Formel beschrieben wird;

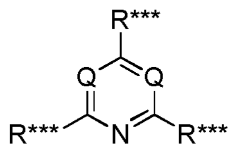

**Unterformel 1.1**

wobei gilt:

Q ist N, CR***, wobei nicht zwei direkt benachbarte Einheiten Q gleichzeitig gleich N sind;

und wobei gilt:

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist;

AF2 ist eine zweite chemische Einheit, aufweisend ein konjugiertes System, insbesondere mindestens sechs in Konjugation stehende $\pi$-Elektronen (z. B. in Form mindestens eines aromatischen Systems), wobei AF2 durch die in Unterformel 1 dargestellte allgemeine Formel beschrieben wird;

wobei gilt:

- die Differenz der Energie des HOMO der (Elektronen-donierend wirkenden) chemischen Einheit (AF2) und der Energie des HOMO der (Elektronen-akzeptierend wirkenden) chemischen Einheit (AF1) ist größer als 0,8 eV ($\Delta$ HOMO = HOMO(AF2) - HOMO(AF1) > 0,8 eV);
- die Differenz der Energie des LUMO der (donierenden) chemischen Einheit (AF2) und der Energie des LUMO der (akzeptierenden) chemischen Einheit (AF1) ist größer als 0,8 eV ($\Delta$ LUMO = LUMO(AF2) - LUMO(AF1) > 0,8 eV); und
- die Differenz der Energie des LUMO der (akzeptierenden) chemischen Einheit (AF1) und der Energie des HOMO der (donierenden) chemischen Einheit (AF2) ist größer als 0,9 eV ($\Delta$ Gap = LUMO (AF1) - HOMO(AF2) > 0,9 eV);

wobei die Energiewerte HOMO(AF1), HOMO(AF2), LUMO(AF1), LUMO(AF2) mithilfe der Dichtefunktionaltheorie (DFT) berechnet wurden, wobei die Anknüpfungspositionen der chemischen Einheiten AF1, AF2 und der Separatoren mit einem Wasserstoffatom entsprechend ihrer chemischen Valenzen abgesättigt werden; und wobei gilt: die angegebenen Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827);

Separator (S) ist eine chemische Einheit, die AF1 und AF2 verknüpft und deren elektronische Kommunikation über konjugierte Bindungen miteinander unterbricht; wobei optional in erfindungsgemäßen Molekülen kein Separator S enthalten ist;

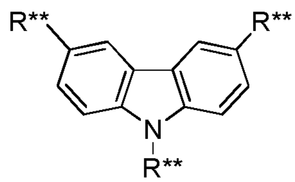

**Unterformel 1**

und wobei in Unterformel 1 bedeutet:
R** ist bei jedem Auftreten unabhängig voneinander entweder ein Rest R*, oder eine chemische Bindung an einen Separator S, oder eine chemische Bindung an einen Rest AF1; wobei die Anknüpfung an die chemische Einheit AF1 auch über ein Atom des Rests R* erfolgen kann;

wobei gilt:

$R^*$ ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^{9-}$, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^*$ auch miteinander ein monozyklisches aliphatisches Ringsystem mit insgesamt fünf oder sechs Ringgliedern bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^{9-}$, -C≡C-, bzw. eine benachbarte $CH_2$-Gruppe durch $-Si(R^4)_2-$, - $Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, - $As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^{9-}$, -C≡C-, bzw. eine benachbarte $CH_2$-Gruppe durch $-Si(R^4)_2-$, $-Ge(R^4)_2-$, $-Sn(R^4)_2$, -C(=O)-, - C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine

Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C{=}CR^9-$, -C≡C-, bzw. eine benachbarte $CH_2$-Gruppe durch $-Si(R^4)_2-$, $-Ge(R^4)_2-$, $-Sn(R^4)_2$, -C(=O)-, - C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C{=}CR^9-$, -C≡C-, bzw. eine benachbarte $CH_2$-Gruppe durch-$Si(R^4)_2-$, $-Ge(R^4)_2-$, $-Sn(R^4)_2$, -C(=O)-, - C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C{=}CR^{9-}$, -C≡C-, bzw. eine benachbarte $CH_2$-Gruppe durch $-Si(R^4)_2-$, - $Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, - $As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-, - C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, - $S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind insbesondere N, O, und S. Werden in der Beschreibung der vorliegenden Erfindung davon abweichende Angaben gemacht, beispielsweise bezüglich der Zahl der aromatischen Ringatome oder der enthaltenen Heteroatome, so gelten diese.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind insbesondere ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (insbesondere weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch Systeme wie 9,9'-Diarylfluoren, Triarylamin, Diarylether, Stilben etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen;

**Tabelle 1a:** Liste erfindungsgemäßer Einheiten AF1, wobei die möglichen Anknüpfungspositionen an den Separator und/oder AF2 mit Kleinbuchstaben gekennzeichnet sind.

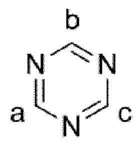

195

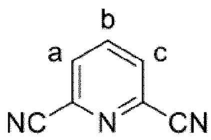

371

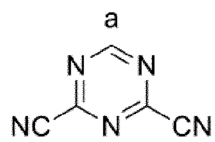

429

**Tabelle 1b:** Liste weiterer Einheiten AF1, wobei die möglichen Anknüpfungspositionen an den Separator und/oder AF2 mit Kleinbuchstaben gekennzeichnet sind.

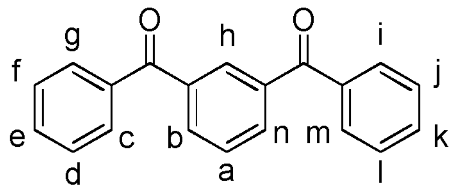

27

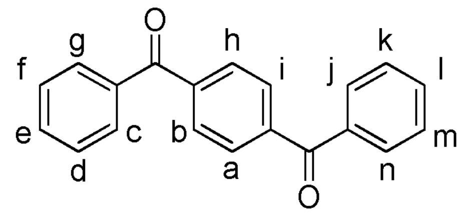

28

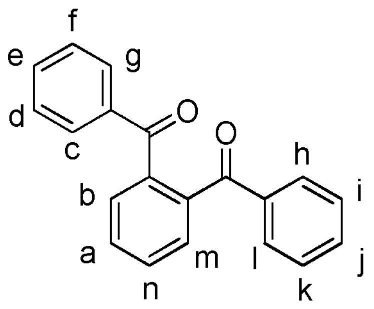

29

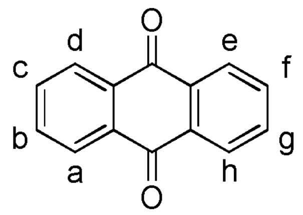

36

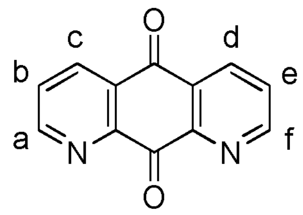

37

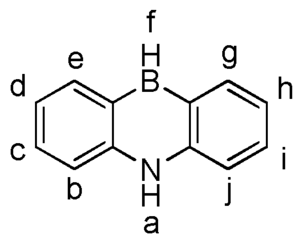

38

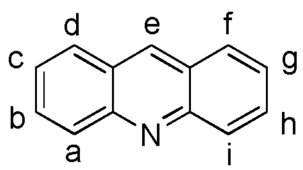

40

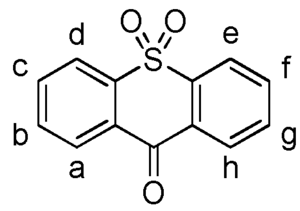

43

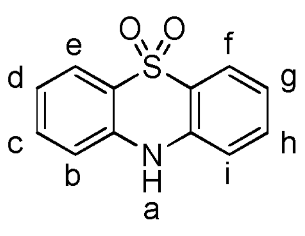

44

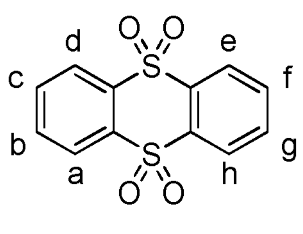

46

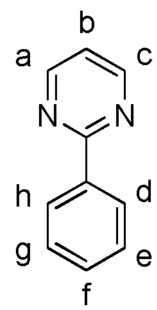

56

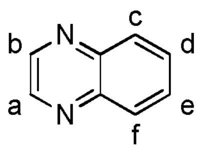

61

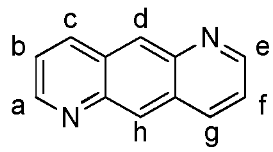

62

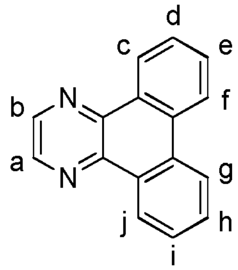

65

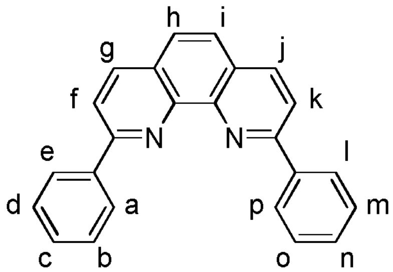

68

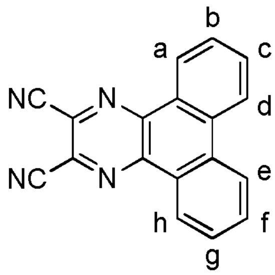

70

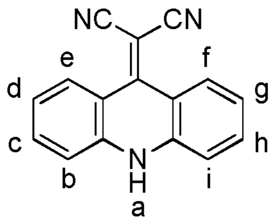

82

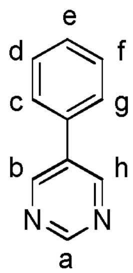

83

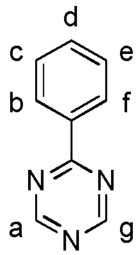

85

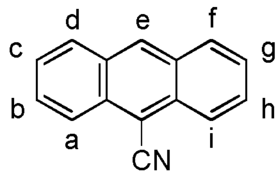

86

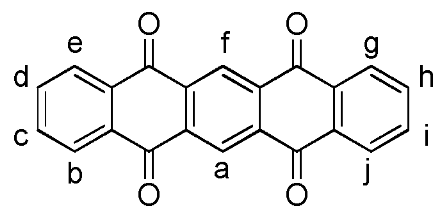

93

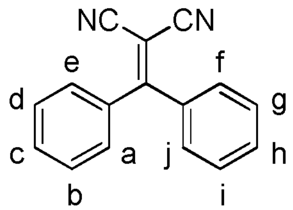

109

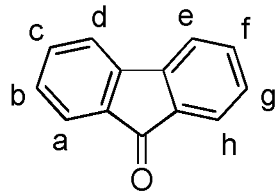

110

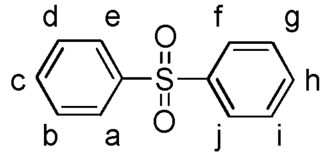

111

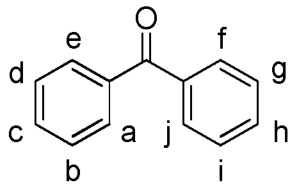

112

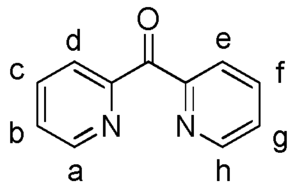

113

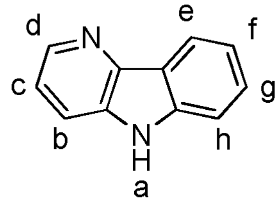

114

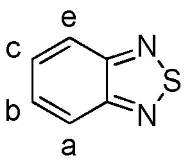

115

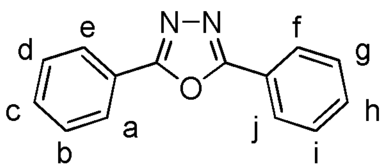

118

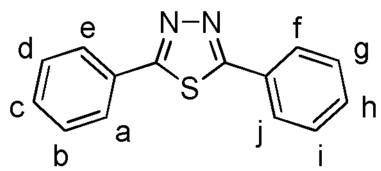

119

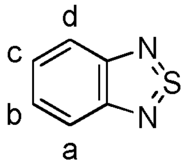

121

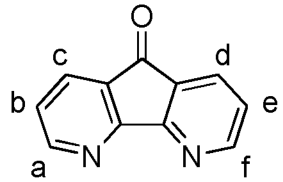

123

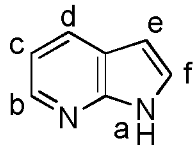

127

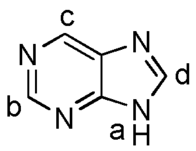

131

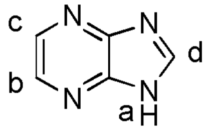

132

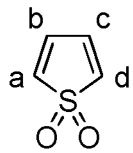

133

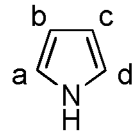

134

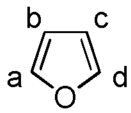

135

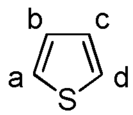

136

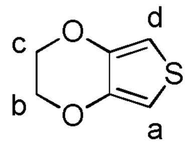

140

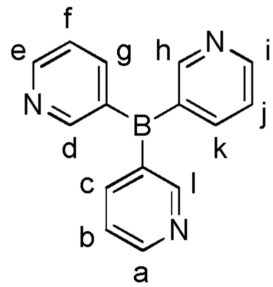

144

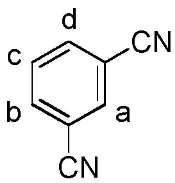

150

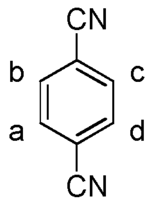

151

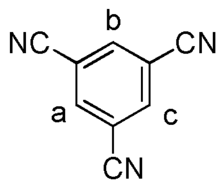

152

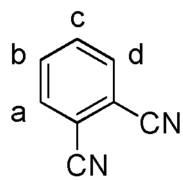

153

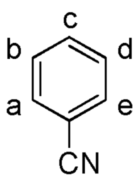

154

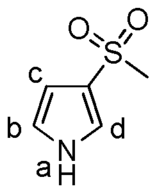

157

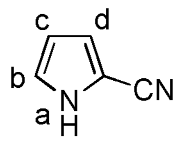

158

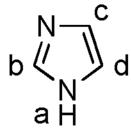

159

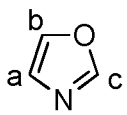

160

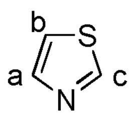

161

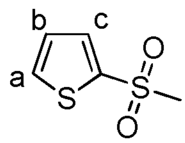

166

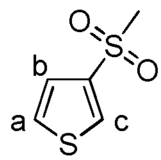

167

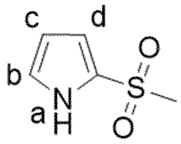
168

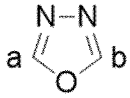
170

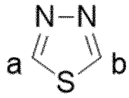
171

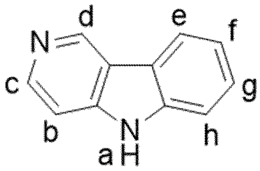
175

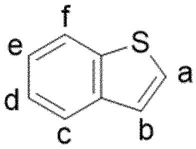
176

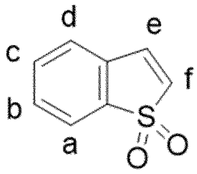
177

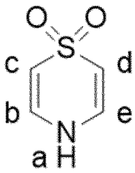
182

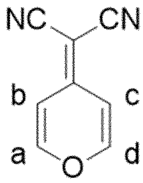
183

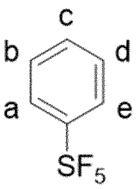
184

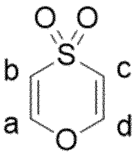
187

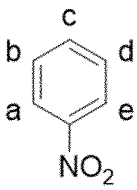
192

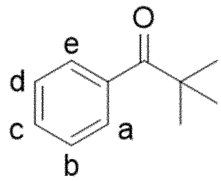
193

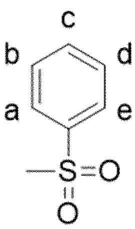
194

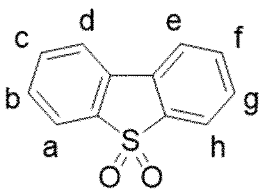
198

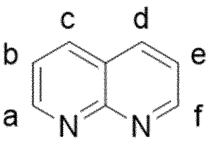
205

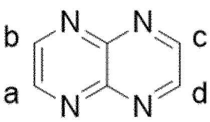
206

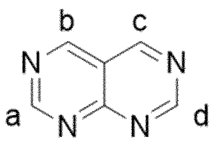
207

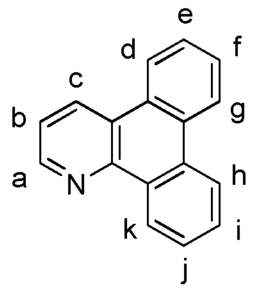

208

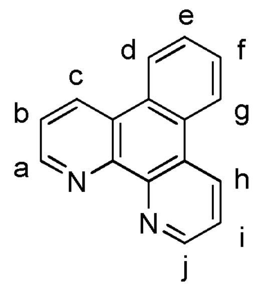

209

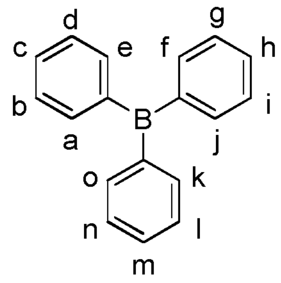

213

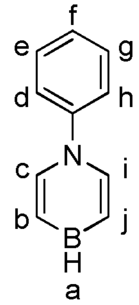

214

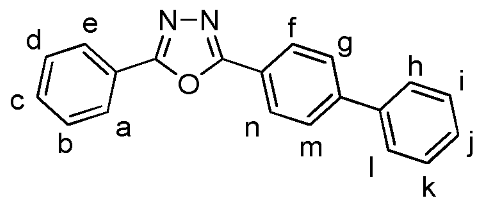

217

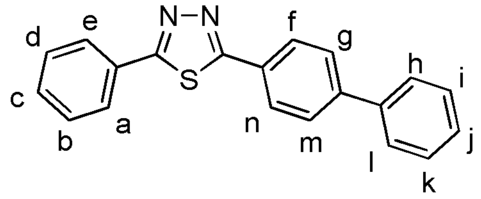

218

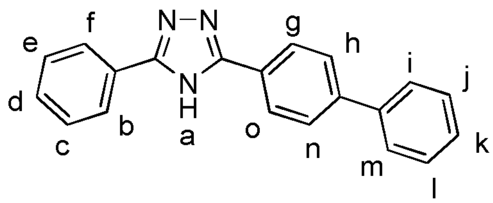

219

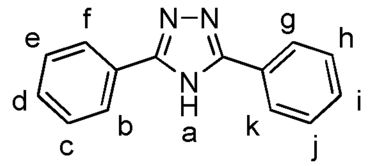

222

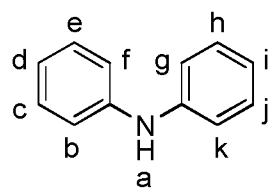

223

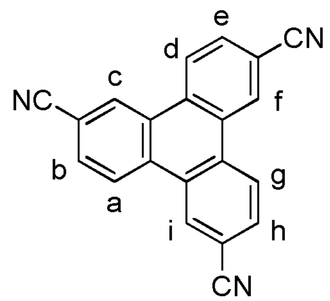

231

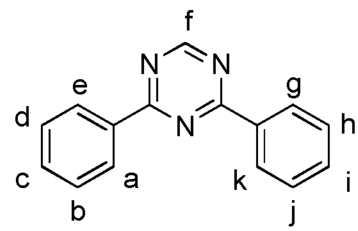

233

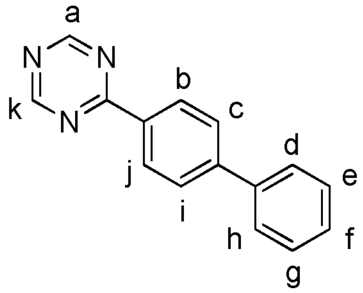

234

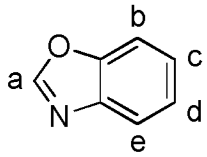

236

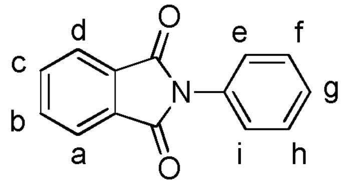

237

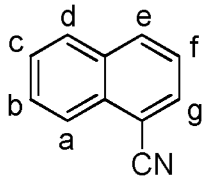

244

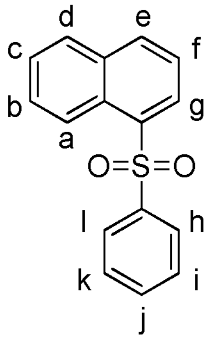

245

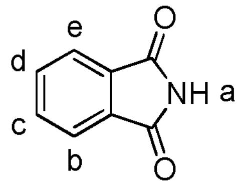

246

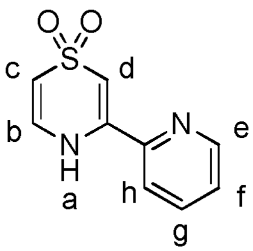

275

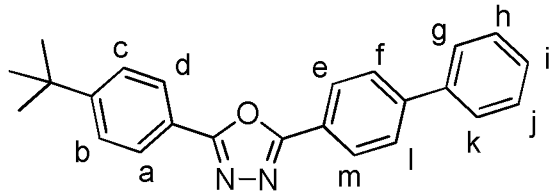

285

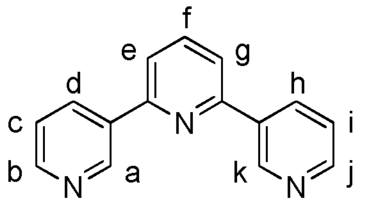

286

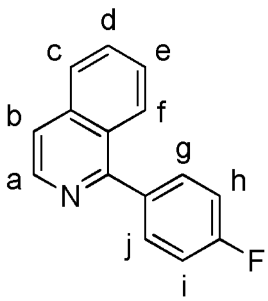

287

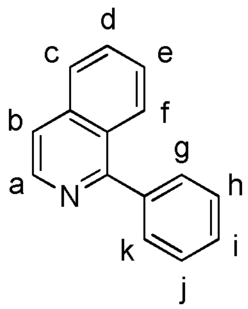

288

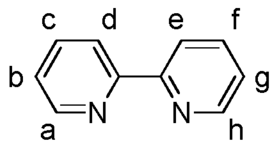

297

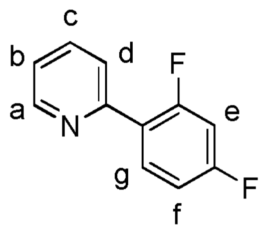

300

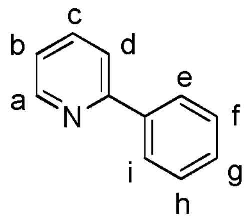

303

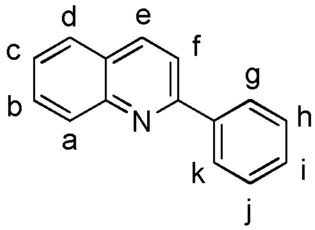

304

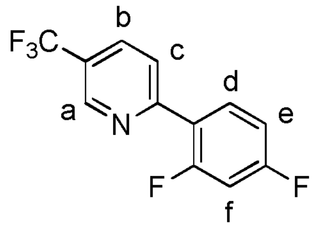

305

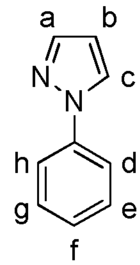

308

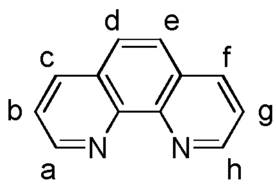

311

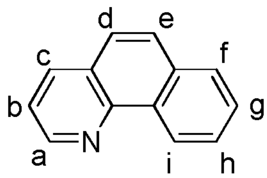

312

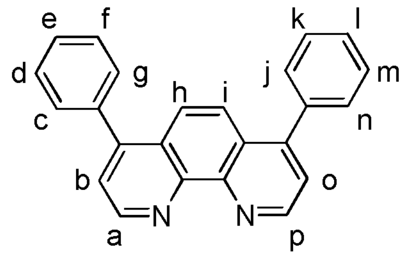

313

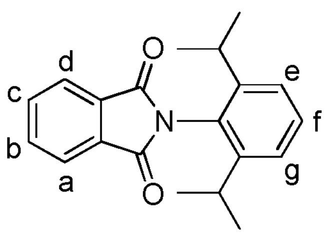

327

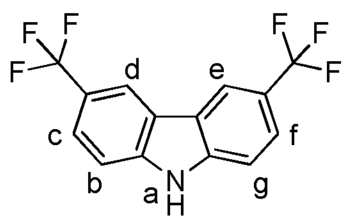

331

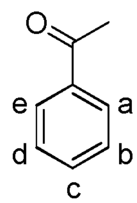

332

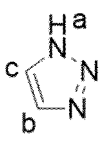

338

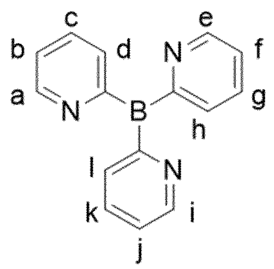

341

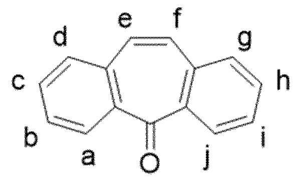

358

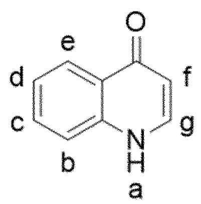

360

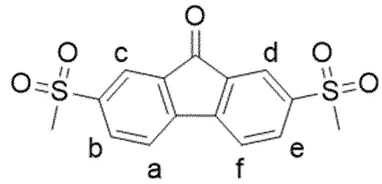

364

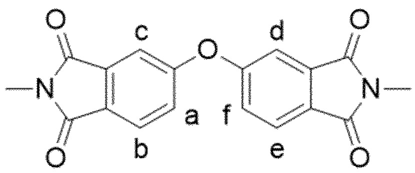

367

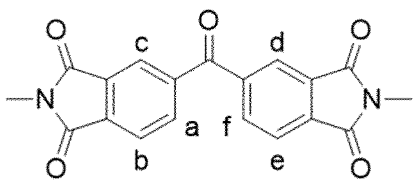

368

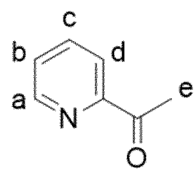

370

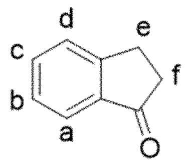

391

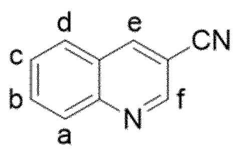

395

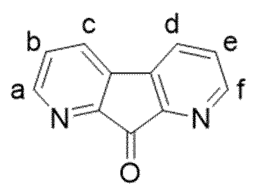

420

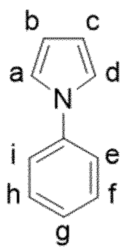

421

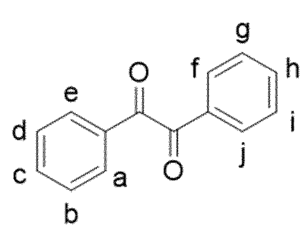

430

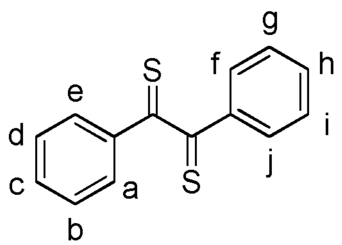

431

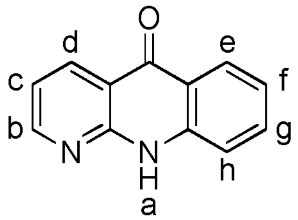

434

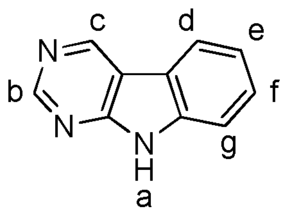

437

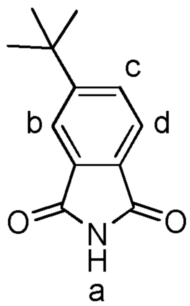

440

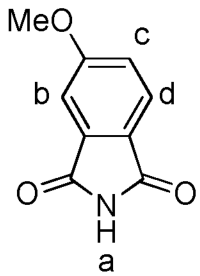

441

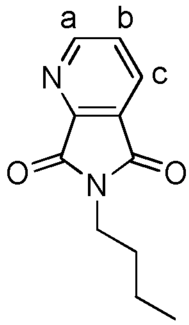

473

In einer Ausführungsform weist die chemische Einheit AF2 im erfindungsgemäßen organischen Molekül eine Struktur der Formel 2a-2d auf oder hat eine Struktur der Formel 2a-2d.

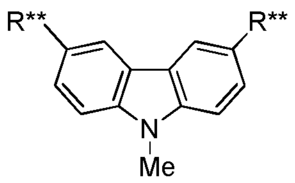

**Formel 2a**

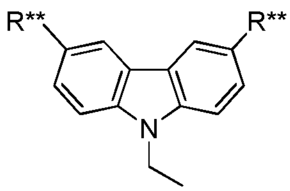

**Formel 2b**

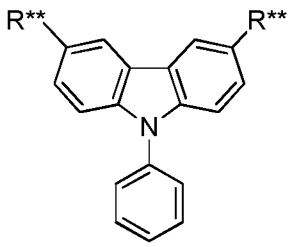

**Formel 2c**

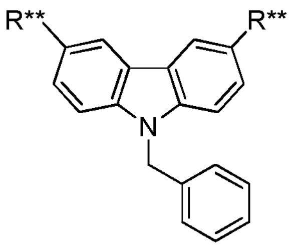

**Formel 2d**

und wobei in den einzelnen Formeln 2a bis 2d jeweils beide R** eine chemische Bindung an AF1 oder eine chemische Bindung an einen Separator S sind;

In einer Ausführungsform der erfindungsgemäßen organischen Moleküle sind die chemischen Einheiten AF2 ausgewählt aus den in Tabelle 2 aufgeführten Strukturen und die chemischen Einheiten AF1 ausgewählt aus den in Tabelle 1a dargestellten Strukturen; wobei die kovalente Anbindung der Einheiten AF1 an den Separator S oder an eine Einheit AF2 über je eine der mit Kleinbuchstaben bezeichneten Positionen (s. Tabelle 1a) erfolgt. Tabelle 1b: Nicht-erfindungsgemäße Einheiten AF1.

**Tabelle 2:** Chemische Einheiten AF2, wobei die kovalente Anbindung der Einheiten AF2 an den Separator S und/oder an eine Einheit AF1 über je eine der mit Kleinbuchstaben bezeichneten Positionen erfolgt.

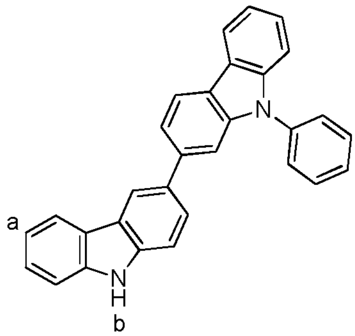

1

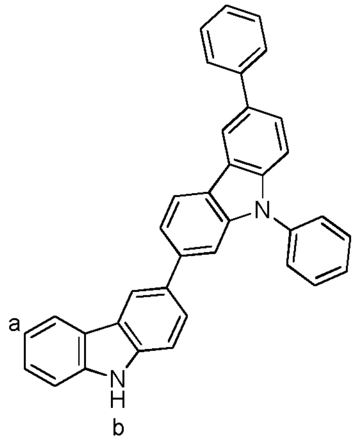

2

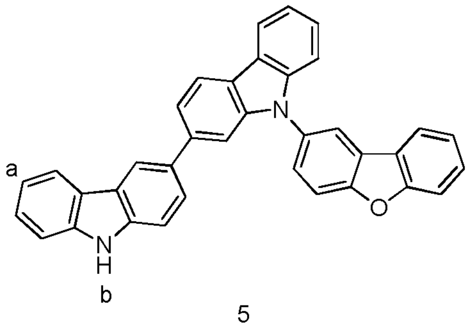

5

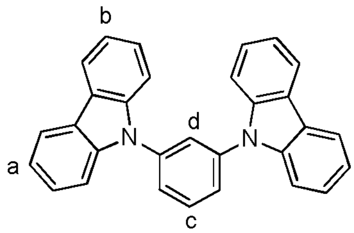

14

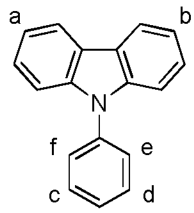

55

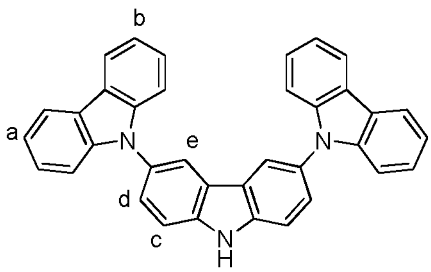

74

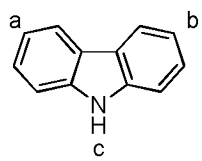

328

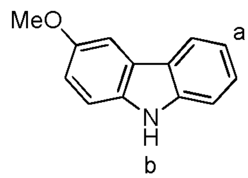

436

In einer weiteren Ausführungsform weisen die erfindungsgemäßen Moleküle einen Separator S der Formel 4 auf oder haben eine Struktur der Formel 4,

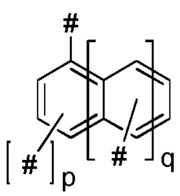

**Formel 4**

wobei die Anknüpfungspositionen für die chemischen Einheiten AF1 und AF2 durch den Platzhalter # gekennzeichnet sind.
p = 0 oder 1,
q = 0 oder 1,
und wobei p ≠ q.

In einer weiteren Ausführungsform weisen die erfindungsgemäßen Moleküle einen Separator S auf, der ausgewählt ist aus den in Tabelle 3 gezeigten Strukturen; wobei die Anknüpfungspositionen für die chemischen Einheiten AF1 und AF2 durch den Platzhalter # gekennzeichnet sind.

In einer Ausführungsform sind die chemischen Einheiten AF1 und AF2 derart miteinander verknüpft, dass die elektronische Kommunikation zwischen ihnen unterbrochen wird. Diese Unterbrechung ist durch eine Lokalisierung der Grenzorbitale HOMO und LUMO auf separaten Molekülteilen gekennzeichnet, sodass ein Charge-Transfer Übergang ermöglicht wird.

In einer Ausführungsform sind die chemischen Einheiten AF1 und AF2 derart über einen Separator miteinander verbunden, dass die elektronische Kommunikation zwischen ihnen unterbrochen wird, was anhand der Lokalisierung der Grenzorbitale HOMO und LUMO auf separaten Molekülteilen gekennzeichnet ist. Der Separator kann dabei eine beliebige Struktur aufweisen, solange eine Unterbrechung der elektronischen Kommunikation gewährleistet wird.

Von Vorteil ist, wenn der Separator die elektronische Kommunikation von AF1 und AF2 über konjugierte Bindungen miteinander unterbricht. Unterbrechung der elektronischen Kommunikation über konjugierte Bindungen bedeutet dabei, dass die Orbitale HOMO und LUMO weit genug voneinander entfernt sind, sodass keine signifikante Überlappung stattfinden kann, wobei ein maximaler Überlapp der Orbitale von 20% des Gesamtvolumens der Orbitale erlaubt ist. Wird dieser Wert überschritten, so wird von einer signifikanten Überlappung gesprochen. Eine Bestimmung und Quantifizierung der Unterbrechung bzw. des Überlapps kann beispielsweise durch quantenchemische Standardverfahren wie DFT-Rechnungen erfolgen, in dem der Orbitalüberlapp der Wellenfunktionen HOMO und LUMO bestimmt wird. Die elektronische Kommunikation zwischen den zwei chemischen Einheiten AF1 und AF2 über konjugierte Bindungen mit einem optionalen Separator ist unterbrochen, wenn die Grenzorbitale HOMO und LUMO auf separaten Molekülteilen lokalisiert sind, sodass ein Charge-Transfer Übergang ermöglicht wird. Die Lokalisierung der Grenzorbitale HOMO oder LUMO wird dabei mithilfe der Dichtefunktionaltheorie (DFT) mit dem BP86-Funktional (Becke, A. D. Phys. Rev. A1988, 38, 3098-3100; Perdew, J. P. Phys. Rev. B1986, 33, 8822-8827) visualisiert: Aus der Einelektronen-Wellenfunktion wird die Einelektronendichte durch Quadrieren berechnet und über den Raum integriert, den der untersuchte Molekülteil einnimmt. Dieser Raum kann aus den Atomkoordinaten und den van-der-Waals-Radien der Atome bestimmt werden. Die resultierende Zahl entspricht dem Anteil des Orbitals auf dem Molekülteil. Eine mehrheitliche Trennung der Grenzorbitale entspricht dabei einem Überlappungsparameter O im Bereich 0.1-20%, um einen Charge-Transfer Übergang zu ermöglichen. Der Überlappungsparameter O zwischen der HOMO-Wellenfunktion $\phi_a$ und der der LUMO-Wellenfunktion $\phi_b$ ergibt sich aus dem Integral über den gesamten Raum über den jeweils kleineren Wert der quadrierten Wellenfunktion:

$$O = \int \rho \; d\tau \text{ mit } \rho = \begin{cases} \varphi_a^2, & \text{wenn } |\varphi_a| < |\varphi_b| \\ \varphi_b^2, & \text{wenn } |\varphi_a| \geq |\varphi_b| \end{cases}$$

**Tabelle 3:** Beispiele für Separatoren S

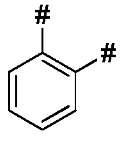
S-7

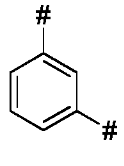
S-8

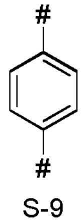
S-9

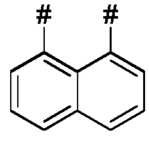
S-10

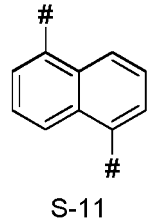
S-11

In einer weiteren Ausführungsform weisen die erfindungsgemäßen Moleküle eine Struktur der Formel 5 auf oder haben eine Struktur der Formel 5,

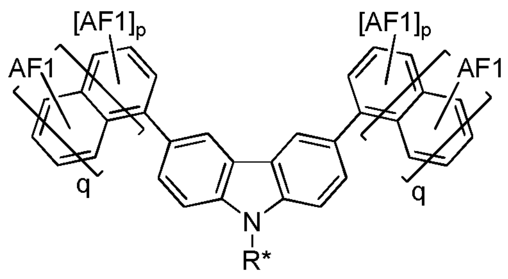

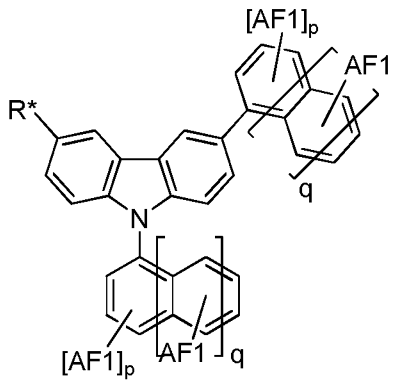

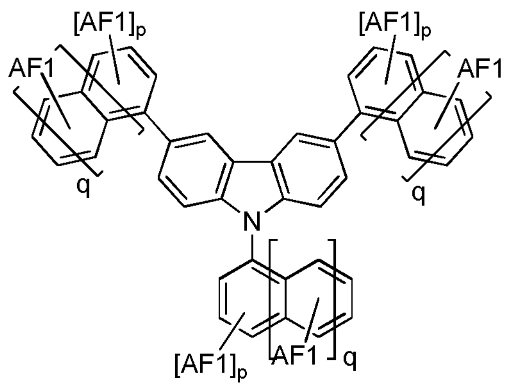

**Formel 5**

wobei

p = 0 oder 1,
q = 0 oder 1,
und wobei p ≠ q;
und wobei im Übrigen die für Unterformel 1 gegebenen Definitionen gelten.

In einer weiteren Ausführungsform weisen die erfindungsgemäßen Moleküle eine Struktur der Formel 6 auf oder haben eine Struktur der Formel 6,

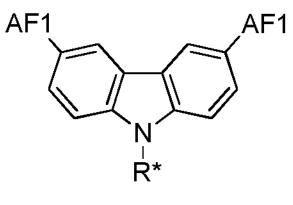

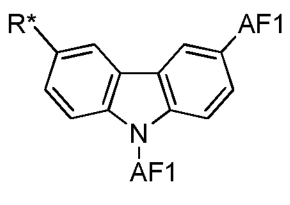

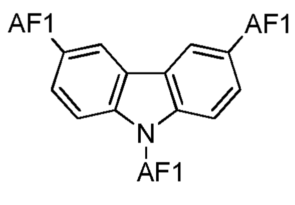

**Formel 6**

wobei im Übrigen die für Unterformel 1 gegebenen Definitionen gelten.

In einer weiteren Ausführungsform weist die Einheit AF1 eine Struktur der Unterformeln 1.2 bis 1.10 auf oder hat eine Struktur der Unterformeln 1.2 bis 1.10

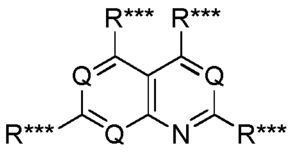

**Unterformel 1.2**

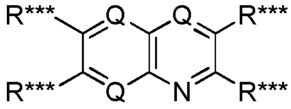

**Unterformel 1.3**

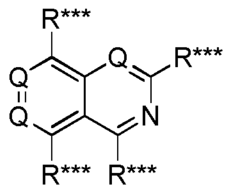

**Unterformel 1.4**

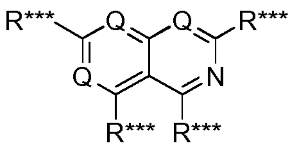

**Unterformel 1.5**

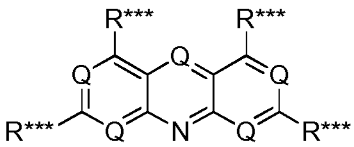

**Unterformel 1.6**

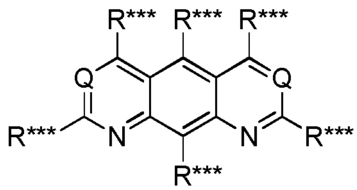

**Unterformel 1.7**

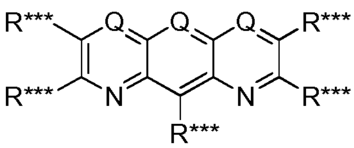

**Unterformel 1.8**

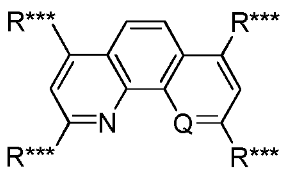

**Unterformel 1.9**

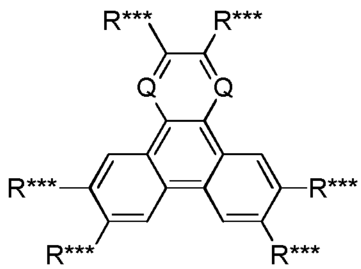

**Unterformel 1.10**

und wobei gilt:

Q ist N, CR***, wobei nicht zwei direkt benachbarte Einheiten Q gleichzeitig gleich N sind;

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist.

In einer weiteren Ausführungsform weist die Einheit AF1 eine Struktur der Unterformeln 1.1 bis 1.13 auf oder hat eine Struktur der Unterformeln 1.11 bis 1.13

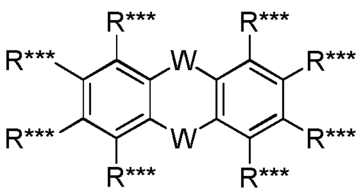

**Unterformel 1.11**

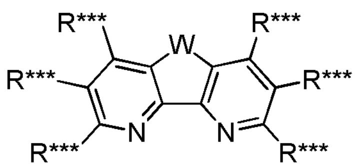

**Unterformel 1.12**

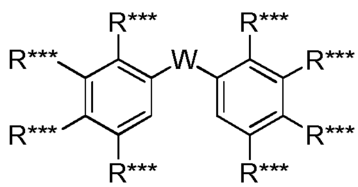

**Unterformel 1.13**

und wobei gilt:

W ist

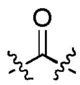, 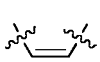, 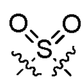,

eine Element-Element-Einfachbindung, wobei nicht zwei Einheiten W gleichzeitig eine Element-Element-Einfachbindung sind, NR***, wobei nicht zwei Einheiten W gleichzeitig gleich NR*** sind;

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist.

In einer weiteren Ausführungsform weist die Einheit AF1 eine Struktur der Unterformel 1.14 auf oder hat eine Struktur der Unterformel 1.14

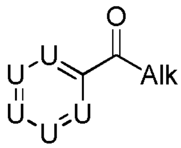

**Unterformel 1.14**

und wobei gilt:

U ist CR***, N, wobei maximal 3 Einheiten U gleichzeitig gleich N sind und wobei keine benachbarten Einheiten U gleichzeitig gleich N sind;

Alk ist Methyl, Ethyl, Propyl *iso*-Propyl, Butyl, tert-Butyl, Pentyl, Hexyl, 2-Ethylhexyl, Cyclohexyl, Heptyl, Octyl, Nonyl, Decyl, Undecyl oder Dodecyl;

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist.

In einer weiteren Ausführungsform weist die Einheit AF1 eine Struktur der Unterformeln 1.15 bis 1.22 auf oder hat eine Struktur der Unterformeln 1.15 bis 1.22

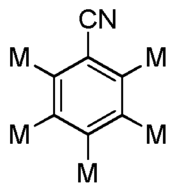

**Unterformel 1.15**

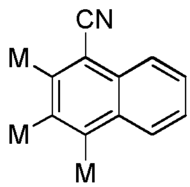

**Unterformel 1.16**

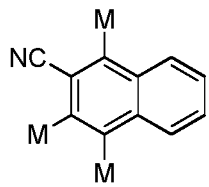

**Unterformel 1.17**

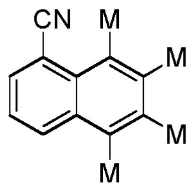

**Unterformel 1.18**

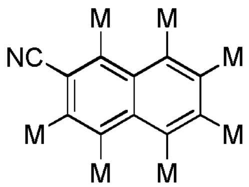

**Unterformel 1.19**

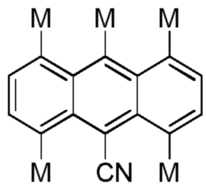

**Unterformel 1.20**

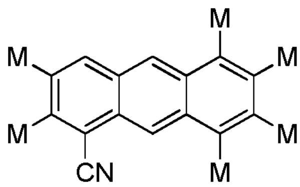

**Unterformel 1.21**

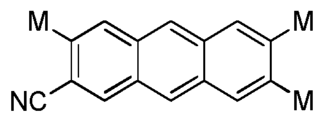

**Unterformel 1.22**

und wobei gilt:
M ist H, Deuterium, Alk, Phenyl, Pyridyl, CN, wobei maximal 4 Einheiten M gleichzeitig gleich CN sind, oder kennzeichnet eine chemische Bindung an einen Separator S oder an einen Rest AF2, wobei genau ein M eine chemische Bindung an einen Separator S oder an einen Rest AF2 kennzeichnet.

In einer weiteren Ausführungsform weist die Einheit AF1 eine Struktur der Unterformeln 1.23 bis 1.24 auf oder hat eine Struktur der Unterformeln 1.23 bis 1.24

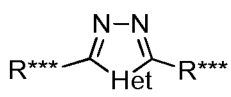

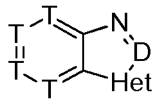

**Unterformel 1.23** **Unterformel 1.24**

und wobei gilt: Het ist NR***, O, S, $SO_2$;

D ist N, CR***;

T ist N, CR***, wobei maximal 2 Einheiten T gleich N sind, und wobei keine benachbarten Einheiten T gleichzeitig gleich N sind;

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist.

In einer weiteren Ausführungsform weist die Einheit AF1 eine Struktur der Unterformeln 1.25 bis 1.36 auf oder hat eine Struktur der Unterformeln 1.25 bis 1.36

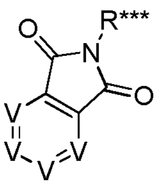

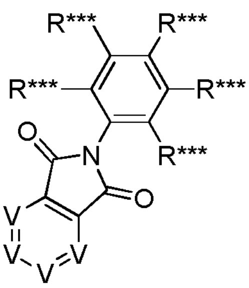

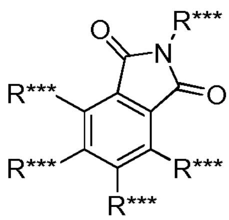

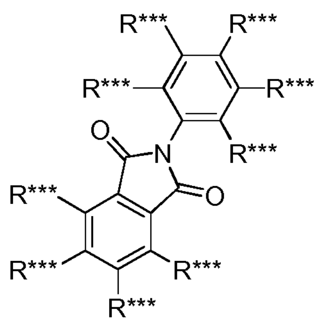

**Unterformel 1.25** **Unterformel 1.26** **Unterformel 1.27** **Unterformel 1.28**

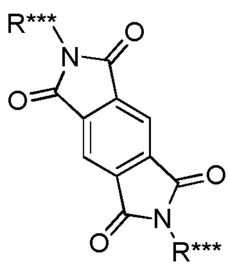

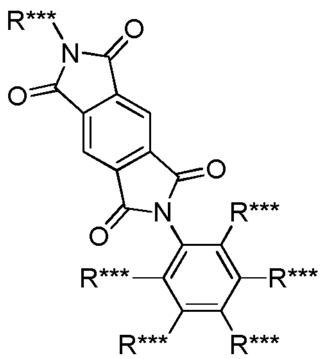

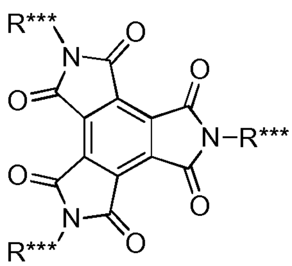

**Unterformel 1.29** **Unterformel 1.30** **Unterformel 1.31**

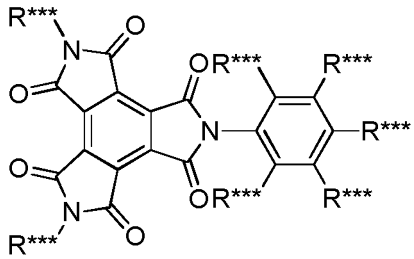

**Unterformel 1.32**

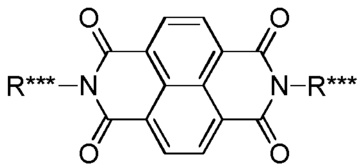

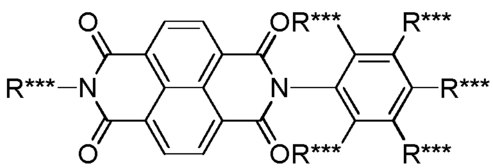

**Unterformel 1.35** **Unterformel 1.36**

und wobei gilt: V ist bei jedem Auftreten CR***, N, wobei mindestens eine Einheit V gleich N ist; und wobei maximal zwei Einheiten V gleichzeitig gleich N sind; und wobei nicht zwei einander benachbarte Einheiten V gleichzeitig gleich N sind;

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist.

In einer weiteren Ausführungsform weisen die beschriebenen Moleküle eine Struktur der Formel 7 auf oder haben eine Struktur der Formel 7,

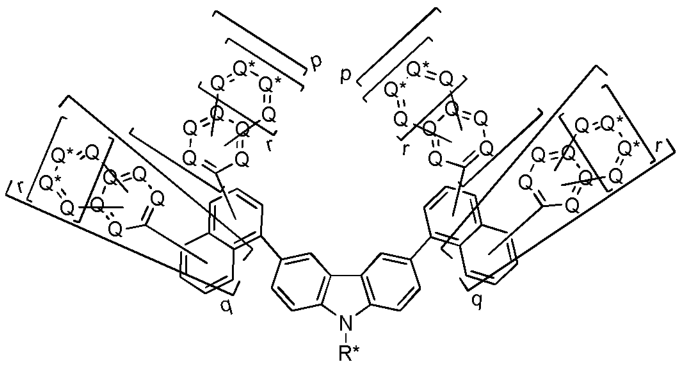

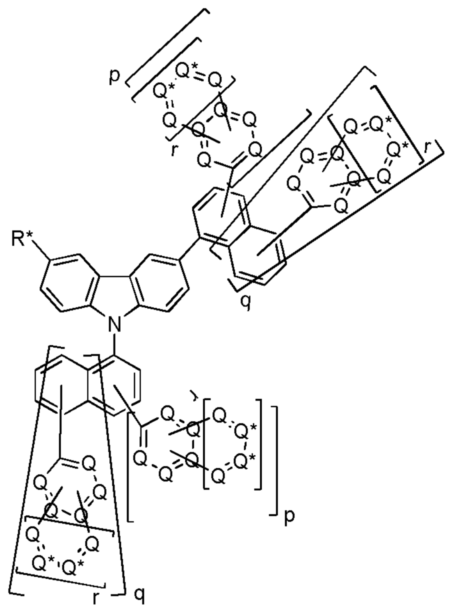

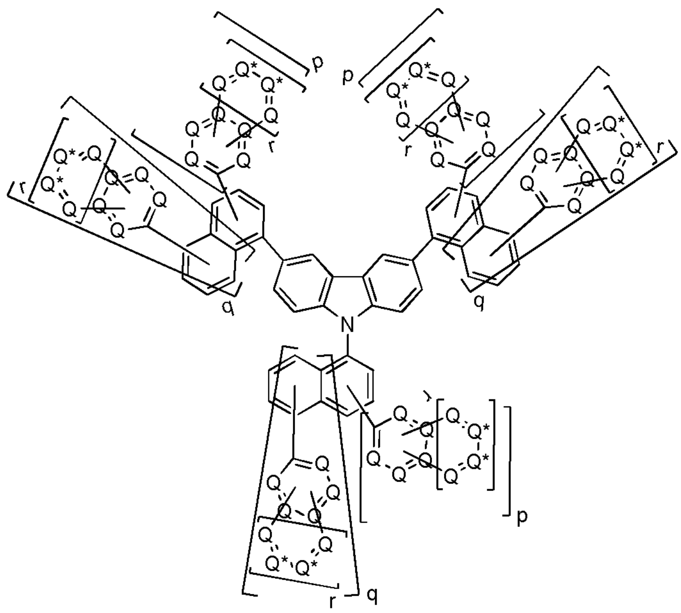

**Formel 7**

wobei Q ist N, CR*, C,

Q* ist N, CR*,

wobei mindestens ein Q oder Q* pro Molekül/Einheit ein N ist, und wobei nicht zwei direkt benachbarte Einheiten Q oder Q* gleichzeitig gleich N sind;

p = 0 oder 1,

q = 0 oder 1,
r = 0 oder 1,
und wobei p ≠ q.
wobei im Übrigen die für Unterformel 1 gegebenen Definitionen gelten.

In einer weiteren Ausführungsform weisen die beschriebenen Moleküle eine Struktur der Formel 8 auf oder haben eine Struktur der Formel 8,

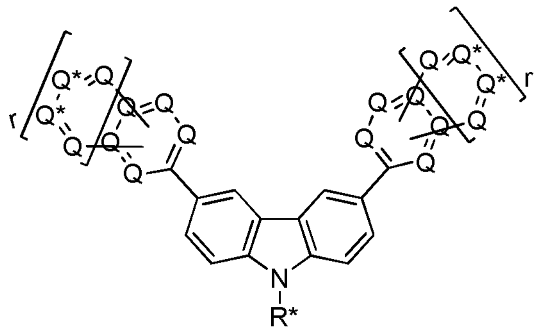

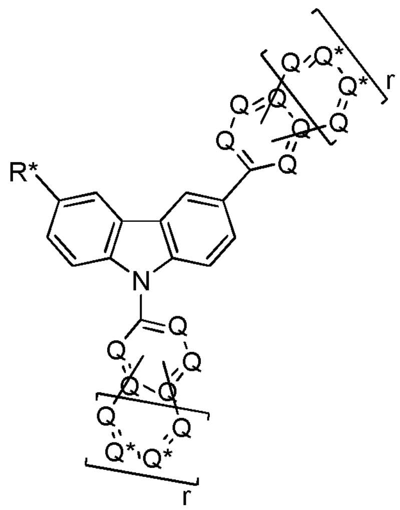

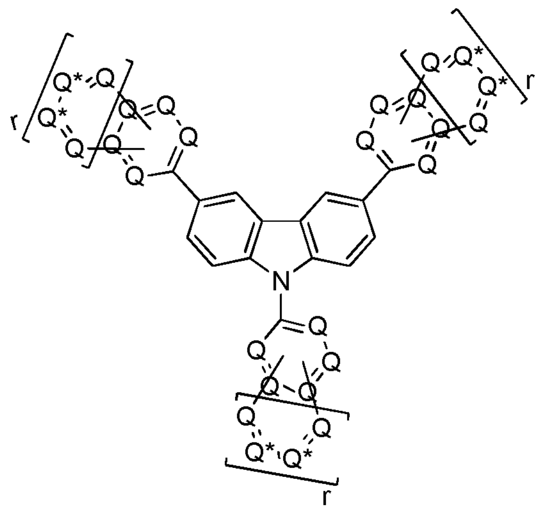

**Formel 8**

wobei Q ist N, CR*, C,

Q* ist N, CR*,

wobei mindestens ein Q oder Q* pro Molekül/Einheit ein N ist, und wobei nicht zwei direkt benachbarte Einheiten Q oder Q* gleichzeitig gleich N sind;

r = 0 oder 1,
wobei im Übrigen die für Unterformel 1 gegebenen Definitionen gelten.

In einer weiteren Ausführungsform weisen die beschriebenen Moleküle eine Struktur der Formel 9 auf oder haben eine Struktur der Formel 9,

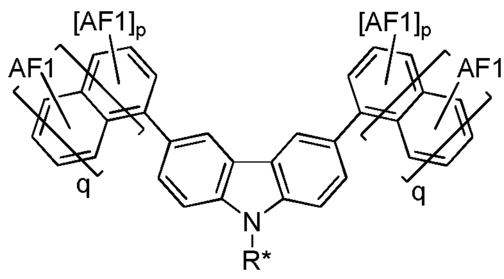

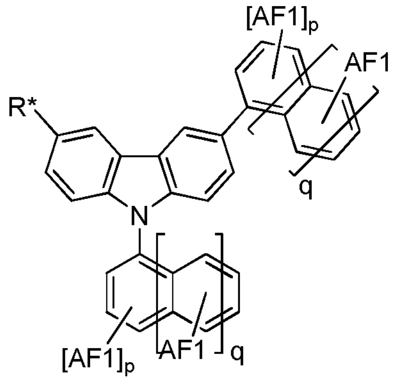

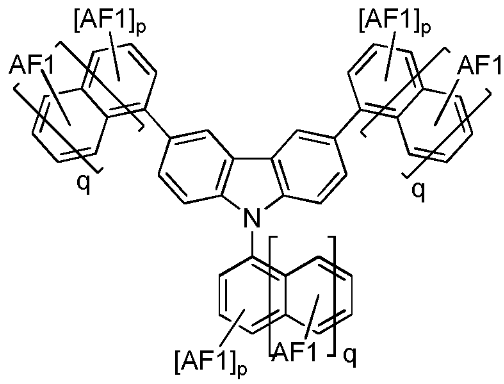

**Formel 9**

wobei

p = 0 oder 1,
q = 0 oder 1,
und wobei $p \neq q$,
AF1 eine Struktur der Formel 9b aufweist;

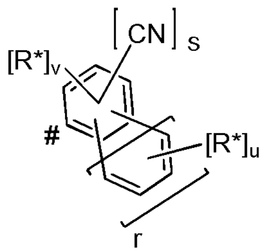

**Formel 9b**

wobei die Anknüpfungsposition der chemischen Einheit AF1 an den jeweiligen Separator durch den Platzhalter # gekennzeichnet ist,
r = 0 oder 1,

s = 1 bis 5, für r = 1 ist s = 1 bis 3,

u ist 1, 2, 3 oder 4

v ist 1, 2 oder 3; wobei gilt: u + v = 3 + 4r - q

und wobei im Übrigen die für Unterformel 1 gegebenen Definitionen gelten.

In einer weiteren Ausführungsform weisen die beschriebenen Moleküle eine Struktur der Formel 10 auf oder haben eine Struktur der Formel 10,

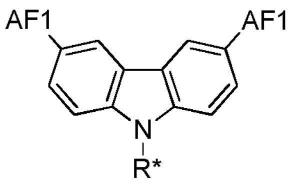 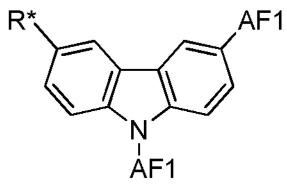 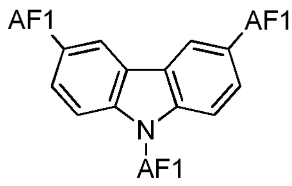

**Formel 10**

wobei
AF1 eine Struktur der Formel 9b aufweist;

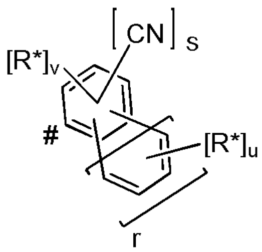

**Formel 9b**

wobei die Anknüpfungsposition der chemischen Einheit AF1 an die chemische Einheit AF2 durch den Platzhalter # gekennzeichnet ist,
r = 0 oder 1,

s = 1 bis 5, für r = 1 ist s = 1 bis 3,

u ist 1, 2, 3 oder 4

v ist 1, 2 oder 3; wobei gilt: u + v = 3 + 4r - q

und wobei im Übrigen die für Unterformel 1 gegebenen Definitionen gelten.

In einer weiteren Ausführungsform weisen die beschriebenen Moleküle eine Struktur der Formel 11 auf oder haben eine Struktur der Formel 11

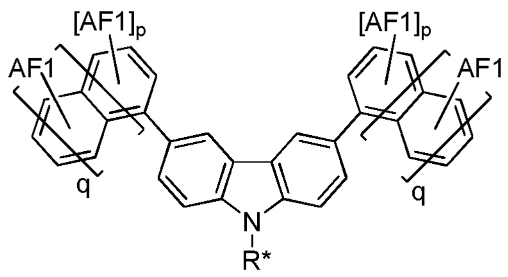

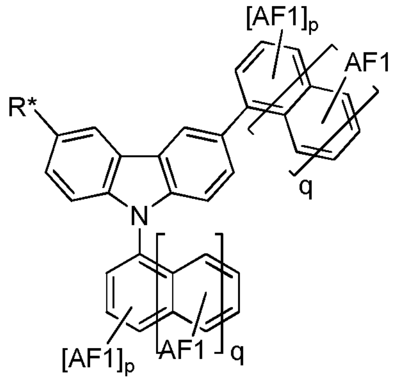

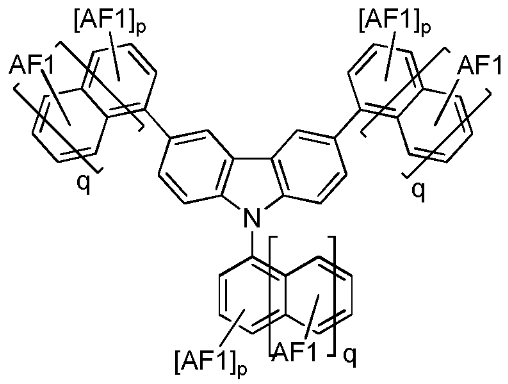

**Formel 11**

wobei

p = 0 oder 1,
q = 0 oder 1,
und wobei p ≠ q,
AF1 eine Struktur der Formel 11b aufweist;

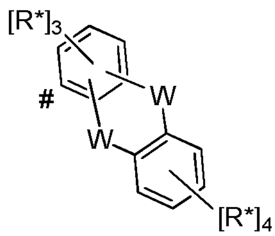

**Formel 11b**

wobei die Anknüpfungsposition der chemischen Einheit AF1 an den jeweiligen Separator durch den Platzhalter # gekennzeichnet ist,

W ist ausgewählt aus der Gruppe bestehend aus

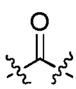, 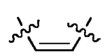, 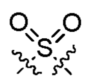,

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;

und wobei im Übrigen die für Unterformel 1 gegebenen Definitionen gelten.

In einer weiteren Ausführungsform weisen die beschriebenen Moleküle eine Struktur der Formel 12 auf oder haben eine Struktur der Formel 12,

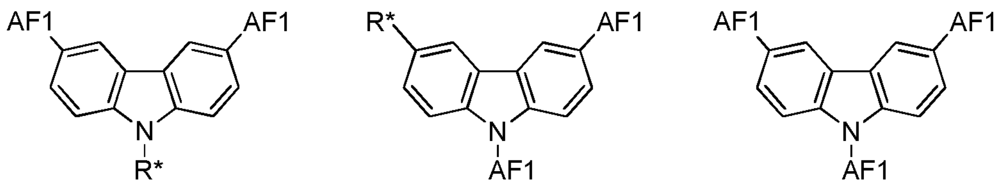

**Formel 12**

wobei
AF1 eine Struktur der Formel 11b aufweist;

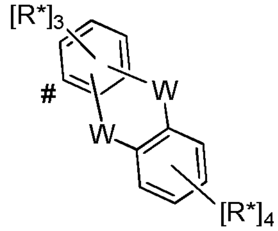

**Formel 11b**

wobei die Anknüpfungsposition der chemischen Einheit AF1 an die chemische Einheit AF2 durch den Platzhalter # gekennzeichnet ist,

W ist ausgewählt aus der Gruppe bestehend aus

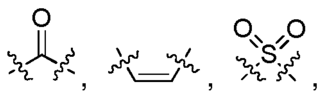

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;

und wobei im Übrigen die für Unterformel 1 gegebenen Definitionen gelten.

In einer weiteren Ausführungsform weisen die beschriebenen Moleküle eine Struktur der Formel 13 auf oder haben eine Struktur der Formel 13,

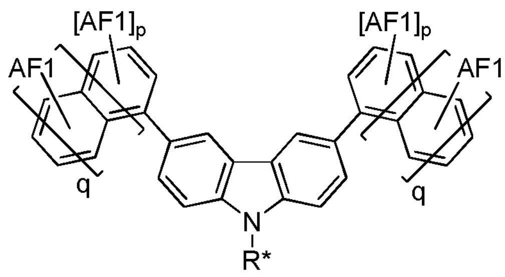

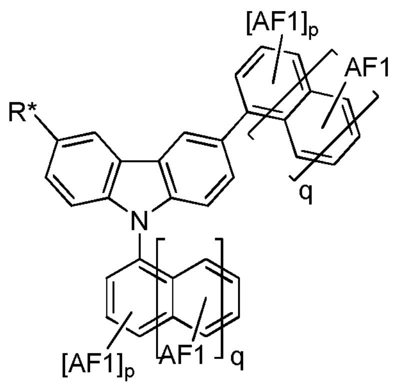

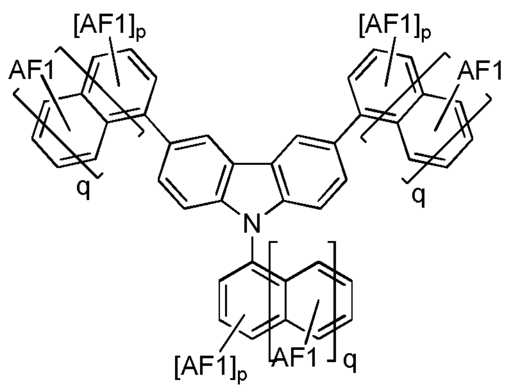

**Formel 13**

wobei

p = 0 oder 1,
q = 0 oder 1,
und wobei p ≠ q,
AF1 eine Struktur der Formel 13b aufweist;

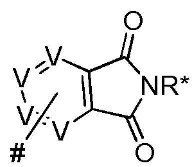

**Formel 13b**

wobei

V ist bei jedem Auftreten unabhängig voneinander CR* oder N, wobei maximal zwei Einheiten V gleichzeitig gleich N sind; und wobei nicht zwei einander benachbarte Einheiten V gleichzeitig gleich N sind, und wobei ein V gleich C ist, worüber die Anbindung an den jeweiligen Separator erfolgt;

wobei die Anknüpfungsposition der chemischen Einheit AF1 an den jeweiligen Separator durch den Platzhalter # gekennzeichnet ist,
und wobei im Übrigen die für Unterformel 1 gegebenen Definitionen gelten.

In einer weiteren Ausführungsform weisen die beschriebenen Moleküle eine Struktur der Formel 14 auf oder haben eine Struktur der Formel 14,

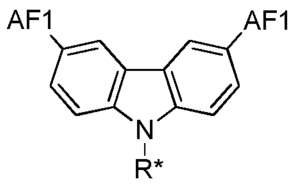
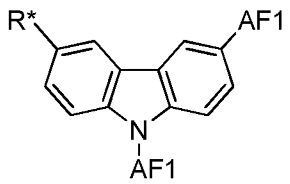
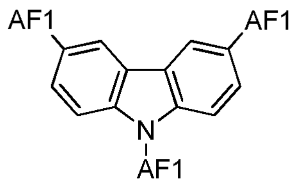

**Formel 14**

wobei

AF1 eine Struktur der Formel 13b aufweist;

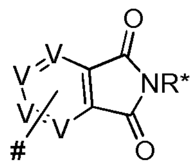

**Formel 13b**

wobei

V ist bei jedem Auftreten unabhängig voneinander CR* oder N, wobei maximal zwei Einheiten V gleichzeitig gleich N sind; und wobei nicht zwei einander benachbarte Einheiten V gleichzeitig gleich N sind, und wobei ein V gleich C ist, worüber die Anbindung an die chemische Einheit AF2 erfolgt;

wobei die Anknüpfungsposition der chemischen Einheit AF1 an die chemische Einheit AF2 durch den Platzhalter # gekennzeichnet ist,
und wobei im Übrigen die für Unterformel 1 gegebenen Definitionen gelten.

In einer weiteren Ausführungsform weisen die beschriebenen Moleküle eine Struktur der Formel 15 auf oder haben eine Struktur der Formel 15,

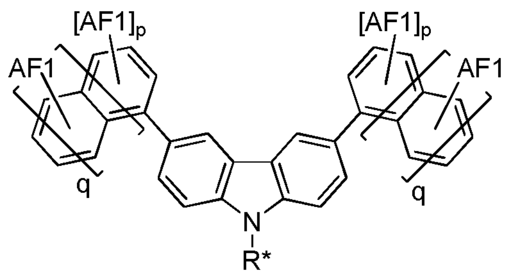

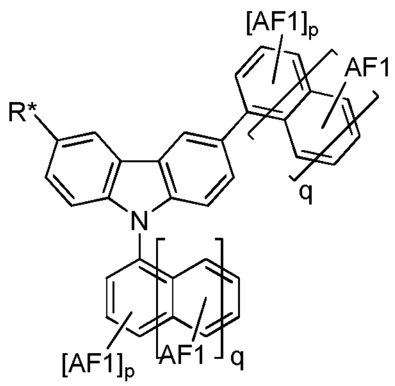

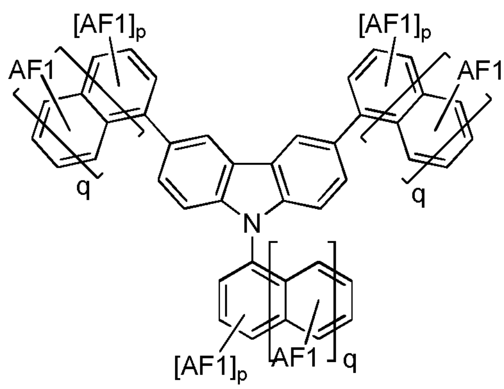

**Formel 15**

wobei

p = 0 oder 1,
q = 0 oder 1,
und wobei p ≠ q,
AF1 eine Struktur der Formel 15b aufweist;

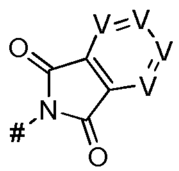

**Formel 15b**

wobei

V ist bei jedem Auftreten unabhängig voneinander CR* oder N, wobei maximal zwei Einheiten V gleichzeitig gleich N sind; und wobei nicht zwei einander benachbarte Einheiten V gleichzeitig gleich N sind;

wobei die Anknüpfungsposition der chemischen Einheit AF1 an den jeweiligen Separator durch den Platzhalter # gekennzeichnet ist,
und wobei im Übrigen die für Unterformel 1 gegebenen Definitionen gelten.

In einer weiteren Ausführungsform weisen die beschriebenen Moleküle eine Struktur der Formel 16 auf oder haben eine Struktur der Formel 16,

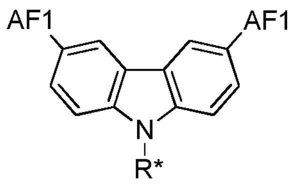
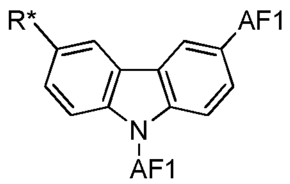
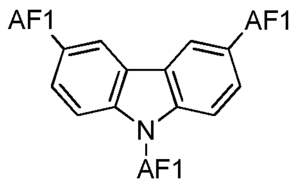

**Formel 16**

wobei
AF1 eine Struktur der Formel 16b aufweist;

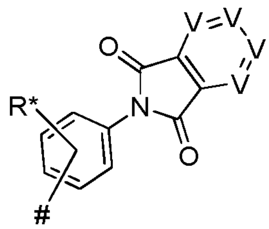

**Formel 16b**

wobei

V ist bei jedem Auftreten unabhängig voneinander CR* oder N, wobei maximal zwei Einheiten V gleichzeitig gleich N sind; und wobei nicht zwei einander benachbarte Einheiten V gleichzeitig gleich N sind;

wobei die Anknüpfungsposition der chemischen Einheit AF1 an die chemische Einheit AF2 durch den Platzhalter # gekennzeichnet ist,
und wobei im Übrigen die für Unterformel 1 gegebenen Definitionen gelten.

In einer Ausführungsform der Erfindung unterscheidet insbesondere der Separator S die organischen Moleküle funktionell von Molekülen gemäß dem Stand der Technik, da die hier gezeigte Art der Trennung von AFs (oder Donoren und Akzeptoren) bisher noch nicht bekannt ist. Separatoren dienen der Unterbrechung der elektronischen Kommunikation zwischen den chemischen Einheiten AF1 und AF2 durch eine derartige Verknüpfung der Einheiten, dass die Grenzorbitale HOMO und LUMO auf mehrheitlich separaten Molekülteilen liegen, was ohne den Separator nicht zwingend der Fall sein muss. Separatoren im Sinne dieser Erfindung verändern die Lage des HOMO bzw. LUMO der AFs nicht signifikant. Als nicht signifikant gilt im Rahmen dieser Erfindung eine Veränderung von nicht mehr als +/- 0,4 eV. Die Berechnung derartiger Energien ist bekannt und funktioniert nach der oben beschriebenen Weise per DFT-Rechnung.
Anhand spektroskopischer Auswahlregeln (symmetrische Moleküle) oder durch Messung des Extinktionskoeffizienten (UV/VIS-Spektroskopie) oder anhand quantenchemischer Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 300 $\mu$s, insbesondere < 100 $\mu$s, oder von < 50 $\mu$s. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.
Ein Maß für die Abklingdauer ist das quantenmechanischen Überlappungsintegral, welches näherungsweise durch den oben definierten Überlappungsparameter O dargestellt wird. Je kleiner das Überlappungsintegral, desto stärker sind die Grenzorbitale HOMO und LUMO getrennt, und umso wahrscheinlicher der Charge-Transfer-Übergang. Jedoch sinkt gleichermaßen die Wahrscheinlichkeit einer TADF-Emission aufgrund abnehmender Oszillatorstärken. Bei einem Wert von 1 für den Überlappungsparameter O liegt nicht mehr verzögerte Fluoreszenz (TADF) aufgrund eines Charge-Transfer Übergangs vor.
Um eine effiziente TADF-Emission mit kurzen Abklingdauern zu erreichen, muss das Überlappungsintegral gezielt gesteuert werden. Die gewünschte Überlappung wird durch die geeignete Wahl eines erfindungsgemäßen Separators S erreicht.
Ein Maß für die Abklingdauer ist der $\Delta E(S_1-T_1)$-Abstand. Dieser wird durch die Überlappung von HOMO und LUMO beeinflusst. Die Größe des quantenmechanischen Überlappungsintegrals, welche nach oben genannter DFT-Methode berechenbar ist, kann durch Wahl des Separators gezielt gesteuert werden. Kommt es zur völligen Trennung von HOMO und LUMO hat dieses einen Wert von 0. Die Wahrscheinlichkeit einer effizienten Emission des organischen Moleküls sinkt drastisch. Bei einem Wert von 1 liegt nicht mehr verzögerte Fluoreszenz (TADF) sondern spontane Emission vor. Die gewünschte Überlappung wird durch die geeignete Wahl eines erfindungsgemäßen Separators S erreicht.

Die erfindungsgemäßen Separatoren S erfüllen dabei insbesondere zwei wesentliche funktionelle Merkmale (s. Figur 2):

- die HOMO-Energie ist niedriger als die HOMO-Energie der als Donor fungierenden chemischen Einheit AF und
- die LUMO-Energie ist höher als die LUMO-Energie der als Akzeptor fungierenden chemischen Einheit AF.

Beispiele für erfindungsgemäße Moleküle mit der Struktur AF2-(S-AF1)$_2$ sind in Tabelle 4 aufgeführt, wobei die Benennung der einzelnen Moleküle folgendermaßen erfolgte: die Zahlen rechts und links entsprechen den Nummern der Einheiten AF1 aus Tabelle 1a und der Einheiten AF2 aus Tabelle 2, S-i (i ist 7 bis 11) steht für die entsprechenden Separatoren aus Tabelle 3. Weitere Beispiele für Moleküle gemäß der Beschreibung sind in Tabelle 4b aufgeführt.

**Tabelle 4a:** Erfindungsgemäße Moleküle der Struktur AF2–(S–AF1)$_2$.

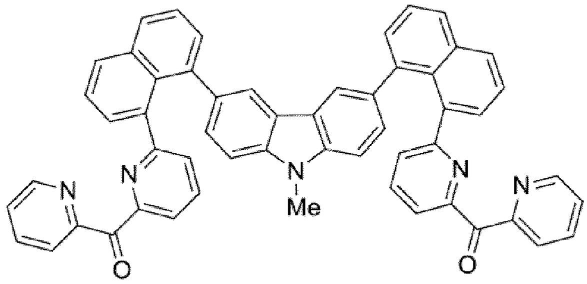

328–(S-10–113)$_2$

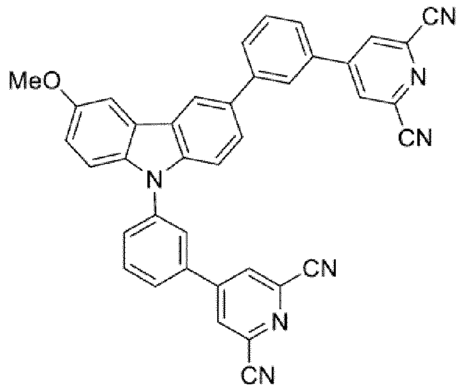

436–(S-8–371)$_2$

**Tabelle 4b:** Nicht-erfindungsgemäße Moleküle der Struktur AF2–(S–AF1)$_2$.

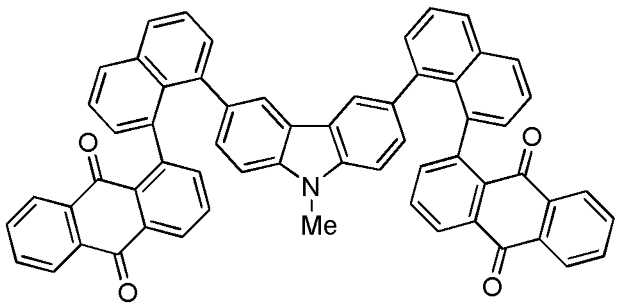

328–(S-10–36)$_2$

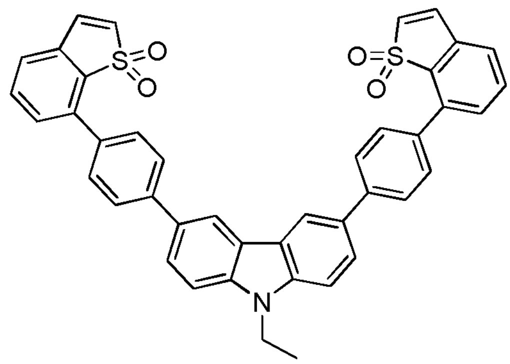

328–(S-9–177)$_2$

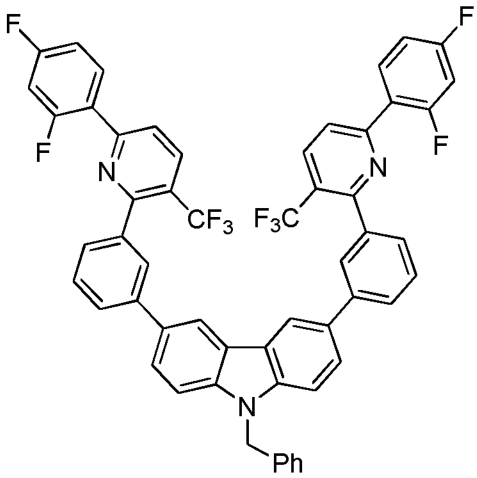

55–(S-8–305)$_2$

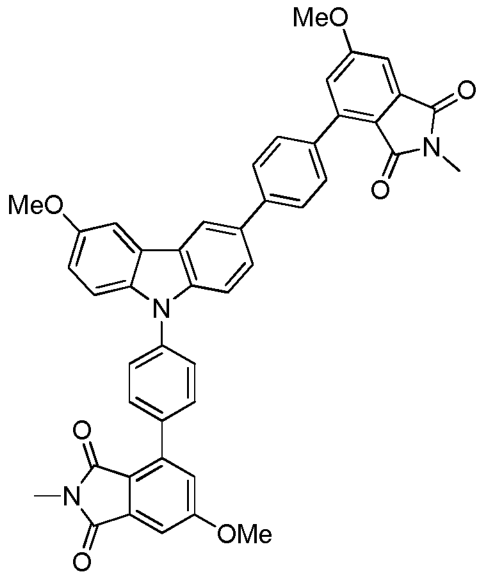

436–(S-9–441)$_2$

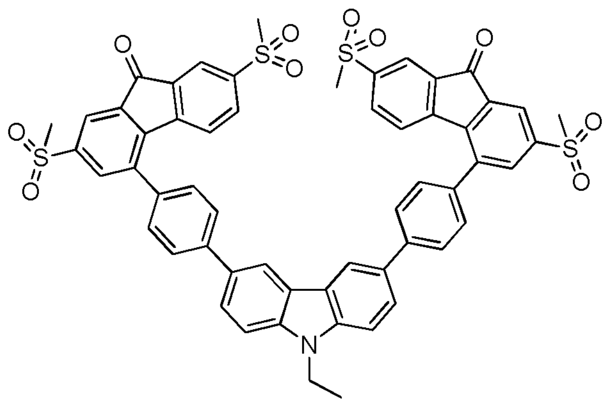

328–(S-9–364)$_2$

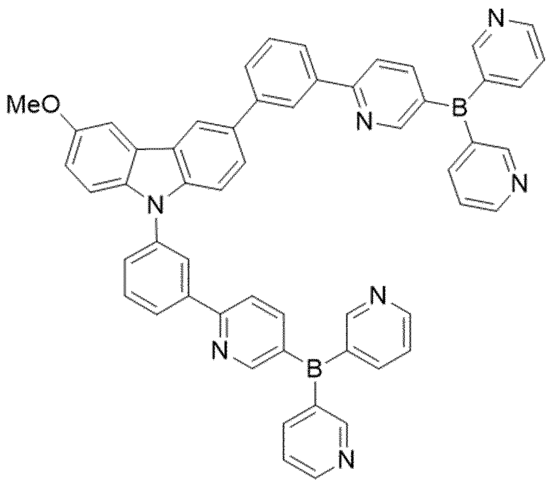

436–(S-8–144)$_2$

In einer Ausführungsform weisen erfindungsgemäße Moleküle keinen Separator S auf, sodass sich beispielsweise Moleküle der Form AF2-(AF1)$_2$ oder AF1-AF2 bzw. AF2-AF1 ergeben bei denen die chemischen Einheiten AF1 und AF2 direkt kovalent aneinander gebunden sind, wobei die kovalente Bindung zwischen den in Tabelle 1 bzw. Tabelle 2 mit Kleinbuchstaben bezeichneten Positionen der Einheiten AF1 und AF2 erfolgt. Beispiele derartiger erfindungsgemäßer Moleküle sind in Tabelle 5a aufgeführt. In Tabelle 5b sind weitere nicht-erfindungsgemäße Moleküle aufgeführt.

**Tabelle 5a:** Erfindungsgemäße Moleküle der Form AF2–(AF1)$_2$.

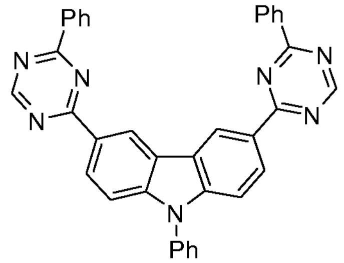

55–(85)$_2$

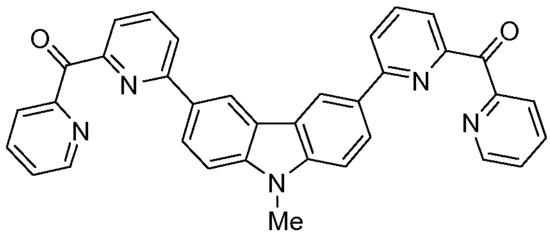

328–(113)$_2$

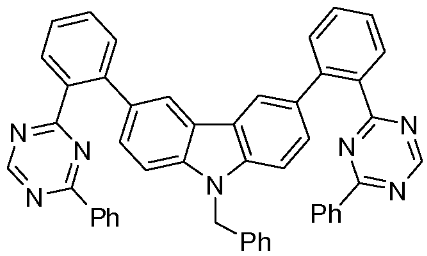

55–(233)$_2$

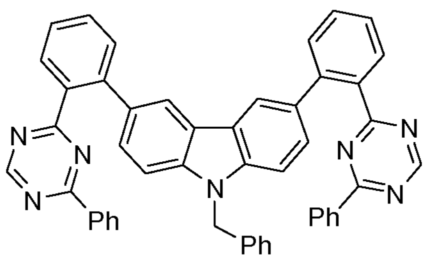

55–(233)$_2$

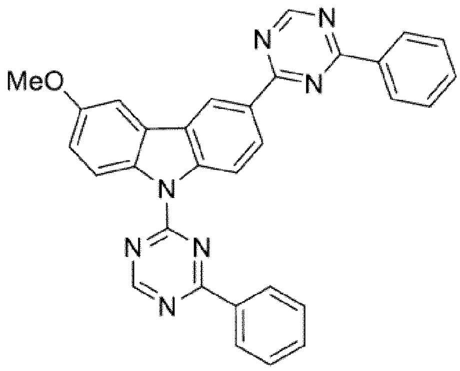

436–(85)$_2$

**Tabelle 5b:** Nicht-erfindungsgemäße Moleküle der Form AF2–(AF1)$_2$.

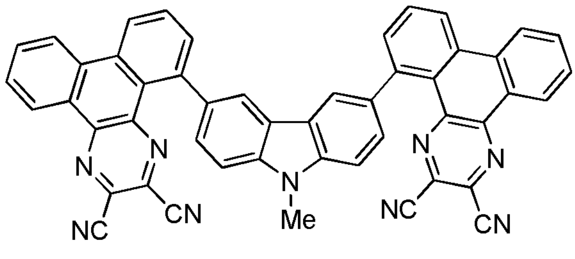

328–(70)$_2$

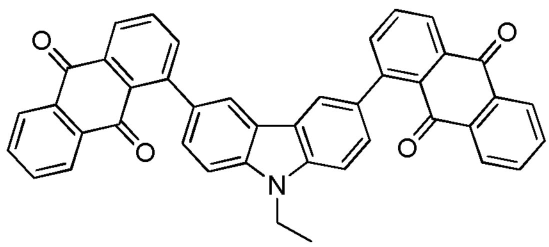

328–(36)$_2$

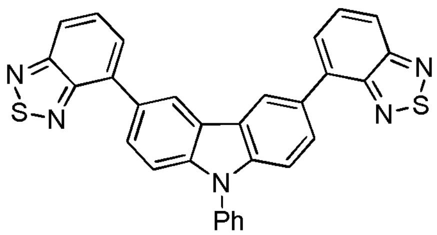

55–(115)$_2$

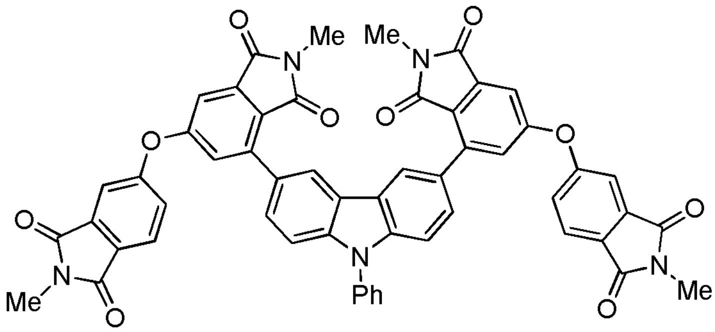

55–(367)$_2$

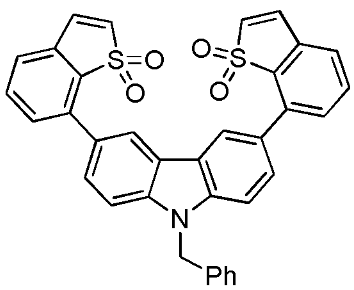

55–(177)$_2$

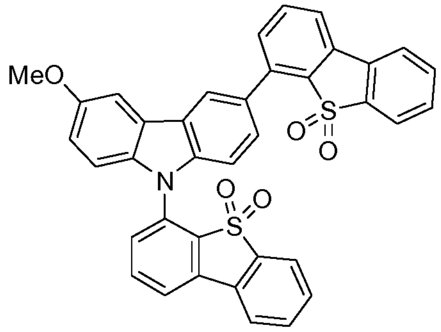

436–(198)$_2$

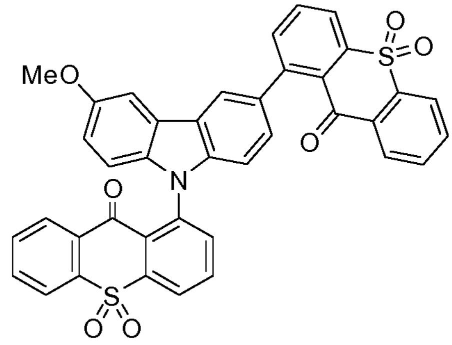

436–(43)

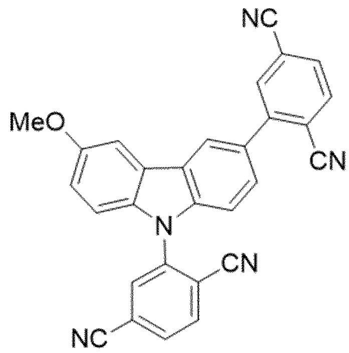

436–(151)$_2$

Weitere Beispiele für erfindungsgemäße und nicht-erfindungsgemäße Moleküle, wobei erfindungsgemäße Moleküle mit * gekennzeichnet sind.

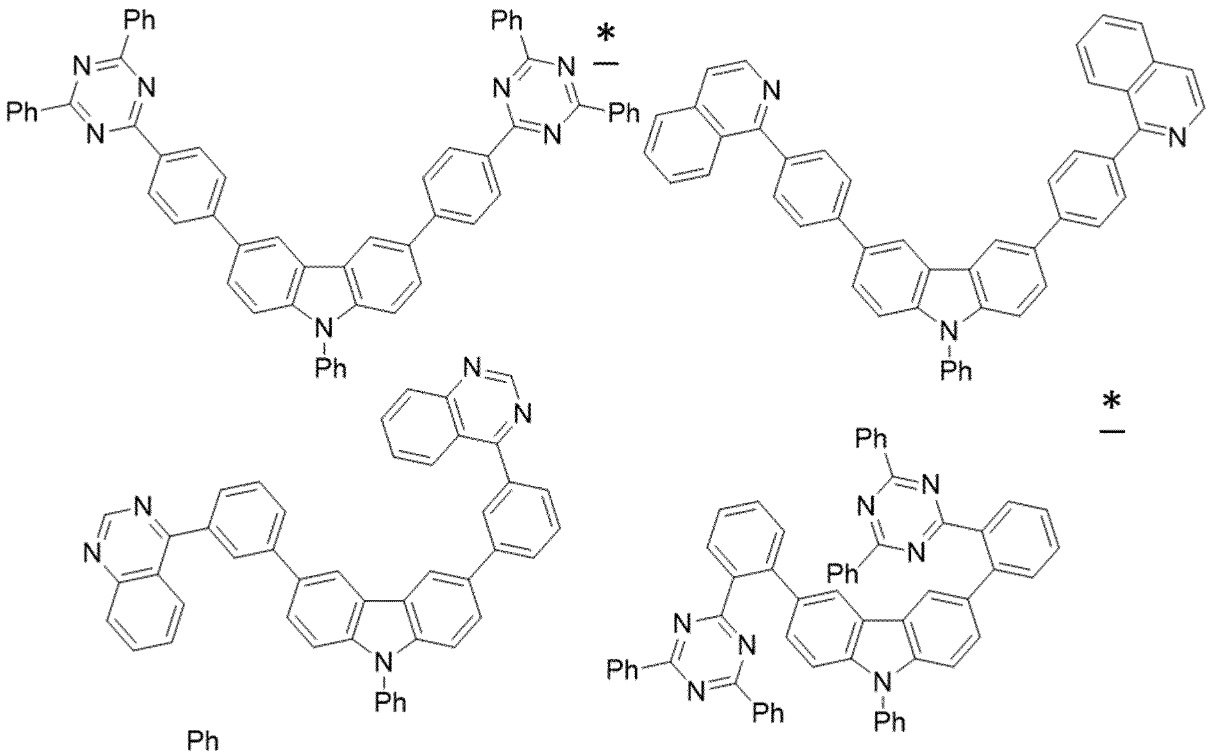

*

*

*

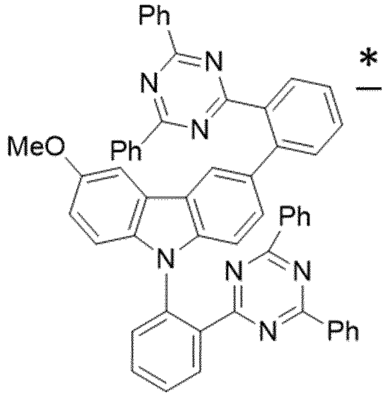

*

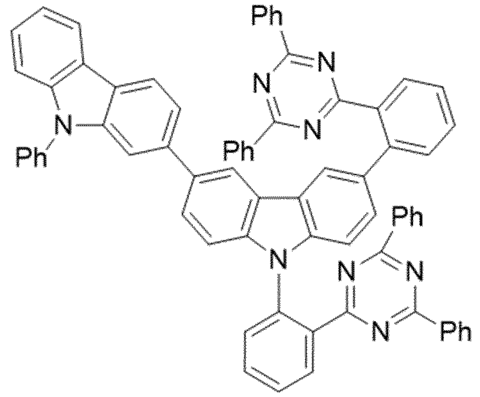

*

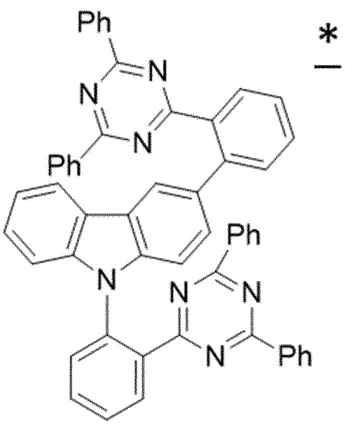

*

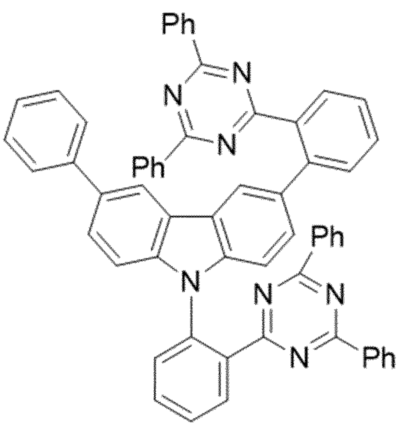

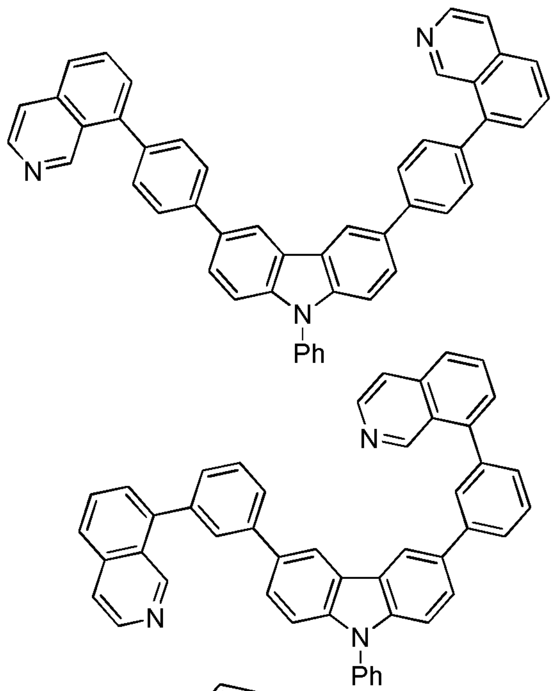

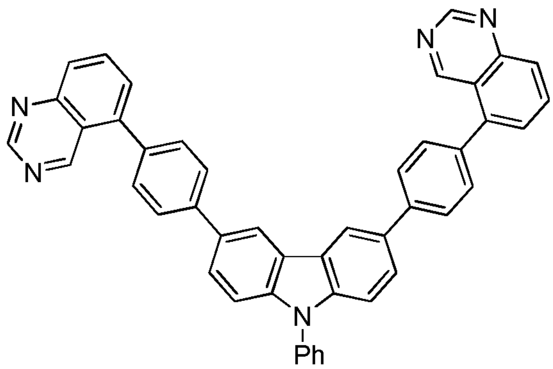

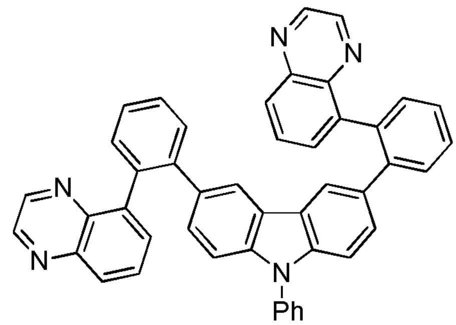

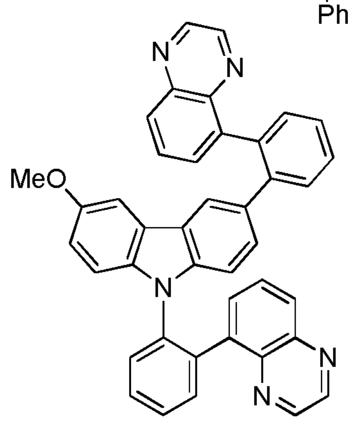

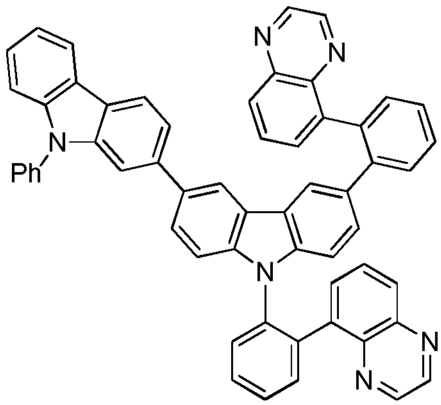

*

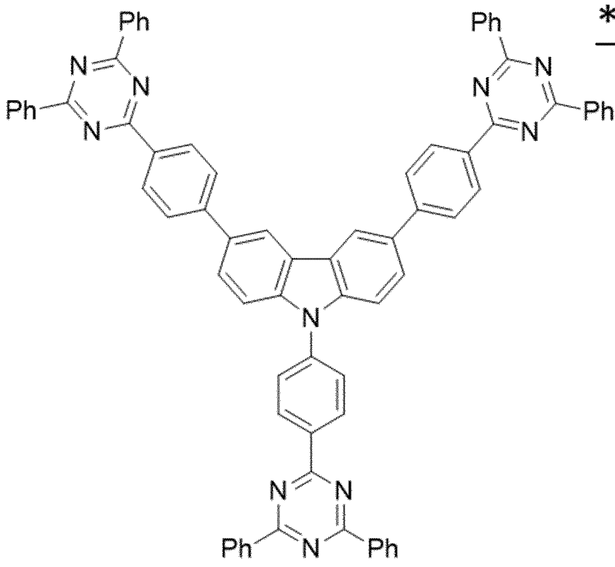

*

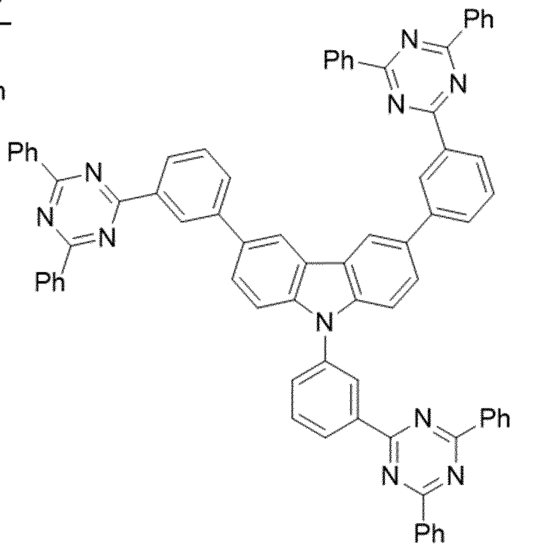

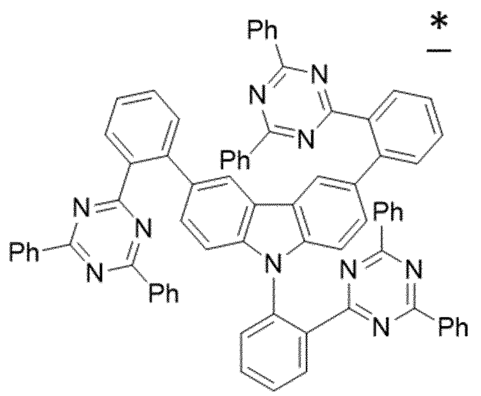

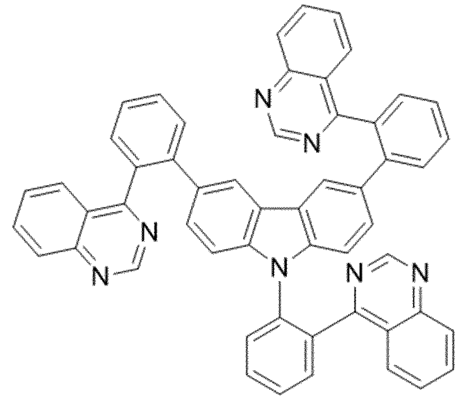

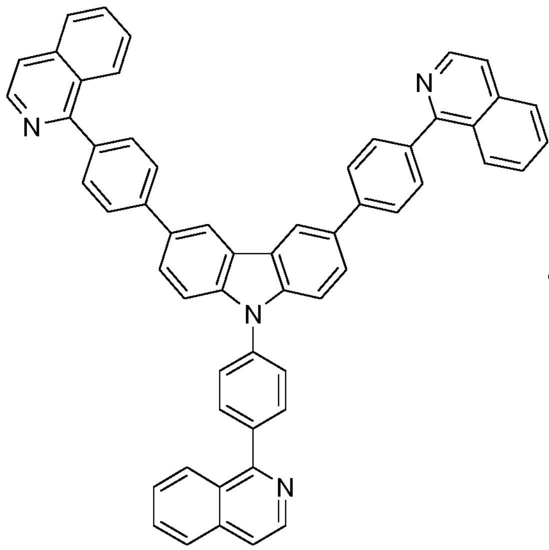

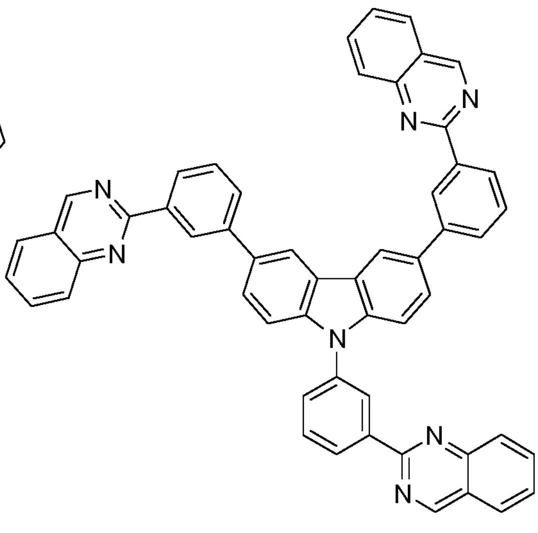

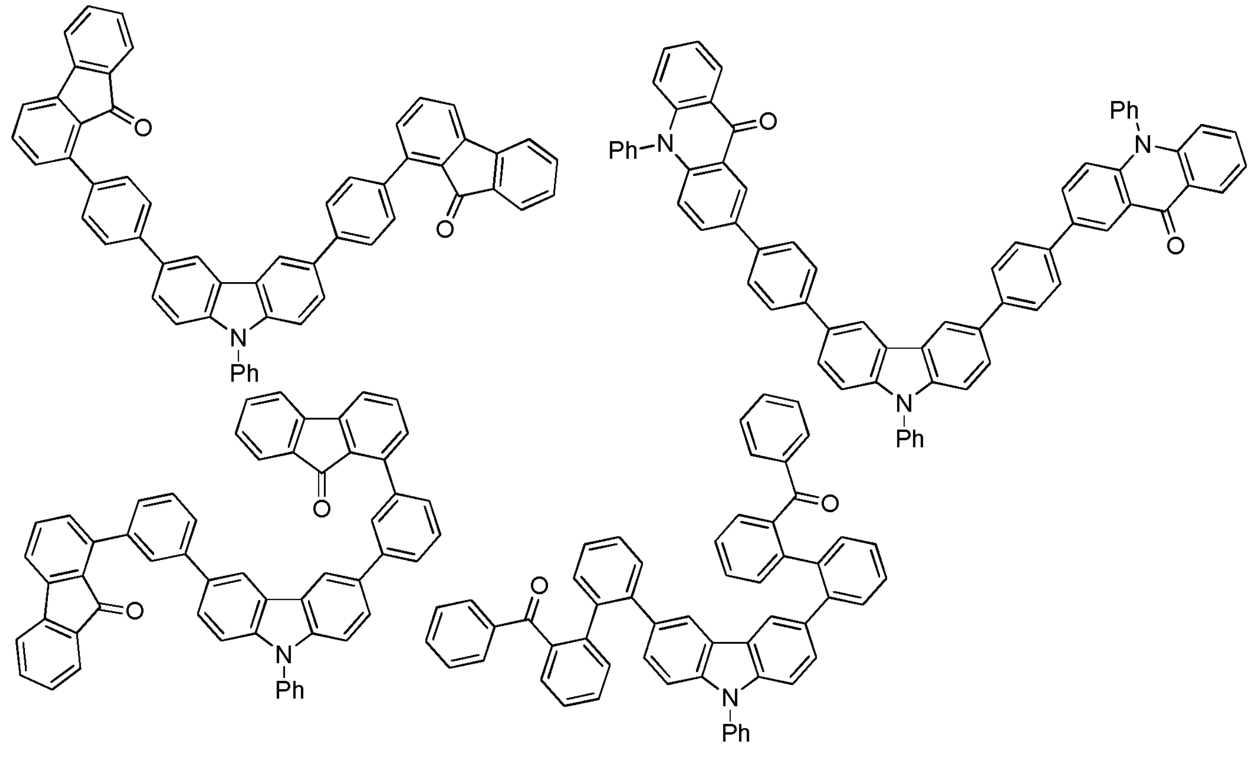

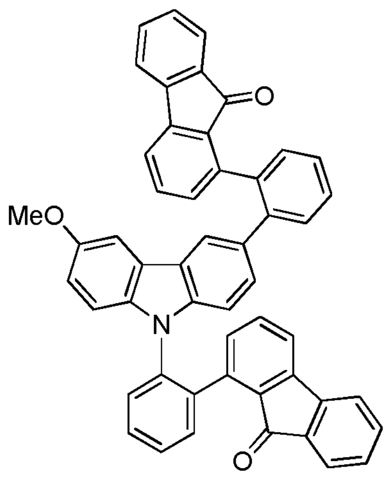

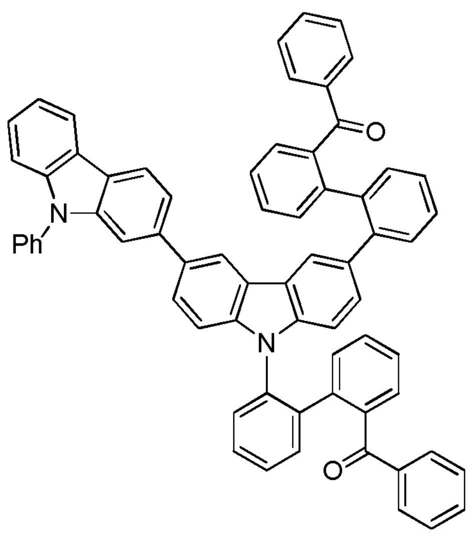

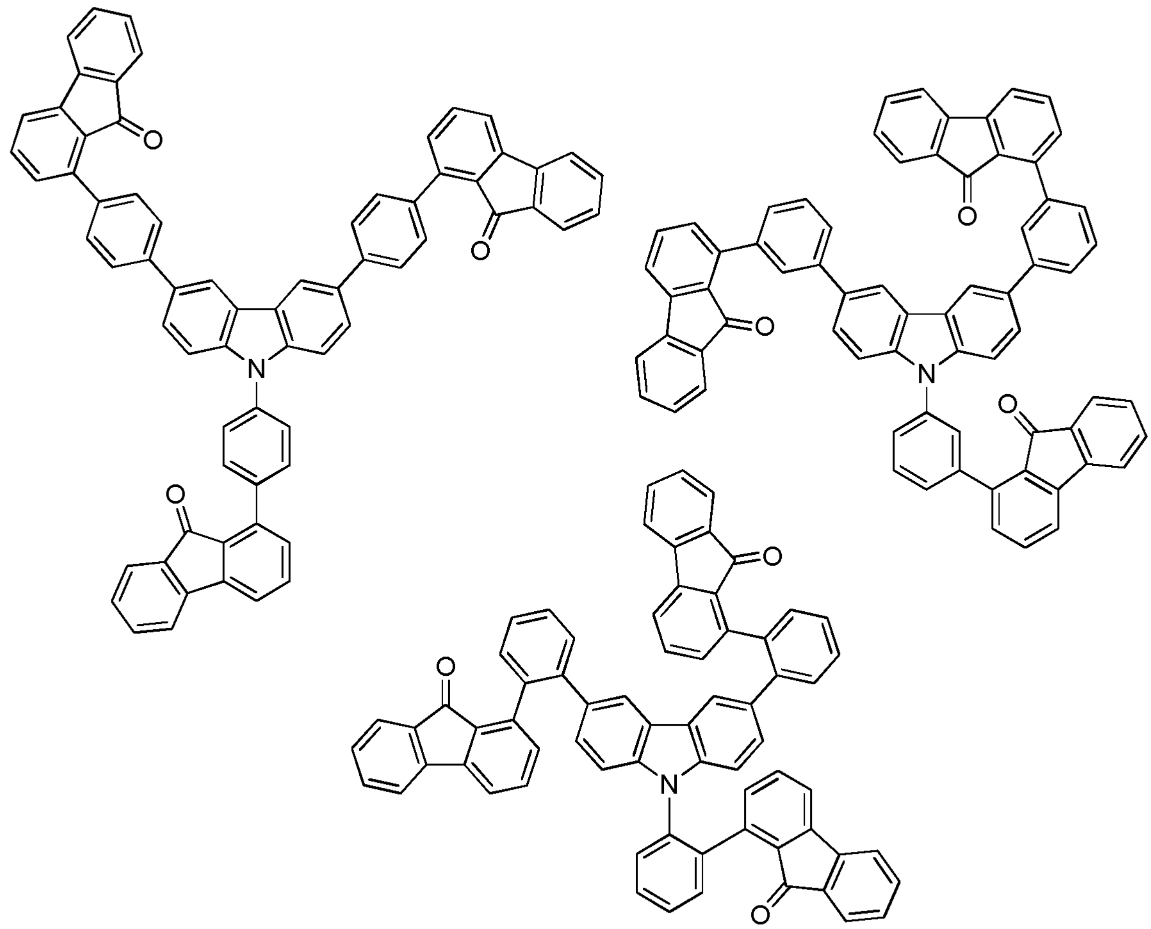

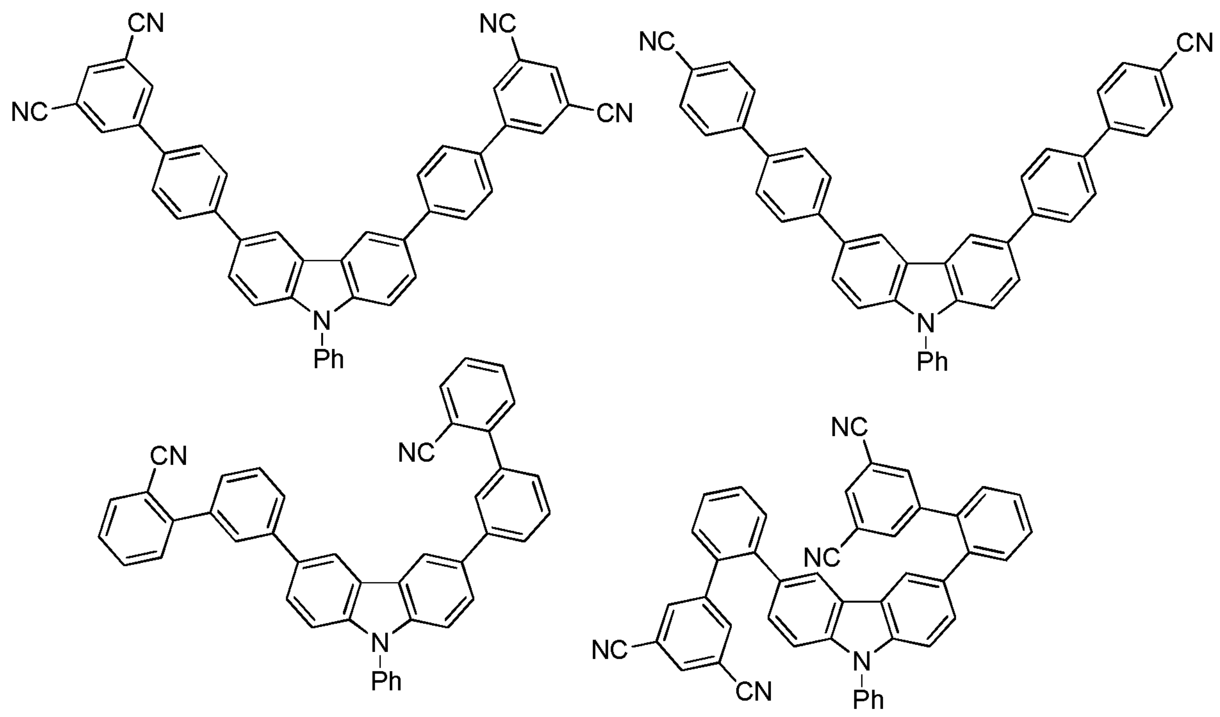

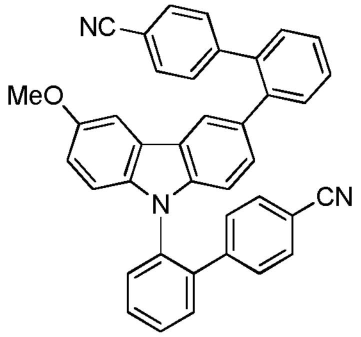

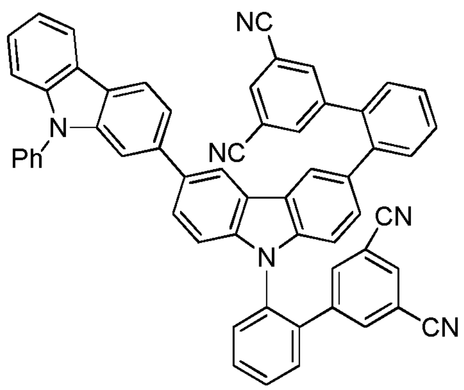

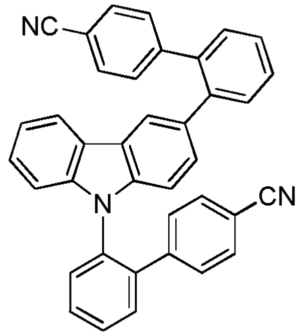

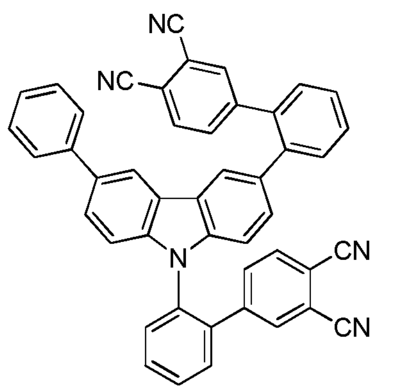

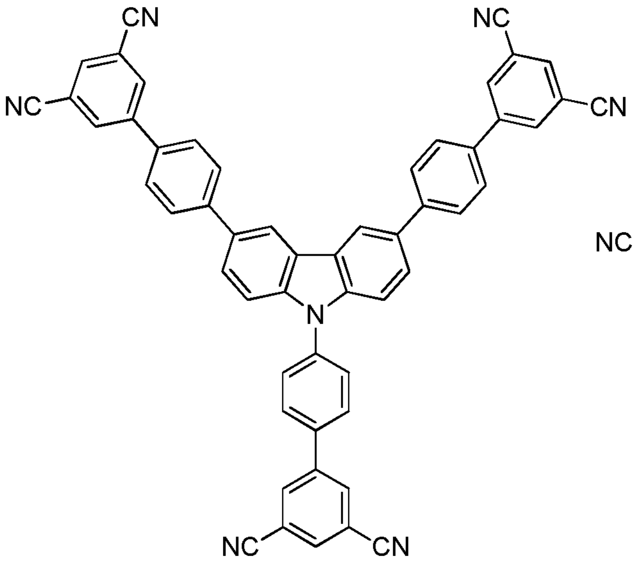

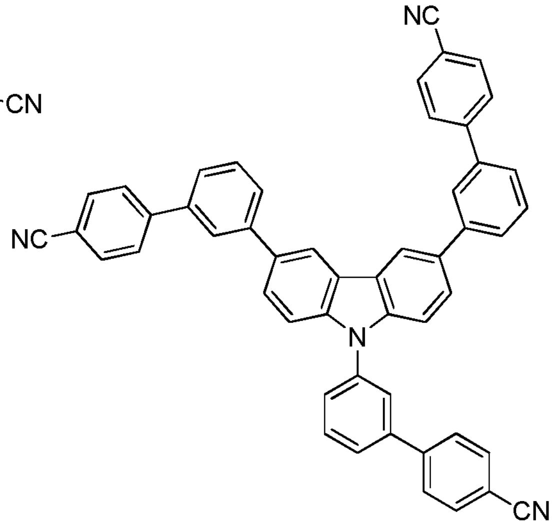

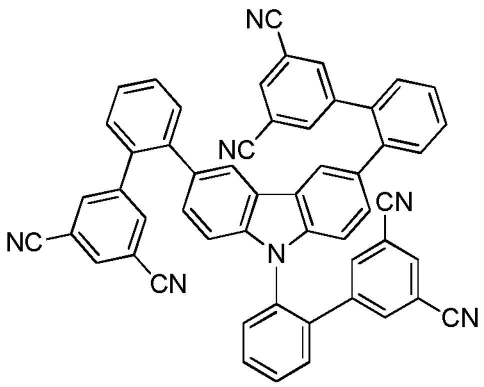

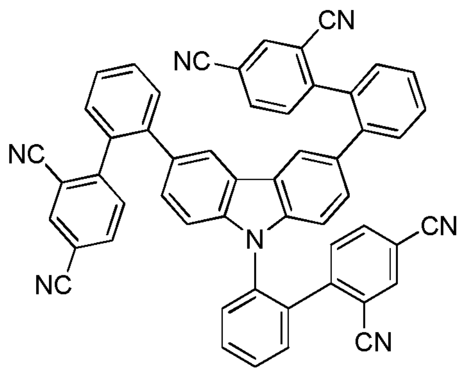

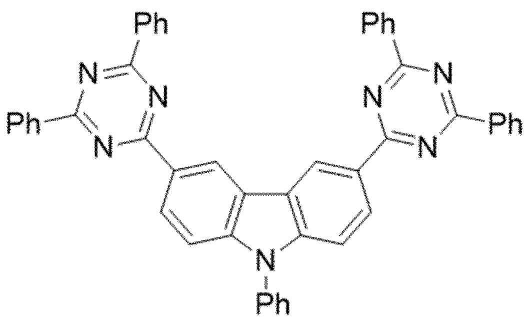

*

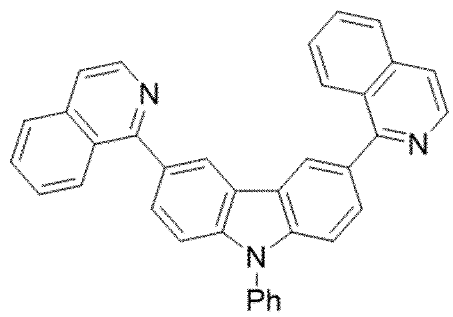

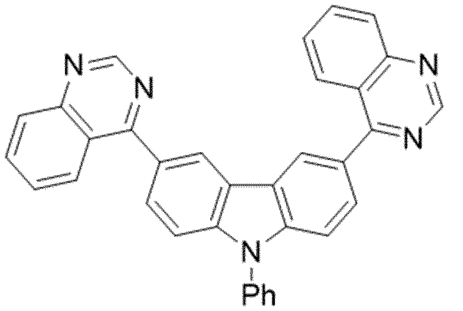

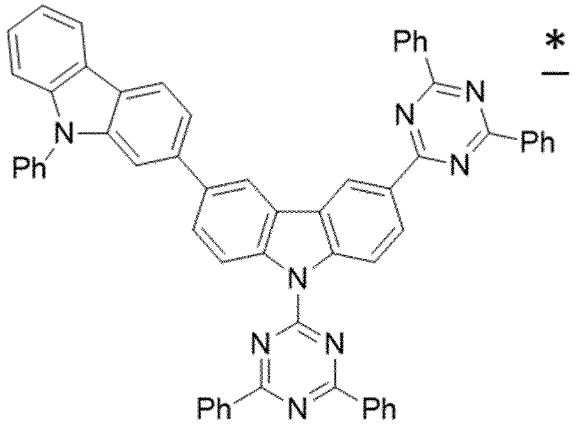

*

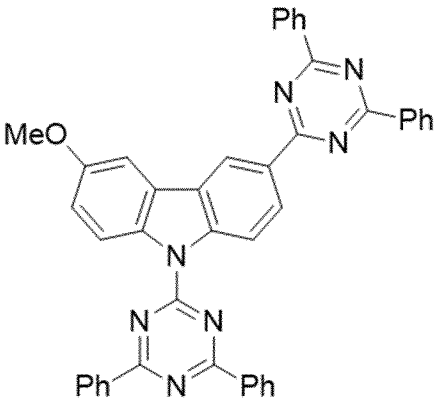

*

*

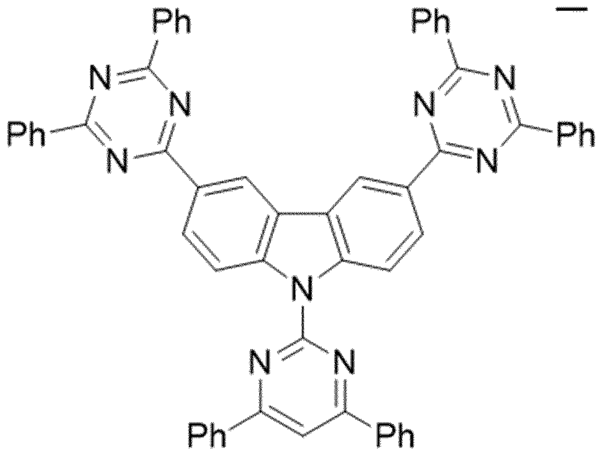

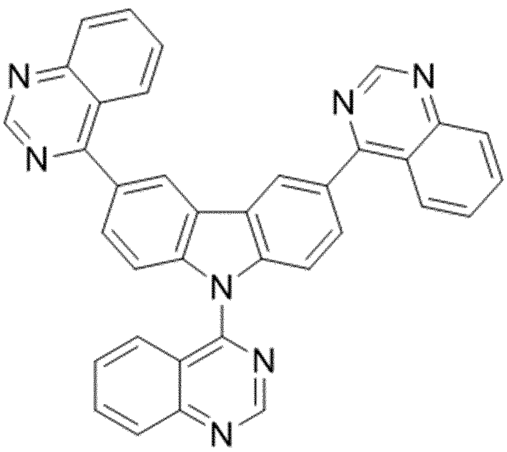

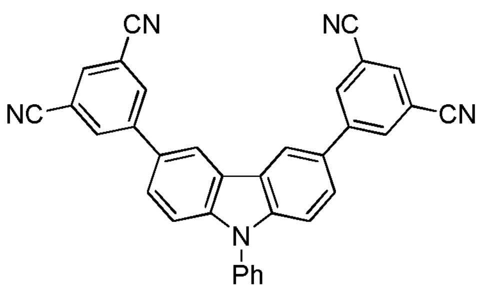

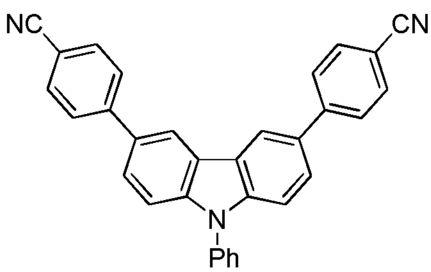

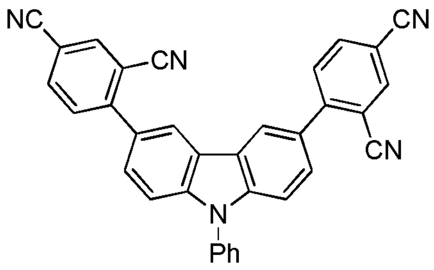

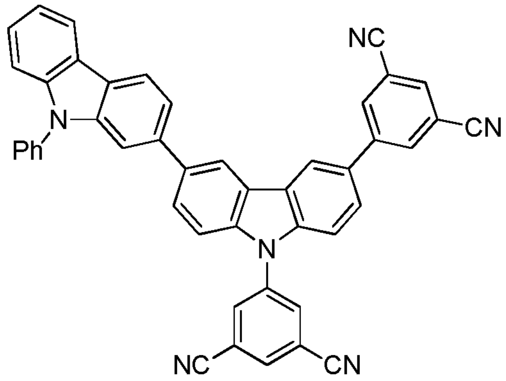

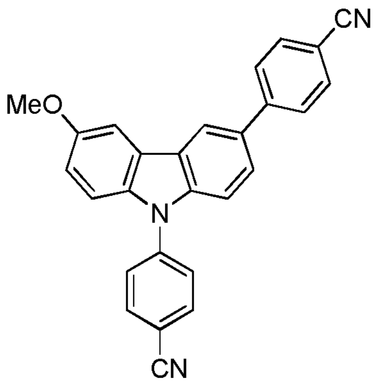

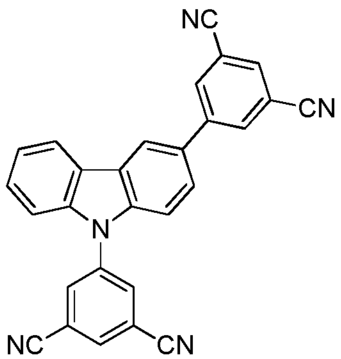

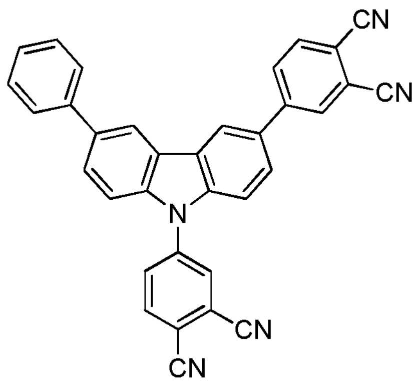

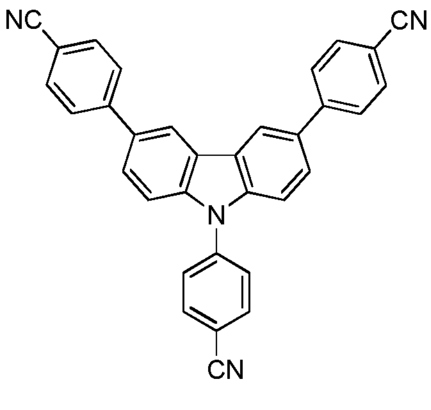

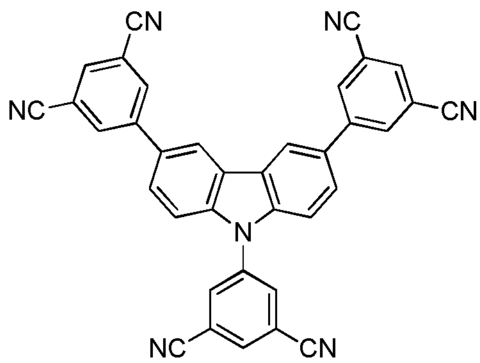

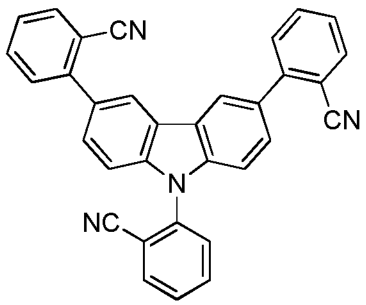

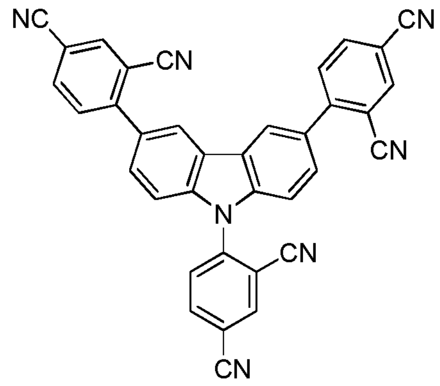

In einer Ausführungsform tragen erfindungsgemäße Moleküle, die keinen Separator S aufweisen, an mindestens einer der kovalenten Bindung zwischen AF1 und AF2 benachbarten Position einen Rest R****, wobei gilt: R**** ist entweder ein Rest AF1, oder ein Rest R*, wobei für R* die Definition aus Unterformel 1 gilt; wobei die Reste R* insbesondere gleich Methyl, Ethyl, *iso*-Propyl, *tert*-Butyl oder Phenyl sind.

Durch die Anwesenheit eines oder mehrerer dieser Substituenten in ortho-Position wird eine Verdrillung der chemischen Einheiten AF1 und AF2 gegeneinander hervorgerufen wird. Dies führt zu einer räumlichen Trennung der Grenzorbitale und damit zu niedrigen $\Delta E_{S1-T1}$-Werten im Bereich einiger $k_BT$ (T = 300 K). Gleichermaßen wird eine Verdrillung um weniger als 90 ° gewährleistet, sodass das Überlappungsintegral zwischen HOMO und LUMO und damit die Oszillatorstärke des radiativen Übergangs einen von Null verschiedenen Wert annimmt (s. nächster Abschnitt).

Die Erlaubtheit eines quantenmechanischen Überganges ist, wie allgemein bekannt, entweder durch theoretisch ableitbare spektroskopische Auswahlregeln (symmetrische Moleküle) oder durch Messung des Extinktionskoeffizienten (UV/VIS-Spektroskopie) oder quantenchemische Berechnung der Oszillatorstärke zugänglich, wobei sich Erlaubtheit durch eine große Oszillatorstärke auszeichnet. Je größer die Oszillatorstärke, desto schneller der damit verbundene Prozess. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert. Wird wie in der vorliegenden Erfindung durch eine geeignete Wahl des Anknüpfungsmotivs der AF1 eine gewisse Durchdringung von HOMO und LUMO erreicht, steigt das quantenmechanische Überlappungsintegral, und damit auch die Oszillatorstärke, so dass die Abklingzeit auf Werte unter 50 $\mu$s absinkt.

In einer Ausführungsform werden an die chemisch substituierbaren Positionen der erfindungsgemäßen organischen Moleküle nach Formel 1 weitere Reste R angefügt, um die Löslichkeit der Emitter zu steigern und/oder die Polymerisierbarkeit zu ermöglichen ohne dabei die elektronischen Eigenschaften des Moleküls signifikant zu verändern, sodass auch bei Verwendung von R ein Emitter vorliegt, wobei gilt:

jedes R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$, - $NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder

eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, - $Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-, - C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-, - C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, - $As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine

benachbarte $CH_2$-Gruppe durch-$Si(R^4)_2$-, -$Ge(R^4)_2$-, -$Sn(R^4)_2$, -C(=O)-, - C(=S)-, -C(=Se)-, -C=N-, -C(=O)O-, -C(=O)N($R^3$)-, -P(=O)($R^7$)-, -As(=O)($R^7$)-, -P(=S)($R^7$)-, - As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus Phenyl, Naphthyl, $N(R^2)_2$, CN, $CF_3$, C(=O)O$R^3$, C(=O)$N(R^3)_2$, $Si(R^4)_3$, C(=O)$R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, bzw. eine benachbarte $CH_2$-Gruppe durch -$Si(R^4)_2$-, - $Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-, - As(=O)($R^7$)-, -P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, C(=O)O$R^3$, C(=O)$N(R^3)_2$, $Si(R^4)_3$, $B(OR^5)_2$, C(=O)$R^3$, P(=O)$(R^7)_2$, P(=S)$(R^7)_2$, As(=O)$(R^7)_2$, P(=S)$(R^7)_2$, S(=O)$R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch -$R^3C=CR^3$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-, - C=N-, -C(=O)O-, -C(=O)N($R^3$)-, -P(=O)($R^7$)-, -As(=O)($R^7$)-, -P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem;

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 17 und 18 erhalten werden, die als Polymere in der lichtemittierenden Schicht des optoelektronischen Bauelements Verwendung finden können.

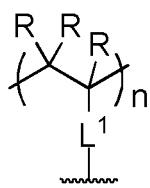 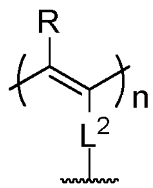

**Formel 17** **Formel 18**

In Formel 17 und 18 stehen L1 und L2 für gleiche oder verschiedene Linkergruppen, die 0 bis 20, insbesondere 1 bis 15, oder 2 bis 10 Kohlenstoffatome aufweist, und wobei die gewellte Linie die Position kennzeichnet, über die die Linkergruppe an das erfindungsgemäße organische Molekül angebunden ist. In einer Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, insbesondere eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form -C(=O)O- auf. R ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten R auch miteinander ein mono- oder polycyclisches, aliphatisches Ringsystem bilden, außerdem kann R sein: eine lineare Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen.

Vorteilhafte Ausführungsformen der Wiederholungseinheiten sind Strukturen der Formeln 19 bis 24:

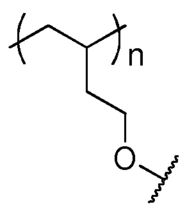 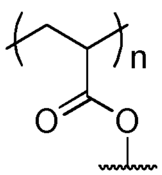 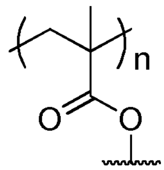 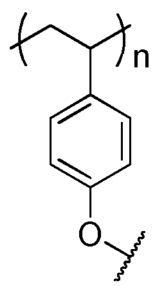 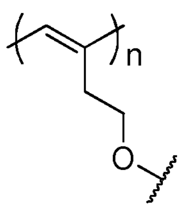 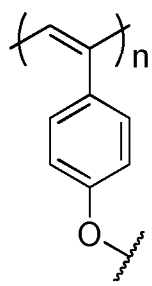

**Formel 19** **Formel 20** **Formel 21** **Formel 22** **Formel 23** **Formel 24**

Zur Herstellung der Polymere, die die Wiederholungseinheiten gemäß Formel 19 bis 24 aufweisen, werden die polymerisierbaren funktionellen Einheiten über eine Linkergruppe der Formeln 25 bis 30, die eine Hydroxyleinheit aufweisen, an ein erfindungsgemäßes organisches Molekül angebunden und die daraus resultierenden Verbindungen mit sich selbst homopolymerisiert oder mit anderen geeigneten Monomeren copolymerisiert.

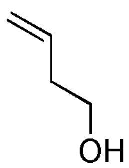 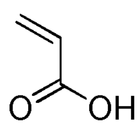 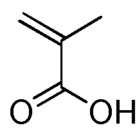 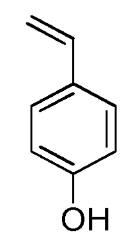 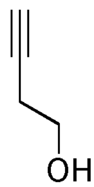 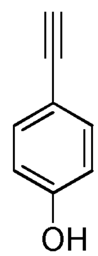

**Formel 25** **Formel 26** **Formel 27** **Formel 28** **Formel 29** **Formel 30**

Polymere, die eine Einheit gemäß Formel 17 oder Formel 18 aufweisen, können dabei entweder ausschließlich Wiederholungseinheiten mit einer Struktur der allgemeinen Formel 17 oder 18, oder Wiederholungseinheiten mit einer

anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche

- einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweisen und/oder
- eine Emissionslebensdauer von höchstens 50 µs aufweisen.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden
- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des Bauelements wird das organische Molekül als Emissionsmaterial in einer Emissionsschicht eingesetzt, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül mindestens ein Hostmaterial auf, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform weist das optoelektronische Bauelement mindestens ein Hostmaterial aus einem erfindungsgemäßen Material auf.

In einer weiteren Ausführungsform des optoelektronischen Bauelements beinhaltet die lichtemittierende Schicht fluoreszente oder phosphoreszente Materialien, welche eine Struktur der erfindungsgemäßen Moleküle aufweisen.

In einer weiteren Ausführungsform des optoelektronischen Bauelements bilden ein erfindungsgemäßes organisches Molekül und ein funktionelles Material, beispielsweise in Form eines weiteren Emitter-Materials, eines Host-Materials, oder eines weiteren organisches Moleküls, welches zur Bildung eines Exciplex mit dem Molekül befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

In einer weiteren Ausführungsform des optoelektronischen Bauelements ist die Emission durch thermisch aktivierte verzögerte Fluoreszenz (TADF) charakterisiert.

In einer weiteren Ausführungsform des optoelektronischen Bauelements werden organische Moleküle als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett ($T_1$)- und Singulett ($S_1$)-Energieniveaus des Hostmaterials energetisch höher liegen als die Triplett ($T_1$)- und Singulett ($S_1$)-Energieniveaus des organischen Moleküls, und wobei das organische Molekül Fluoreszenz oder thermisch aktivierte verzögerte Fluoreszenz (TADF) emittiert, und einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform weist das Verfahren den Schritt der Verarbeitung des organischen Moleküls mittels eines Vakuumverdampfungsverfahrens oder aus einer Lösung auf.

In einer Ausführungsform weist das Verfahren das Aufbringen des organischen Moleküls auf einen Träger auf, wobei das Aufbringen insbesondere nass-chemisch, mittels kolloidaler Suspension oder mittels Sublimation erfolgt.

In einer weiteren Ausführungsform des Verfahrens wird mindestens eine Schicht

- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

## Patentansprüche

**1.** Organisches Molekül, wobei gilt:

im Gesamtmolekül sind mindestens zwei chemische Einheiten AF1 und eine davon verschiedene chemische Einheit AF2 enthalten;

AF1 ist eine erste chemische Einheit, aufweisend ein konjugiertes System, wobei AF1 eine Struktur der Unterformel 1.1 aufweist oder eine Struktur der Unterformel 1.1 hat

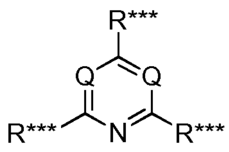

Unterformel 1.1

wobei gilt:
Q ist N, CR***, wobei nicht zwei direkt benachbarte Einheiten Q gleichzeitig gleich N sind;
und wobei gilt:

R*** ist bei jedem Auftreten unabhängig voneinander entweder ein Rest R*, oder eine chemische Bindung an einen Separator S, oder eine chemische Bindung an einen Rest AF2, wobei genau ein R*** eine chemische Bindung an einen Separator S, oder eine chemische Bindung an einen Rest AF2 ist;
AF2 ist eine zweite chemische Einheit, aufweisend ein konjugiertes System, wobei AF2 durch die in Unterformel 1 dargestellte allgemeine Formel beschrieben wird;
Separator (S) ist eine chemische Einheit, die AF1 und AF2 verknüpft und deren elektronische Kommunikation über konjugierte Bindungen miteinander unterbricht; wobei optional in dem organischen Molekül kein Separator S enthalten ist;

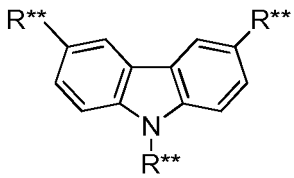

**Unterformel 1**

und wobei in Unterformel 1 bedeutet:

R** ist bei jedem Auftreten unabhängig voneinander entweder ein Rest R*, oder eine chemische Bindung an einen Separator S, oder eine chemische Bindung an einen Rest AF1; wobei mindestens zwei R** eine chemische Bindung an einen Rest AF1 oder eine chemische Bindung an einen Separator S sind,
wobei die Anknüpfung an die chemische Einheit AF1 auch über ein Atom des Rests R* erfolgen kann;

wobei gilt:

R* ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden;

$R^2$ ist bei jedem Auftreten unabhängig voneinander ausgewählt aus der Gruppe bestehend aus H, Deuterium, Phenyl, Naphthyl, $CF_3$, C(=O)O$R^3$, C(=O)N$(R^2)_2$, Si$(R^4)_3$, C(=O)$R^3$, P(=O)$(R^7)_2$, As(=O)$(R^7)_2$ P(=S)$(R^7)_2$, As(=S)$(R^7)_2$, S(=O)$R^3$, S$(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9$C=C$R^{9-}$, -C≡C-, oder eine benachbarte $CH_2$-Gruppe durch -Si$(R^4)_2$-, - Ge$(R^4)_2$-, -Sn$(R^4)_2$, -C(=O)-, -C(=S)-, -C(=Se)-, -C=N-, -C(=O)O-, -C(=O)N$(R^3)$-, -P(=O)$(R^7)$-, - As(=O)$(R^7)$-, -P(=S)$(R^7)$-, -As(=S)$(R^7)$-, -S(=O)-, -S$(=O)_2$-, -N$R^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, N$(R^2)_2$, CN, $CF_3$, OH, C(=O)O$R^3$, C(=O)N$(R^3)_2$, C(=O)$R^3$, P(=O)$(R^7)_2$, As(=O)$(R^7)_2$, P(=S)$(R^7)_2$, As(=S)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9$C=C$R^9$-, -C≡C-, oder eine benachbarte $CH_2$-Gruppe durch -Si$(R^4)_2$-, -Ge$(R^4)_2$-, -Sn$(R^4)_2$, -C(=O)-, - C(=S)-, -C(=Se)-, -C=N-, -C(=O)O-, -C(=O)N$(R^3)$-, -P(=O)$(R^7)$-, -As(=O)$(R^7)$-, -P(=S)$(R^7)$, - As(=S)$(R^7)$-, -S(=O)-, -S$(=O)_2$-, -N$R^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, C(=O)$R^3$, P(=O)$(R^7)_2$, As(=O)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9$C=C$R^9$-, -C≡C-, oder eine benachbarte $CH_2$-Gruppe durch -Si$(R^4)_2$-, -Ge$(R^4)_2$-, -Sn$(R^4)_2$, -C(=O)-, - C(=S)-, -C(=Se)-, -C=N-, -C(=O)O-, -C(=O)N$(R^3)$-, -P(=O)$(R^7)$-, -As(=O)$(R^7)$-, -P(=S)$(R^7)$-, - As(=S)$(R^7)$-, -S(=O)-, -S$(=O)_2$-, -N$R^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, Si$(R^4)_3$, C(=O)$R^3$, P(=O)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte

oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch-$Si(R^4)_2$-, -$Ge(R^4)_2$-, -$Sn(R^4)_2$, -C(=O)-, - C(=S)-, -C(=Se)-, -C=N-, -C(=O)O-, -C(=O)$N(R^3)$-, -P(=O)($R^7$)-, -As(=O)($R^7$)-, -P(=S)($R^7$), -As(=S)($R^7$)-, -S(=O)-, -S(=O)$_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, C(=O)$OR^3$, C(=O)$N(R^3)_2$, $Si(R^4)_3$, C(=O)$R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^{9-}$, -C≡C-, oder eine benachbarte $CH_2$-Gruppe durch -$Si(R^4)_2$-, - $Ge(R^4)_2$-, -$Sn(R^4)_2$, -C(=O)-, -C(=S)-, -C(=Se)-, -C=N-, -C(=O)O-, -C(=O)$N(R^3)$-, -P(=O)($R^7$)-, -As(=O)($R^7$)-, -P(=S)($R^7$), -As(=S)($R^7$)-, -S(=O)-, -S(=O)$_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, C(=O)$OR^3$, C(=O)$N(R^3)_2$, $Si(R^4)_3$, $B(OR^5)_2$, C(=O)$R^3$, P(=O)$(R^7)_2$, P(=S)$(R^7)_2$, As(=O)$(R^7)_2$, P(=S)$(R^7)_2$, S(=O)$R^3$, S(=O)$_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C=C-, oder eine benachbarte $CH_2$-Gruppe durch -$Si(R^4)_2$-, -$Ge(R^4)_2$-, -$Sn(R^4)_2$, -C(=O)-, -C(=S)-, -C(=Se)-, - C=N-, -C(=O)O-, -C(=O)$N(R^3)$-, -P(=O)($R^7$)-, -As(=O)($R^7$)-, -P(=S)($R^7$), -As(=S)($R^7$)-, -S(=O)-, - S(=O)$_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

**2.** Organisches Molekül nach Anspruch 1, wobei AF2 eine Struktur der Formel 2a-2d aufweist, wobei jeweils die beiden R** eine chemische Bindung an einen Rest AF1 oder eine chemische Bindung an einen Separator S sind, und im Übrigen die in Anspruch 1 angegebenen Definitionen gelten;

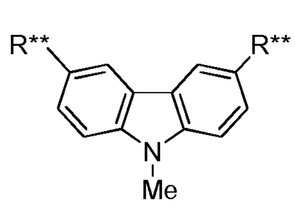

**Formel 2a**

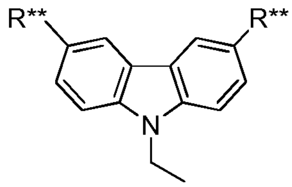

**Formel 2b**

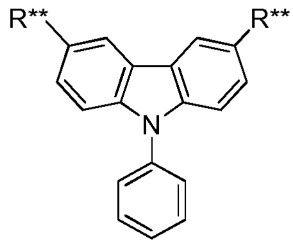

**Formel 2c**

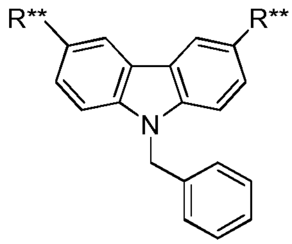

**Formel 2d**

**3.** Organisches Molekül nach Anspruch 1, wobei AF2 eine Struktur der Formel 3a-3h aufweist

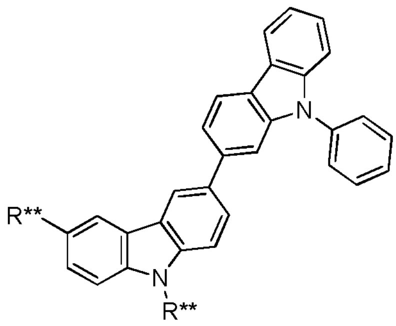

**Formel 3a**

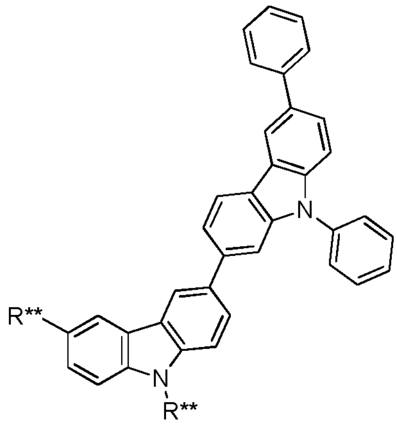

**Formel 3b**

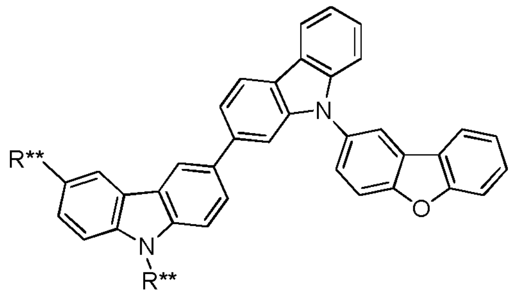

**Formel 3c**

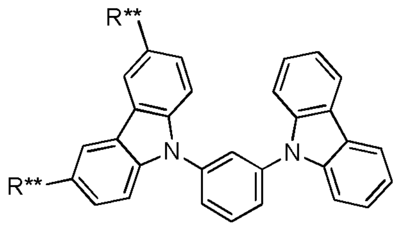

**Formel 3d**

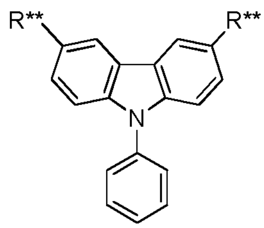

**Formel 3e**

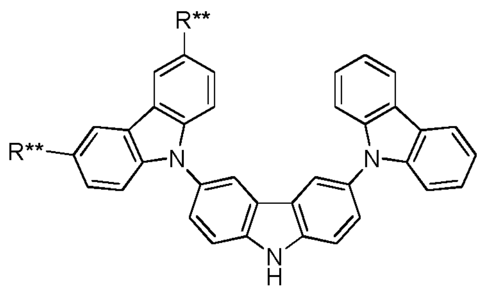

**Formel 3f**

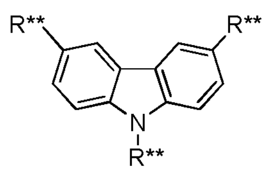

**Formel 3g**

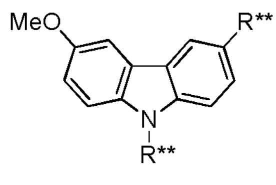

**Formel 3h**

wobei jeweils die beiden R** eine chemische Bindung an einen Rest AF1 oder eine chemische Bindung an einen Separator S sind und die Übrigen die in Anspruch 1 angegebenen Definitionen gelten.

4. Organisches Molekül nach Anspruch 1, wobei die chemischen Einheiten AF2 ausgewählt sind aus den folgenden Strukturen, wobei die kovalente Anbindung der Einheiten AF2 an den Separator S und/oder an eine Einheit AF1 über je eine der mit Kleinbuchstaben bezeichneten Positionen erfolgt:

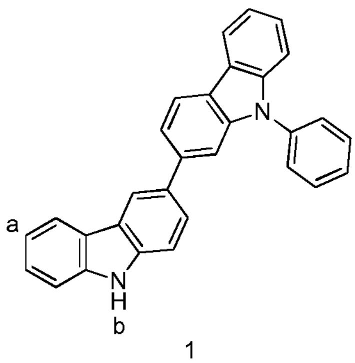

1

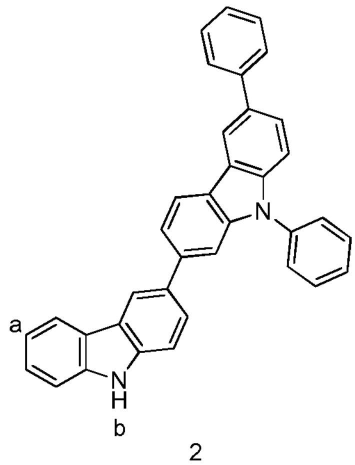

2

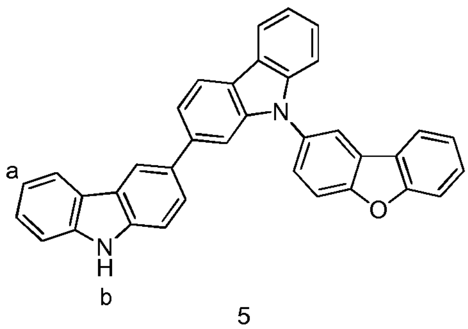

5

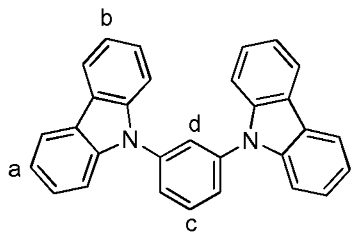

14

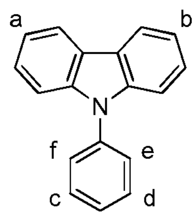

55

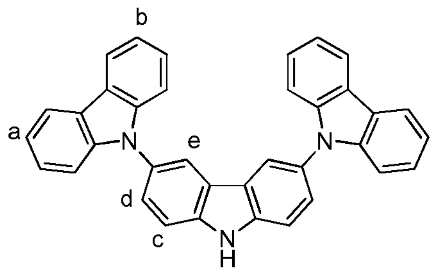

74

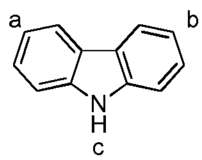

328

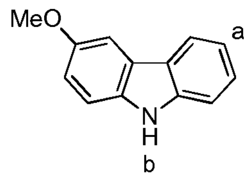

436

5. Organisches Molekül nach einem oder mehreren der Ansprüche 1 bis 4, wobei der Separator S ausgewählt ist aus den folgenden Strukturen, wobei # die Anknüpfungspositionen für die chemischen Einheiten AF1 und AF2 kennzeichnet:

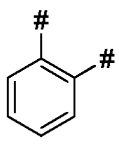
S-7

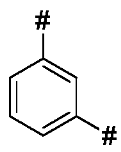
S-8

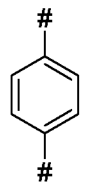
S-9

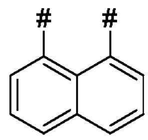
S-10

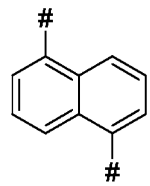
S-11

**6.** Organisches Molekül nach einem oder mehreren der Ansprüche 1 bis 4, wobei kein Separator S enthalten ist und wobei sich optional an mindestens einer der kovalenten Bindung zwischen AF1 und AF2 benachbarten Position ein Rest R**** befindet, wobei gilt: R**** ist entweder ein Rest AF1, oder ein Rest R*, wobei für R* wie in Anspruch 1 definiert ist; wobei die Reste R* insbesondere gleich Methyl, Ethyl, *iso*-Propyl, *tert*-Butyl oder Phenyl sind.

**7.** Organisches Molekül nach einem oder mehreren der Ansprüche 1 bis 6, wobei
das organische Molekül an mindestens einer chemisch substituierbaren Position mindestens einen weiteren Rest R, insbesondere zur Steigerung der Löslichkeit und/oder der Polymerisierbarkeit, aufweist, wobei gilt:
R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R_3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, - $P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden; wobei $R^2$ bis $R^9$ definiert sind wie in Anspruch 1;
und wobei für polymerisierbare Reste gilt:
polymerisierbare Reste sind solche Reste, die polymerisierbare funktionelle Einheiten tragen, die mit sich selbst homopolymerisiert oder mit anderen Monomeren copolymiersiert werden können, wodurch das organische Molekül als Polymer mit Wiederholungseinheiten nach den Formeln 17 und/oder 18 erhalten werden

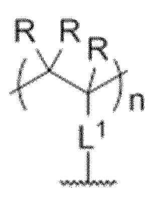
**Formel 17**

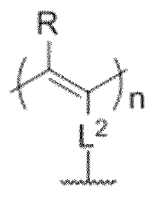
**Formel 18**

mit

gewellte Linie = Position, über die die Linkergruppe $L^1$ oder $L^2$ an das organische Molekül gebunden ist;
$L^1$ und $L^2$ = gleiche oder verschiedene Linkergruppen, aufweisend zwischen 0 bis 20 Kohlenstoffatome; oder aufweisend eine Form -C(=O)O;
und wobei insbesondere $L^1$ und $L^2$ = $-X-L^3$ mit X = O oder S;
$L^3$ = eine weitere Linkergruppe ausgewählt aus der Gruppe aus einer substituierten und unsubstituierten Alkylengruppe (linear, verzweigt oder cyclisch) und einer substituierten und unsubstituierten Arylengruppe, wobei

auch Kombinationen möglich sind.

8. Verwendung eines organischen Moleküls nach einem oder mehreren der Ansprüche 1 bis 7 als lumineszierender Emitter und/oder als Hostmaterial und/oder als Elektronentransportmaterial und/oder als Lochinjektionsmaterial und/oder als Lochblockiermaterial in einem optoelektronischen Bauelement,
wobei das optoelektronische Bauelement insbesondere ausgewählt ist aus der Gruppe bestehend aus:

 - organischen lichtemittierenden Dioden (OLEDs),
 - lichtemittierenden elektrochemischen Zellen,
 - OLED-Sensoren, insbesondere in nicht hermetisch nach außen abgeschirmten Gas- und Dampf-Sensoren,
 - organischen Dioden,
 - organischen Solarzellen,
 - organischen Transistoren,
 - organischen Feldeffekttransistoren,
 - organischen Lasern und
 - Down-Konversions-Elementen.

9. Verwendung nach Anspruch 8, wobei die Konzentration des organischen Moleküls als Emitter in optischen Licht emittierenden Bauelementen, insbesondere in OLEDs, zwischen 5 % und 80 % beträgt.

10. Optoelektronisches Bauelement, aufweisend ein organisches Molekül nach einem oder mehreren der Ansprüche 1 bis 7, insbesondere ausgeformt als ein Bauelement ausgewählt aus der Gruppe bestehend aus organische lichtemittierende Diode (OLED), lichtemittierende elektrochemische Zelle, OLED-Sensor, insbesondere nicht hermetisch nach außen abgeschirmten Gas- und Dampf-Sensoren, organische Diode, organische Solarzelle, organischer Transistor, organischer Feldeffekttransistor, organischer Laser und Down-Konversion-Element.

11. Optoelektronisches Bauelement nach Anspruch 10, aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein organisches Molekül nach einem oder mehreren der Ansprüche 1 bis 7enthält.

12. Optoelektronisches Bauelement nach Anspruch 10 oder 11, in dem die lichtemittierende Schicht mindestens ein Hostmaterial aufweist, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls nach einem oder mehreren der Ansprüche 1 bis 7.

13. Optoelektronisches Bauelement nach Anspruch 10 bis 12, wobei mindestens ein Hostmaterial aus einem organischen Molekül gemäß einem oder mehreren der Ansprüche 1 bis 7 besteht.

14. Optoelektronisches Bauelement nach Anspruch 10 bis 13, wobei die lichtemittierende Schicht ein fluoreszentes oder phosphoreszentes Material aufweist und wobei das Material der lichtemittierenden Schicht insbesondere ausgewählt ist aus organischen Molekülen gemäß einem oder mehreren der Ansprüche 1 bis 7.

15. Optoelektronisches Bauelement nach Anspruch 10 bis 14, in dem ein organisches Molekül nach einem oder mehreren der Ansprüche 1 bis 7 als Ladungstransportschicht verwendet wird.

16. Verfahren zur Herstellung eines optoelektronischen Bauelements, wobei ein organisches Molekül nach einem oder mehreren der Ansprüche 1 bis 7 verwendet wird, insbesondere wobei die Verarbeitung des organischen Moleküls mittels eines Vakuumverdampfungsverfahrens oder aus einer Lösung erfolgt.

## Claims

1. Organic molecule where:

   the overall molecule contains at least two chemical units AF1 and one chemical unit AF2 different therefrom;
   AF1 is a first chemical unit, having a conjugated system, wherein AF1 includes a structure as shown in subformula 1.1 or has a structure as shown in subformula 1.1

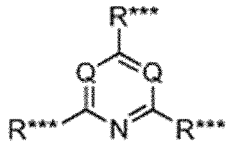

Subformula 1.1

where:
Q is N, CR***, wherein two directly neighbouring units Q are not both N;
and where:

R*** in each instance is independently either a radical R*, or a chemical bond to a separator S, or a chemical bond to a residue AF2, wherein exactly one R*** is a chemical bond to a separator S, or a chemical bond to a residue AF2;
AF2 is a second chemical unit, having a conjugated system, wherein AF2 is described by the general formula shown in subformula 1;

separator (S) is a chemical unit that links AF1 and AF2 and stops their electronic communication with one other through conjugated bonds, wherein optionally there is no separator S present in the molecule;

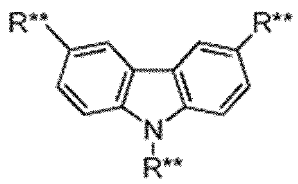

**Subformula 1**

and where in subformula 1:
R** in each instance is independently either a radical R*, or a chemical bond to a separator S, or a chemical bond to a residue AF1; wherein at least two R** are a chemical bond to a residue AF1 or a chemical bond to a separator S,
wherein the link to the chemical unit AF1 may also be via an atom of the radical R*;
where:

R* in each instance is independently selected from the group consisting of H, deuterium, phenyl, naphthyl, $N(R^2)_2$, -CN, -NC, -SCN, $-CF_3$, $-NO_2$, C(=O)OH, C(=O)$OR^3$, C(=O)$N(R^3)_2$, C(=O)$SR^3$, C(=S)$SR^3$, $Si(R^4)_3$, $B(OR^5)_2$, $B(N(R^6)_2)_2$, C(=O)$R_3$, P(=O)$(R^7)_2$, As(=O)$(R^7)_2$, P(=S)$(R^7)_2$, As(=S)$(R^7)_2$, S(=O)$R^3$, S=$NR^3$, S(=O)$NR^3$, $S(=O)_2NR^3$, $S(=O)_2R^3$, O-$S(=O)_2R^3$, $SF_5$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by -$R^9C$=$CR^9$- or -C=C-, or one neighbouring $CH_2$ group by -$Si(R^4)_2$-, -$Ge(R^4)_2$-, -$Sn(R^4)_2$, -C(=O)-, -C(=S)-, - C(=Se)-, -C=N-, -C(=O)O-, -C(=O)N($R^3$)-, -P(=O)($R^7$)-, -As(=O)($R^7$)-, -P(=S)($R^7$)-, -As(=S)($R^7$)-, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^2$, or an aryloxy or heteroaryloxy group which has 5 to 60 aromatic ring atoms and may be substituted with one or more radicals $R^9$, or a diarylamino group, diheteroarylamino group or arylheteroarylamino group which has 10 to 40 aromatic ring atoms and may be substituted with one or more radicals $R^9$, or a combination of said systems; it is also possible for two or more of said substituents R* together to form a monocyclic aliphatic ring system having a total of five or six ring members;
$R^2$ in each instance is independently selected from the group consisting of H, deuterium, phenyl, naphthyl, $CF_3$, C(=O)$OR^3$, C(=O)N($R^2$), $Si(R^4)_3$, C(=O)$R_3$, P(=O)$(R^7)_2$, As(=O)$(R^7)_2$, P(=S)$(R^7)_2$, As(=S)$(R^7)_2$, S(=O)$R^3$, $S(=O)_2R^3$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl,

alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or $-C\equiv C-$, or one neighbouring $CH_2$ group by $-Si(R^4)_2-$, $-Ge(R^4)_2-$, $-Sn(R^4)_2$, -C(=O)-, -C(=S)-, -C(=Se)-, -C=N-, -C(=O)O-, - C(=O)N($R^3$)-, -P(=O)($R^7$)-, -As(=O)($R^7$)-, -P(=S)($R^7$)-, -As(=S)($R^7$)-, -S(=O)-, $-S(=O)_2-$, $-NR^2-$, -O- or - S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^9$, or an aryloxy or heteroaryloxy group which has 5 to 60 aromatic ring atoms and may be substituted with one or more radicals $R^9$, or a diarylamino group, diheteroarylamino group or arylheteroarylamino group which has 10 to 40 aromatic ring atoms and may be substituted with one or more radicals $R^9$, or a combination of said systems; it is also possible for two or more of said substituents $R^2$ together to form a mono- or polycyclic, aliphatic, aromatic and/or benzofused ring system;

$R^3$ in each instance is independently selected from the group consisting of H, deuterium, phenyl, naphthyl, $CF_3$ or an aliphatic, aromatic and/or heteroaromatic hydrocarbon residue having 1 to 20 carbon atoms in which one or more hydrogen atoms may also be replaced by F or $CF_3$; it is also possible for two or more substituents $R^3$ together to form a mono- or polycyclic aliphatic ring system;

$R^4$ in each instance is independently selected from the group consisting of H, deuterium, phenyl, naphthyl, $N(R^2)_2$, CN, $CF_3$, OH, C(=O)$OR^3$, C(=O)$N(R^3)_2$, C(=O)$R_3$, P(=O)$(R^7)_2$, As(=O)$(R^7)_2$, P(=S)$(R^7)_2$, As(=S)$(R^7)_2$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or $-C\equiv C-$, or one neighbouring $CH_2$ group by $-Si(R^4)_2-$, $-Ge(R^4)_2-$, $-Sn(R^4)_2$, -C(=O)-, -C(=S)-, -C(=Se)-, -C=N-, -C(=O)O-, -C(=O)N($R^3$)-, -P(=O)($R^7$)-, - As(=O)($R^7$)-, -P(=S)($R^7$)-, -As(=S)($R^7$)-, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^8$, or an aryloxy or heteroaryloxy group which has 5 to 60 aromatic ring atoms may be substituted with one or more radicals $R^9$, or a diarylamino group, diheteroarylamino group or arylheteroarylamino group which has 10 to 40 aromatic ring atoms and may be substituted with one or more radicals $R^9$, or a combination of said systems; it is also possible for two or more of said substituents $R^4$ together to form a mono- or polycyclic, aliphatic, aromatic and/or benzofused ring system;

$R^5$ in each instance is independently selected from the group consisting of phenyl, naphthyl, $CF_3$, C(=O)$R_3$, P(=O)$(R^7)_2$, As(=O)$(R^7)_2$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or $-C\equiv C-$, or one neighbouring $CH_2$ group by $-Si(R^4)_2-$, $-Ge(R^4)_2-$, $-Sn(R^4)_2$, -C(=O)-, -C(=S)-, -C(=Se)-, -C=N-, -C(=O)O-, -C(=O)N($R^3$)-, -P(=O)($R^7$)-, -As(=O)($R^7$)-, -P(=S)($R^7$)-, -As(=S)($R^7$)-, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^9$, or an aryloxy or heteroaryloxy group which has 5 to 60 aromatic ring atoms and may be substituted with one or more radicals $R^9$, or a diarylamino group, diheteroarylamino group or arylheteroarylamino group which has 10 to 40 aromatic ring atoms and may be substituted with one or more radicals $R^9$, or a combination of said systems; it is also possible for two or more of said substituents $R^5$ together to form a mono- or polycyclic, aliphatic, aromatic and/or benzofused ring system;

$R^6$ in each instance is independently selected from the group consisting of phenyl, naphthyl, $CF_3$, $Si(R^4)_3$, C(=O)$R_3$, P(=O)$(R^7)_2$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or $-C\equiv C-$, or one neighbouring $CH_2$ group by $-Si(R^4)_2-$, $-Ge(R^4)_2-$, $-Sn(R^4)_2$, -C(=O)-, -C(=S)-, -C(=Se)-, -C=N-, -C(=O)O-, -C(=O)N($R^3$)-, -P(=O)($R^7$)-, -As(=O)($R^7$)-, -P(=S)($R^7$)-, -As(=S)($R^7$)-, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^9$, or an aryloxy or heteroaryloxy group which has 5 to 60 aromatic ring atoms and may be substituted with one or more radicals R*, or a diarylamino group, diheteroarylamino group or arylheteroarylamino group which has 10 to 40 aromatic ring atoms and may be

substituted with one or more radicals $R^9$, or a combination of said systems; it is also possible for two or more of said substituents $R^6$ together to form a mono- or polycyclic, aliphatic, aromatic and/or benzofused ring system;

$R^7$ in each instance is independently selected from the group consisting of phenyl, naphthyl, $N(R^2)_2$, CN, $CF_3$, $C(=O)R_3$, $C(=O)N(R^3)_2$, $Si(R^4)_3$, $C(=O)R_3$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or -C≡C-, or one neighbouring $CH_2$ group by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, - $NR^2$-, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^9$, or an aryloxy or heteroaryloxy group which has 5 to 60 aromatic ring atoms and may be substituted with one or more radicals $R^9$, or a diarylamino group, diheteroarylamino group or arylheteroarylamino group which has 10 to 40 aromatic ring atoms and may be substituted with one or more radicals $R^3$, or a combination of said systems; it is also possible for two or more of said substituents $R^7$ together to form a mono- or polycyclic, aliphatic, aromatic and/or benzofused ring system;

$R^8$ in each instance is independently selected from the group consisting of H, deuterium, phenyl, naphthyl, F, $CF_3$ or an aliphatic, aromatic and/or heteroaromatic hydrocarbon residue having 1 to 20 carbon atoms in which one or more hydrogen atoms may also be replaced by F or $CF_3$; it is also possible for two or more substituents $R^8$ together to form a mono- or polycyclic aliphatic ring system;

$R^9$ in each instance is independently selected from the group consisting of H, deuterium, phenyl, naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R_3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^8$, wherein one or more non-neighbouring $CH_2$ groups may be replaced by $-R^3C=CR^3-$ or -C≡C-, or one neighbouring $CH_2$ group by $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, - $P(=S)(R^7)-$, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^8$, or an aryloxy or heteroaryloxy group which has 5 to 60 aromatic ring atoms and may be substituted with one or more radicals $R^3$, or a diarylamino group, diheteroarylamino group or arylheteroarylamino group which has 10 to 40 aromatic ring atoms and may be substituted with one or more radicals $R^8$, or a combination of said systems; it is also possible for two or more of said substituents $R^9$ together to form a mono- or polycyclic, aliphatic, aromatic and/or benzofused ring system.

**2.** Organic molecule according to Claim 1, wherein AF2 has a structure of formula 2a-2d, in which the two R** are each a chemical bond to a residue AF1 or a chemical bond to a separator S, and the definitions stated in Claim 1 otherwise apply.

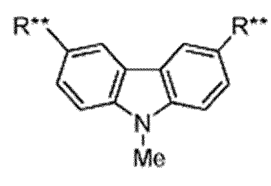
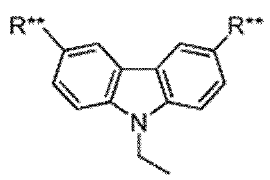
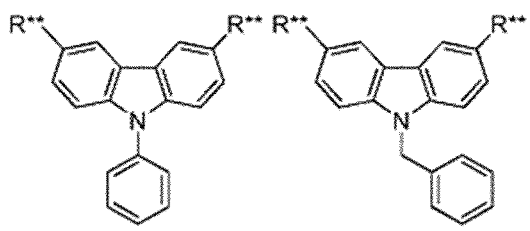

Formula 2a Formula 2b Formula 2c Formula 2d

**3.** Organic molecule according to Claim 1, wherein AF2 has a structure of formula 3a-3h,

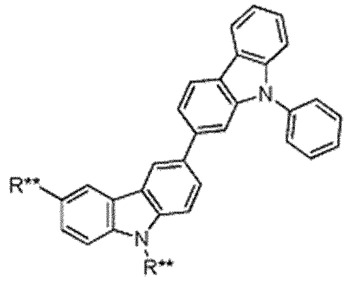

Formula 3a

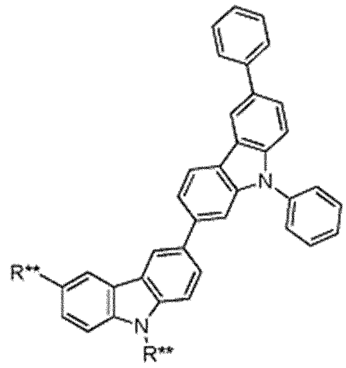

Formula 3b

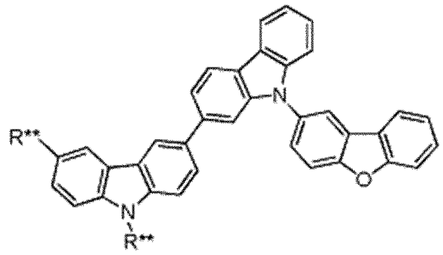

Formula 3c

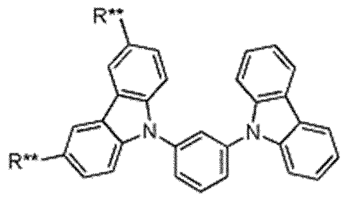

Formula 3d

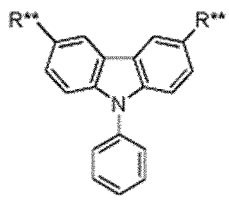

Formula 3e

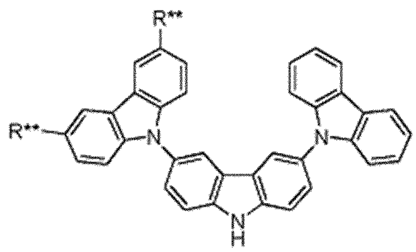

Formula 3f

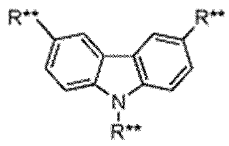

Formula 3g

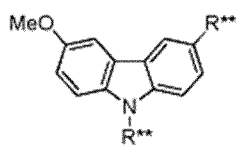

Formula 3h

in which the two R** are each a chemical bond to a residue AF1 or a chemical bond to a separator S, and the definitions stated in Claim 1 otherwise apply.

4. Organic molecule according to Claim 1, wherein the chemical units AF2 are selected from the following structures, in which the covalent bond linking the units AF2 to the separator S and/or to a unit AF1 is in each case through one of the positions marked with a lower-case letter:

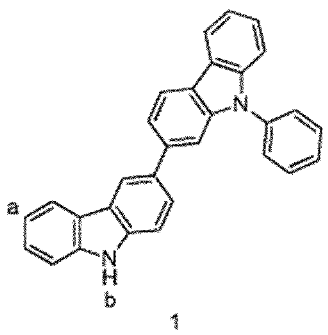

1

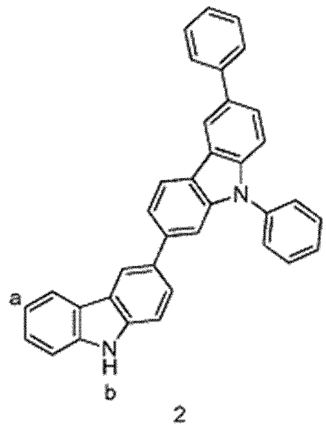

2

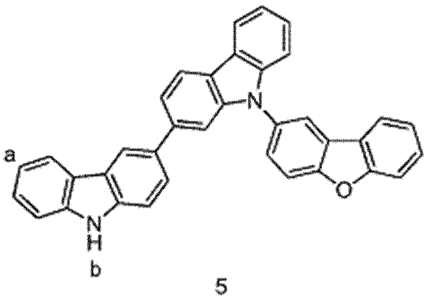
5

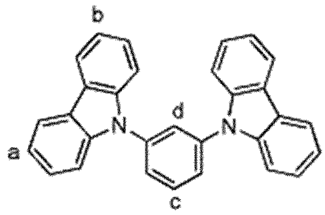
14

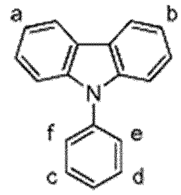
55

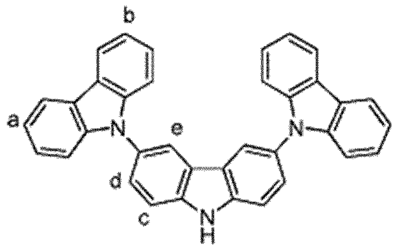
74

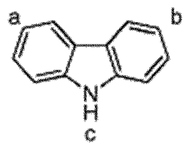
328

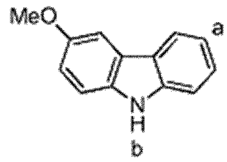
436

**5.** Organic molecule according to one or more of Claims 1 to 4, in which the separator S is selected from the structures below, where # indicates the position of attachment of the chemical units AF1 and AF2.

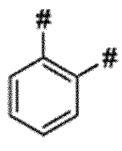
S-7

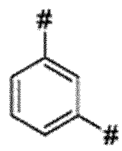
S-8

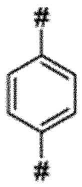
S-9

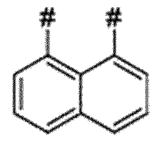
S-10

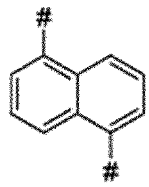
S-11

**6.** Organic molecule according to one or more of Claims 1 to 4, wherein there is no separator S present and wherein optionally a radical R**** is present in at least one position adjacent to the covalent bond between AF1 and AF2, where: R**** is either a residue AF1 or a radical R*, where R* is as defined in Claim 1, wherein the radicals R* are in particular methyl, ethyl, isopropyl, tert-butyl or phenyl.

**7.** Organic molecule according to one or more of Claims 1 to 6, wherein the organic molecule has at least one further radical R in at least one chemically substitutable position, in particular for increasing the solubility and/or polymerizability, wherein:

R in each instance is independently selected from the group consisting of H, deuterium, phenyl, naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R_3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, a straight-chain alkyl, alkoxy or thioalkoxy group having 1 to 40 carbon atoms or a straight-chain alkenyl or alkynyl group having 2 to 40 carbon atoms or a branched or cyclic alkyl, alkenyl, alkynyl, alkoxy or thioalkoxy group having 3 to 40 carbon atoms, which may each be substituted with one or more radicals $R^9$, wherein one or more neighbouring $CH_2$ groups may be replaced by $-R^9C=CR^9-$ or -C≡C-, or one neighbouring $CH_2$ group by $-Si(R^4)_2-$, $-Ge(R^4)_2-$, - $Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O- or -S- and wherein one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted with one or more radicals $R^2$, or an aryloxy or heteroaryloxy group which has 5 to 60 aromatic ring atoms and may be substituted with one or more radicals $R^9$, or a diarylamino group, diheteroarylamino group or arylheteroarylamino group which has 10 to 40 aromatic ring atoms and may be

substituted with one or more radicals $R^9$, or a combination of said systems; it is also possible for two or more of said substituents R together to form a monocyclic aliphatic ring system having a total of five or six ring members; wherein $R^2$ to $R^9$ are defined as in Claim 1;
and wherein polymerizable residues are defined thus:
polymerizable residues are residues bearing polymerizable functional units that are able to undergo self-homopolymerization or copolymerization with other monomers, resulting in a polymer that is an organic molecule with repeat units as shown in formulas 17 and/or 18

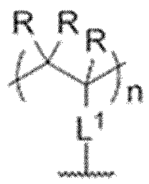
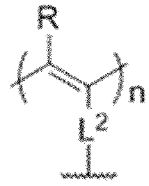

**Formula 17** **Formula 18**

where

> wavy line = position through which the linker group $L^1$ or $L^2$ is attached to the organic molecule;
> $L^1$ and $L^2$ = identical or different linker groups having between 0 and 20 carbon atoms; or having the form -C(=O)O;
> and where in particular $L^1$ and $L^2$ = -X-$L^3$ with X = O or S;
> $L^3$ = a further linker group selected from the group of a substituted and unsubstituted alkylene group (straight-chain, branched or cyclic) and a substituted and unsubstituted arylene group, with combinations also being possible.

**8.** Use of an organic molecule according to one or more of Claims 1 to 7 as a luminescent emitter and/or as a host material and/or as an electron-transport material and/or as a hole-injection material and/or as a hole-blocking material in an optoelectronic component, wherein the optoelectronic component is selected in particular from the group consisting of:

- organic light-emitting diodes (OLEDs),
- light-emitting electrochemical cells,
- OLED sensors, particularly in gas and vapour sensors that are not hermetically shielded from the outside environment,
- organic diodes,
- organic solar cells,
- organic transistors,
- organic field-effect transistors,
- organic lasers and
- down-conversion elements.

**9.** Use according to Claim 8, wherein the concentration of the organic molecule as an emitter in optical light-emitting components, particularly in OLEDs, is between 5% and 80%.

**10.** Optoelectronic component containing an organic molecule according to one or more of Claims 1 to 7, particularly one designed as a component selected from the group consisting of organic light-emitting diode (OLEDs), light-emitting electrochemical cell, OLED sensor, particularly gas and vapour sensors that are not hermetically shielded from the outside environment, organic diode, organic solar cell, organic transistor,
organic field-effect transistor, organic laser and down-conversion element.

**11.** Optoelectronic component according to Claim 10, having a substrate, an anode and a cathode, wherein the anode and the cathode have been applied to the substrate, and at least one light-emitting layer which is positioned between the anode and cathode and which contains an organic molecule according to one or more of Claims 1 to 7.

**12.** Optoelectronic component according to Claims 10 or 11, in which the light-emitting layer includes at least one host material, wherein the singlet excited state ($S_1$) and/or triplet excited state ($T_1$) of the at least one host material is

higher than the singlet excited state ($S_1$) and/or triplet excited state ($T_1$) of the organic molecule according to one or more of Claims 1 to 7.

**13.** Optoelectronic component according to Claims 10 to 12, wherein at least one host material consists of an organic molecule according to one or more of Claims 1 to 7.

**14.** Optoelectronic component according to Claims 10 to 13, wherein the light-emitting layer includes a fluorescent or phosphorescent material and wherein the material of the light-emitting layer is selected in particular from organic molecules according to one or more of Claims 1 to 7.

**15.** Optoelectronic component according to Claims 10 to 14, in which an organic molecule according to one or more of Claims 1 to 7 is used as the charge-transport layer.

**16.** Method for producing an optoelectronic component, wherein an organic molecule according to one or more of Claims 1 to 7 is used, particularly wherein the processing of the organic molecule is by means of a vacuum-evaporation process or from a solution.

**Revendications**

**1.** Molécule organique, dans laquelle :

au moins deux unités chimiques AF1 et une unité chimique AF2 différente de celles-ci sont contenues dans la molécule totale ;
AF1 est une première unité chimique, comprenant un système conjugué, AF1 comprenant une structure de la sous-formule 1.1 ou ayant une structure de la sous-formule 1.1 :

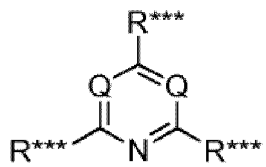

Sous-formule 1.1

dans laquelle

Q représente N, CR***, deux unités Q directement voisines ne représentant pas simultanément N ;
et
les R*** représentent à chaque occurrence indépendamment les uns des autres un radical R*, ou une liaison chimique à un séparateur S, ou une liaison chimique à un radical AF2, exactement un R*** représentant une liaison chimique à un séparateur S ou une liaison chimique à un radical AF2 ;

AF2 est une deuxième unité chimique, comprenant un système conjugué, AF2 étant décrite par la formule générale représentée dans la sous-formule 1 ;
le séparateur (S) est une unité chimique qui relie AF1 et AF2, et interrompt leur communication électronique l'un avec l'autre par des liaisons conjuguées ; aucun séparateur S n'étant éventuellement contenu dans la molécule organique ;

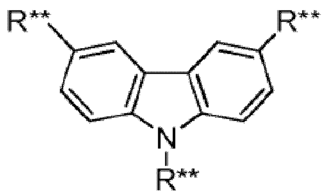

Sous-formule 1

et dans la sous-formule 1 :

les R** représentent à chaque occurrence indépendamment les uns des autres soit un radical R*, soit une liaison chimique à un séparateur S, soit une liaison chimique à un radical AF1, au moins deux R** représentant une liaison chimique à un radical AF1 ou une liaison chimique à un séparateur S,
la liaison à l'unité chimique AF1 pouvant également avoir lieu par le biais d'un atome du radical R* ;

avec :

les R* étant choisis à chaque occurrence indépendamment les uns des autres dans le groupe constitué par H, deutérium, phényle, naphtyle, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R_3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $OS(=O)_2R^3$, $SF_5$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par - $Si(R^4)_2$-, $-Ge(R^4)_2-$, $-Sn(R^4)_2$, -C(=O)-, -C(=S)-, -C(=Se)-, -C=N-, -C(=O)O-, $-C(=O)N(R^3)-$, - $P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué par un ou plusieurs radicaux $R^2$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants R* pouvant également former les uns avec les autres un système cyclique aliphatique monocyclique contenant au total cinq ou six chaînons ;
les $R^2$ étant choisis à chaque occurrence indépendamment les uns des autres dans le groupe constitué par H, deutérium, phényle, naphtyle, $-CF_3$, $C(=O)OR^3$, $C(=O)N(R^2)_2$, $Si(R^4)_3$, $C(=O)R_3$, $P(=O)(R^7)_2$, $As(=O)(R^7)_2$, $P(=S)(R^7)_2$, $As(=S)(R^7)_2$, $S(=O)R^3$, $S(=O)_2R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2$, -C(=O)-, -C(=S)-, - C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, - S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué par un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^2$ pouvant également former les uns avec les autres un système cyclique mono- ou polycyclique, aliphatique, aromatique et/ou benzoannelé ;
les $R^3$ étant choisis à chaque occurrence indépendamment les uns des autres dans le groupe constitué par H, deutérium, phényle, naphtyle, $CF_3$ ou un radical hydrocarboné aliphatique, aromatique et/ou hétéroaromatique de 1 à 20 atomes C, dans lequel un ou plusieurs atomes H peuvent également être remplacés

par F ou $CF_3$; deux substituants $R^3$ ou plus pouvant également former les uns avec les autres un système cyclique aliphatique mono- ou polycyclique ;

les $R^4$ étant choisis à chaque occurrence indépendamment les uns des autres dans le groupe constitué par H, deutérium, phényle, naphtyle, $N(R^2)_2$, CN, $CF_3$, OH, $C(=O)OR^3$, $C(=O)N(R^3)_2$, $C(=O)R_3$, $P(=O)(R^7)_2$, $As(=O)(R^7)_2$, $P(=S)(R^7)_2$, $As(=S)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2$, -C(=O)-, -C(=S)-, - C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, - S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^8$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^4$ pouvant également former les uns avec les autres un système cyclique mono- ou polycyclique, aliphatique, aromatique et/ou benzoannelé ;

les $R^5$ étant choisis à chaque occurrence indépendamment les uns des autres dans le groupe constitué par phényle, naphtyle, $CF_3$, $C(=O)R_3$, $P(=O)(R^7)_2$, $As(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, - C≡C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, - $As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^5$ pouvant également former les uns avec les autres un système cyclique mono- ou polycyclique, aliphatique, aromatique et/ou benzoannelé ;

les $R^6$ étant choisis à chaque occurrence indépendamment les uns des autres dans le groupe constitué par phényle, naphtyle, $CF_3$, $Si(R^4)_3$, $C(=O)R^3$, $P(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, $-Sn(R^4)_2$, -C(=O)-, -C(=S)-, - C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, - S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux R*, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^6$ pouvant également former les uns avec les autres un système cyclique mono- ou polycyclique, aliphatique, aromatique et/ou benzoannelé ;

les $R^7$ étant choisis à chaque occurrence indépendamment les uns des autres dans le groupe constitué par phényle, naphtyle, $N(R^2)_2$, CN, $CF_3$, $C(=O)OR^3$, $C(=O)N(R^3)_2$, $Si(R^4)_3$, $C(=O)R_3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par $-Si(R^4)_2-$, $-Ge(R^4)_2-$, - $Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, - $P(=S)(R^7)$, $-As(=S)(R^7)-$, -S(=O)-, $-S(=O)_2-$, $-NR^2-$, -O- ou -S-, et un ou plusieurs atomes H

pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^3$, ou une combinaison de ces systèmes; deux ou plus de ces substituants $R^7$ pouvant également former les uns avec les autres un système cyclique mono- ou polycyclique, aliphatique, aromatique et/ou benzoannelé ;

les $R^8$ étant choisis à chaque occurrence indépendamment les uns des autres dans le groupe constitué par H, deutérium, phényle, naphtyle, F, $CF_3$ ou un radical hydrocarboné aliphatique, aromatique et/ou hétéroaromatique de 1 à 20 atomes C, dans lequel un ou plusieurs atomes H peuvent également être remplacés par F ou $CF_3$; deux substituants $R^8$ ou plus pouvant également former les uns avec les autres un système cyclique aliphatique mono- ou polycyclique ;

les $R^9$ étant choisis à chaque occurrence indépendamment les uns des autres dans le groupe constitué par H, deutérium, phényle, naphtyle, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^8$, un ou plusieurs groupes $CH_2$ voisins pouvant être remplacés par $-R^9C=CR^9-$, -C≡C-, ou un groupe $CH_2$ voisin pouvant être remplacé par - $Si(R^4)_2$-, -$Ge(R^4)_2$-, -$Sn(R^4)_2$, -C(=O)-, -C(=S)-, -C(=Se)-, -C=N-, -C(=O)O-, -$C(=O)N(R^3)$-, - $P(=O)(R^7)$-, -$As(=O)(R^7)$-, -$P(=S)(R^7)$, -$P(=S)(R^7)$-, -S(=O)-, -$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^8$, ou un groupe aryloxy ou hétéroaryloxy de 5 à 60 atomes de cycle aromatique, qui peut être substitué par un ou plusieurs radicaux $R^3$, ou un groupe diarylamino, un groupe dihétéroarylamino ou un groupe arylhétéroarylamino de 10 à 40 atomes de cycle aromatique, qui peut être substitué par un ou plusieurs radicaux $R^8$, ou une combinaison de ces systèmes; deux ou plus de ces substituants $R^9$ pouvant également former les uns avec les autres un système cyclique mono- ou polycyclique, aliphatique, aromatique et/ou benzoannelé.

**2.** Molécule organique selon la revendication 1, dans laquelle AF2 comprend une structure des formules 2a à 2d, dans lesquelles les deux R** sont à chaque fois une liaison chimique à un radical AF1 ou une liaison chimique à un séparateur S, et, pour le reste, les définitions indiquées dans la revendication 1 s'appliquent :

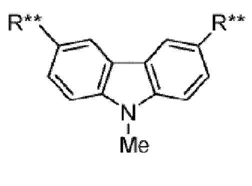
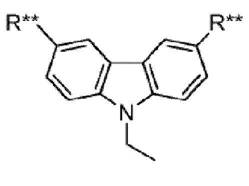
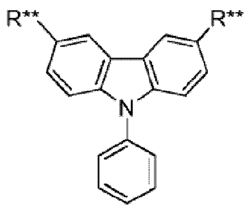
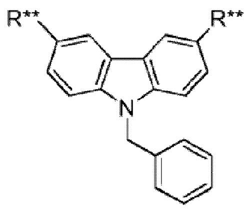

**Formule 2a** **Formule 2b** **Formule 2c** **Formule 2d**

**3.** Molécule organique selon la revendication 1, dans laquelle AF2 comprend une structure des formules 3a à 3h :

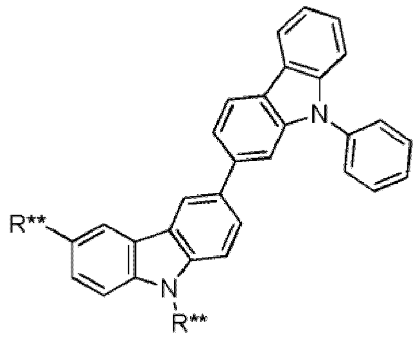

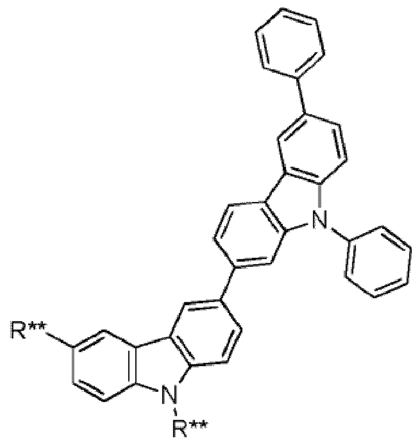

**Formule 3a** **Formule 3b**

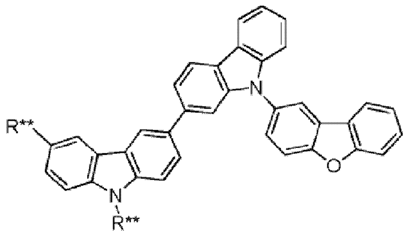

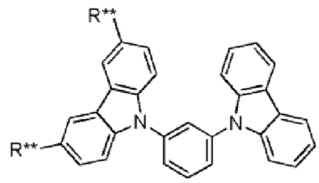

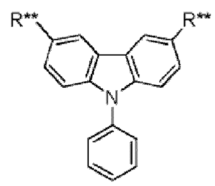

**Formule 3c** **Formule 3d** **Formule 3e**

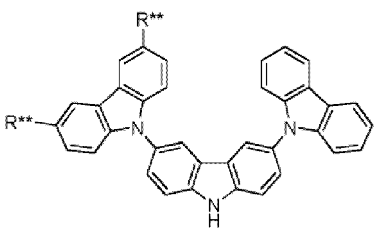

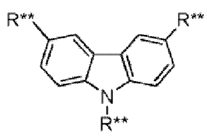

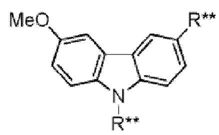

**Formule 3f** **Formule 3g** **Formule 3h**

dans lesquelles les deux R** sont à chaque fois une liaison chimique à un radical AF1 ou une liaison chimique à un séparateur S, et, pour le reste, les définitions indiquées dans la revendication 1 s'appliquent.

**4.** Molécule organique selon la revendication 1, dans laquelle
les unités chimiques AF2 sont choisies parmi les structures suivantes, la liaison covalente des unités AF2 au séparateur S et/ou à une unité AF1 ayant lieu à chaque fois par une des positions **caractérisées par** une lettre minuscule :

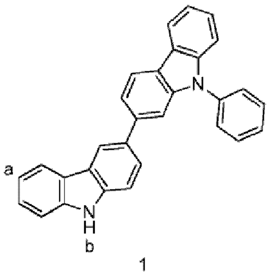
1

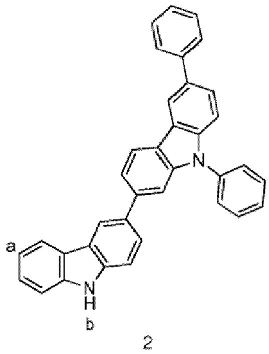
2

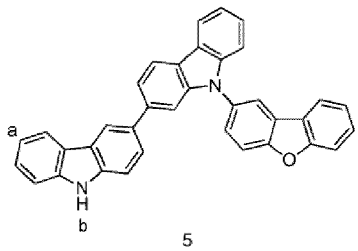
5

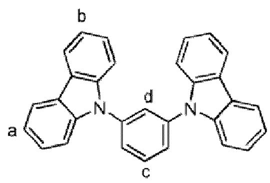
14

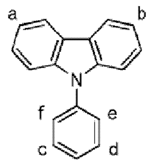
55

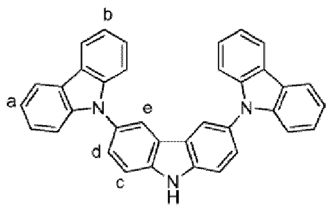
74

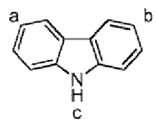
328

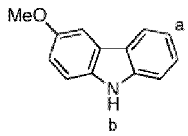
436

5. Molécule organique selon une ou plusieurs des revendications 1 à 4, dans laquelle le séparateur S est choisi parmi les structures suivantes, dans lesquelles # caractérise les positions de liaison pour les unités chimiques AF1 et AF2 :

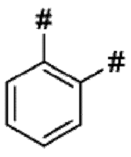
S-7

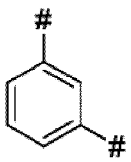
S-8

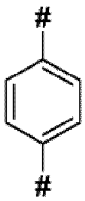
S-9

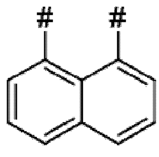
S-10

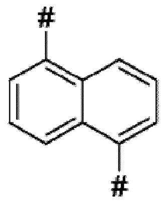
S-11

6. Molécule organique selon une ou plusieurs des revendications 1 à 4, dans laquelle aucun séparateur S n'est contenu et un radical R**** se trouve éventuellement à au moins une position voisine de la liaison covalente entre AF1 et AF2, avec : R**** est soit un radical AF1, soit un radical R*, R* étant tel que défini dans la revendication 1; les radicaux R* représentant notamment méthyle, éthyle, *iso*-propyle, *tert*-butyle ou phényle.

7. Molécule organique selon une ou plusieurs des revendications 1 à 6, dans laquelle la molécule organique comprend au moins un radical R supplémentaire à au moins une position pouvant être substituée chimiquement, notamment pour augmenter la solubilité et/ou l'aptitude à la polymérisation, avec :

les R étant choisis à chaque occurrence indépendamment les uns des autres dans le groupe constitué par H, deutérium, phényle, naphtyle, F, Cl, Br, I, $N(R^2)_2$, -CN, -NC, -SCN, $-CF_3$, $-NO_2$, - OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes

C, qui peut à chaque fois être substitué avec un ou plusieurs radicaux $R^9$, un ou plusieurs groupes $CH_2$ voisins pouvant être remplacés par $-R^9C=CR^9-$, -C≡C-, ou un groupe $CH_2$ voisin pouvant être remplacé par - $Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, - $P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué par un ou plusieurs radicaux $R^2$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes; deux ou plus de ces substituants R pouvant également former les uns avec les autres un système cyclique aliphatique monocyclique contenant au total cinq ou six chaînons; $R^2$ à $R^9$ étant tels que définis dans la revendication 1 ;

et avec, pour les radicaux polymérisables :

les radicaux polymérisables étant des radicaux qui portent des unités fonctionnelles polymérisables, qui peuvent être homopolymérisées avec elles-mêmes ou copolymérisées avec d'autres monomères, la molécule organique étant ainsi obtenue sous la forme d'un polymère contenant des unités de répétition selon les formules 17 et/ou 18 :

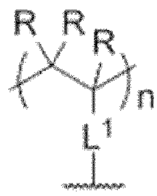

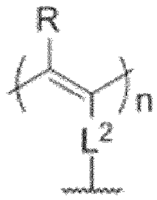

**Formule 17 Formule 18**

avec

ligne ondulée = position par laquelle le groupe de liaison $L^1$ ou $L^2$ est relié à la molécule organique ;
$L^1$ et $L^2$ = groupes de liaison identiques ou différents, comprenant 0 à 20 atomes de carbone ; ou présentant une forme -C(=O)O ;
et notamment $L^1$ et $L^2$ = $-X-L^3$ avec X = O ou S ;
$L^3$ = un groupe de liaison supplémentaire choisi dans le groupe constitué par un groupe alkylène substitué ou non substitué (linéaire, ramifié ou cyclique) et un groupe arylène substitué ou non substitué, des combinaisons étant également possibles.

**8.** Utilisation d'une molécule organique selon une ou plusieurs des revendications 1 à 7 en tant qu'émetteur luminescent et/ou en tant que matériau hôte et/ou en tant que matériau de transport d'électrons et/ou en tant que matériau d'injection de trous et/ou en tant que matériau de blocage de trous dans un composant optoélectronique, le composant optoélectronique étant notamment choisi dans le groupe constitué par :

- les diodes électroluminescentes organiques (OLED),
- les cellules électrochimiques électroluminescentes,
- les capteurs OLED, notamment dans des capteurs de gaz et de vapeur non blindés hermétiquement vers l'extérieur,
- les diodes organiques,
- les cellules solaires organiques,
- les transistors organiques,
- les transistors à effet de champ organiques,
- les lasers organiques et
- les éléments de conversion à la baisse.

**9.** Utilisation selon la revendication 8, dans laquelle la concentration de la molécule organique en tant qu'émetteur dans des composants optiques électroluminescents, notamment dans des OLED, est comprise entre 5 % et 80 %.

**10.** Composant optoélectronique, comprenant une molécule organique selon une ou plusieurs des revendications 1 à 7, notamment configuré sous la forme d'un composant choisi dans le groupe constitué par les diodes électroluminescentes organiques (OLED), les cellules électrochimiques électroluminescentes, les capteurs OLED, notamment dans des capteurs de gaz et de vapeur non blindés hermétiquement vers l'extérieur, les diodes organiques, les cellules solaires organiques, les transistors organiques, les transistors à effet de champ organiques, les lasers organiques et les éléments de conversion à la baisse.

**11.** Composant optoélectronique selon la revendication 10, comprenant un substrat, une anode et une cathode, l'anode et la cathode étant appliquées sur le substrat, et au moins une couche électroluminescente, qui est agencée entre l'anode et la cathode, et qui contient une molécule organique selon une ou plusieurs des revendications 1 à 7.

**12.** Composant optoélectronique selon la revendication 10 ou 11, dans lequel la couche électroluminescente comprend au moins un matériau hôte, l'état singulet excité ($S_1$) et/ou l'état triplet excité ($T_1$) dudit au moins un matériau hôte étant notamment plus élevés que l'état singulet excité ($S_1$) et/ou l'état triplet excité ($T_1$) de la molécule organique selon une ou plusieurs des revendications 1 à 7.

**13.** Composant optoélectronique selon les revendications 10 à 12, dans lequel au moins un matériau hôte est constitué par une molécule organique selon une ou plusieurs des revendications 1 à 7.

**14.** Composant optoélectronique selon les revendications 10 à 13, dans lequel la couche électroluminescente comprend un matériau fluorescent ou phosphorescent, et le matériau de la couche électroluminescente est notamment choisi parmi les molécules organiques selon une ou plusieurs des revendications 1 à 7.

**15.** Composant optoélectronique selon les revendications 10 à 14, dans lequel une molécule organique selon une ou plusieurs des revendications 1 à 7 est utilisée en tant que couche de transport de charges.

**16.** Procédé de fabrication d'un composant optoélectronique, dans lequel une molécule organique selon une ou plusieurs des revendications 1 à 7 est utilisée, l'usinage de la molécule organique ayant notamment lieu au moyen d'un procédé d'évaporation sous vide ou à partir d'une solution.

Figur 1

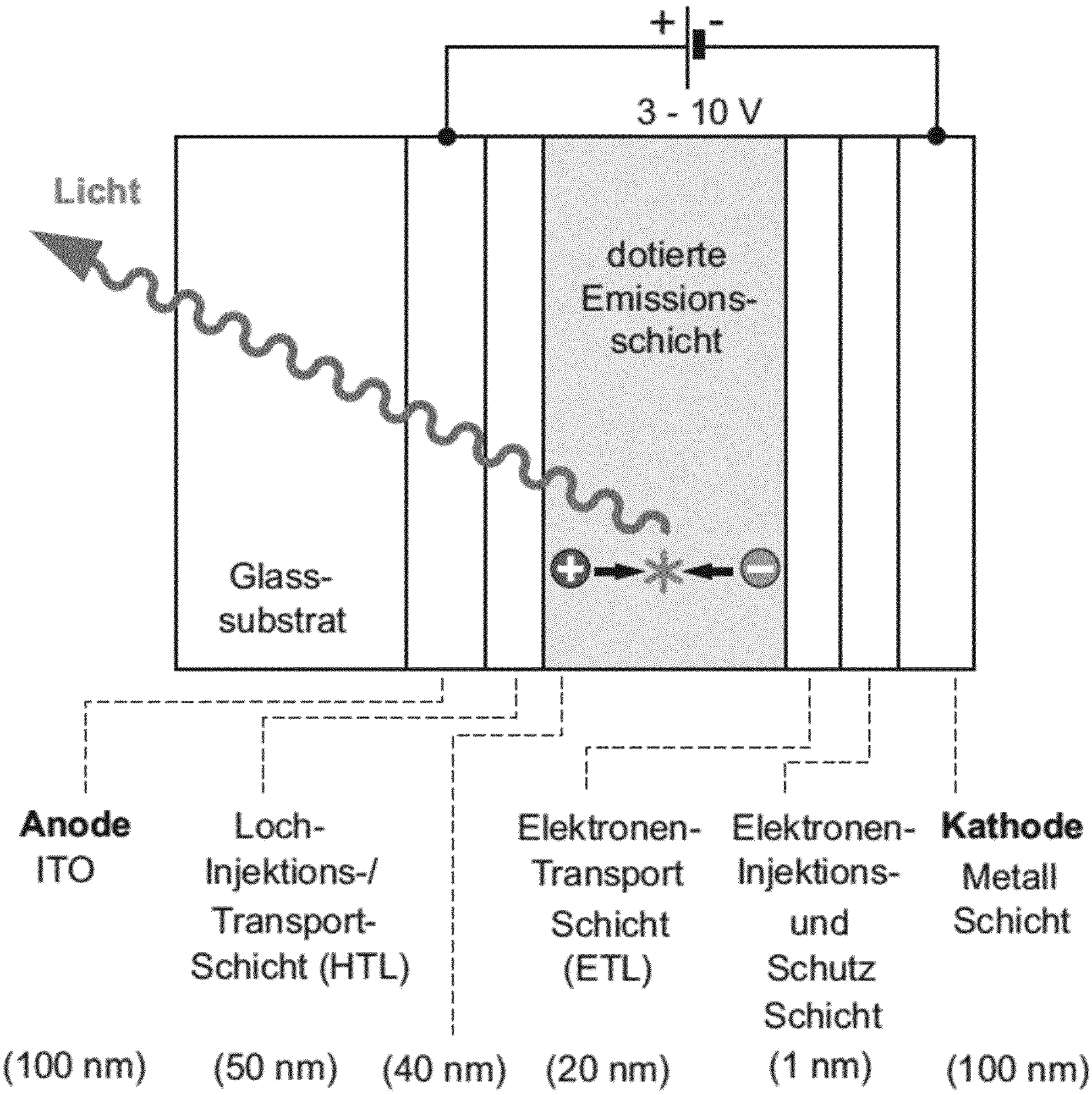
+
-
3 - 10 V
Licht
dotierte Emissions-schicht
Glass-substrat
Anode
ITO
(100 nm)
Loch-Injektions-/Transport-Schicht (HTL)
(50 nm)
(40 nm)
Elektronen-Transport Schicht (ETL)
(20 nm)
Elektronen-Injektions- und Schutz Schicht
(1 nm)
Kathode
Metall Schicht
(100 nm)

Figur 2

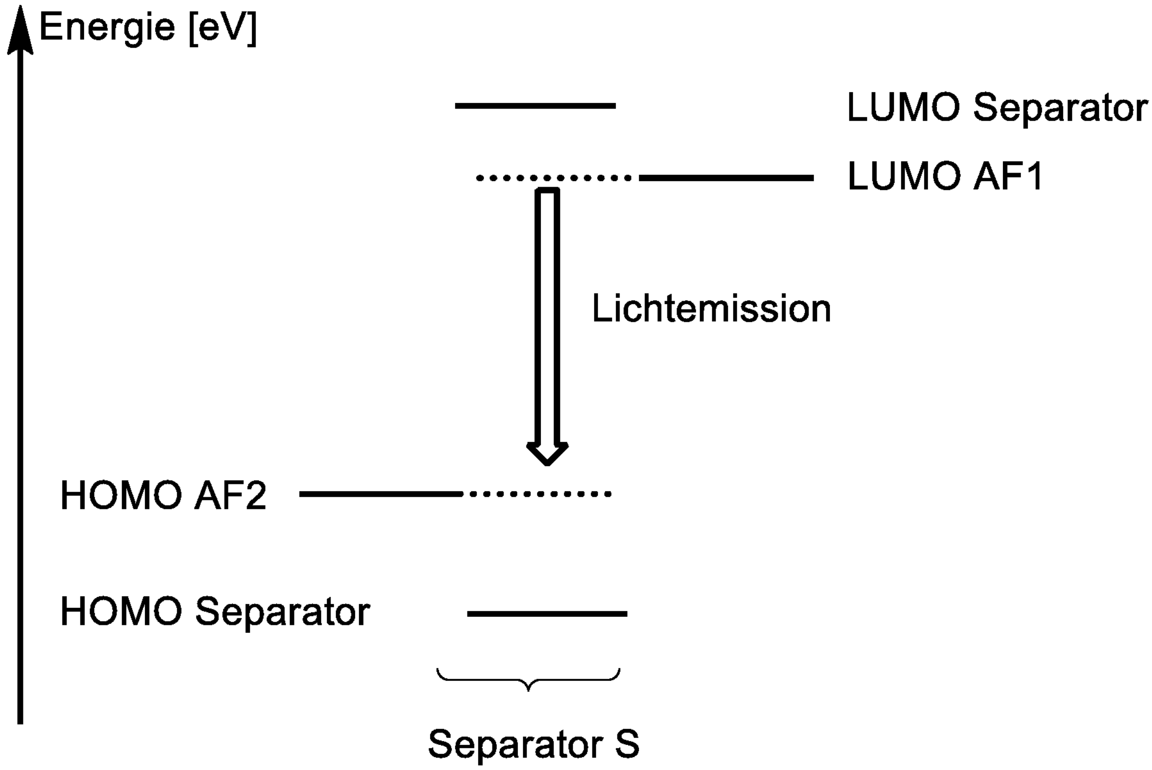


Figur3

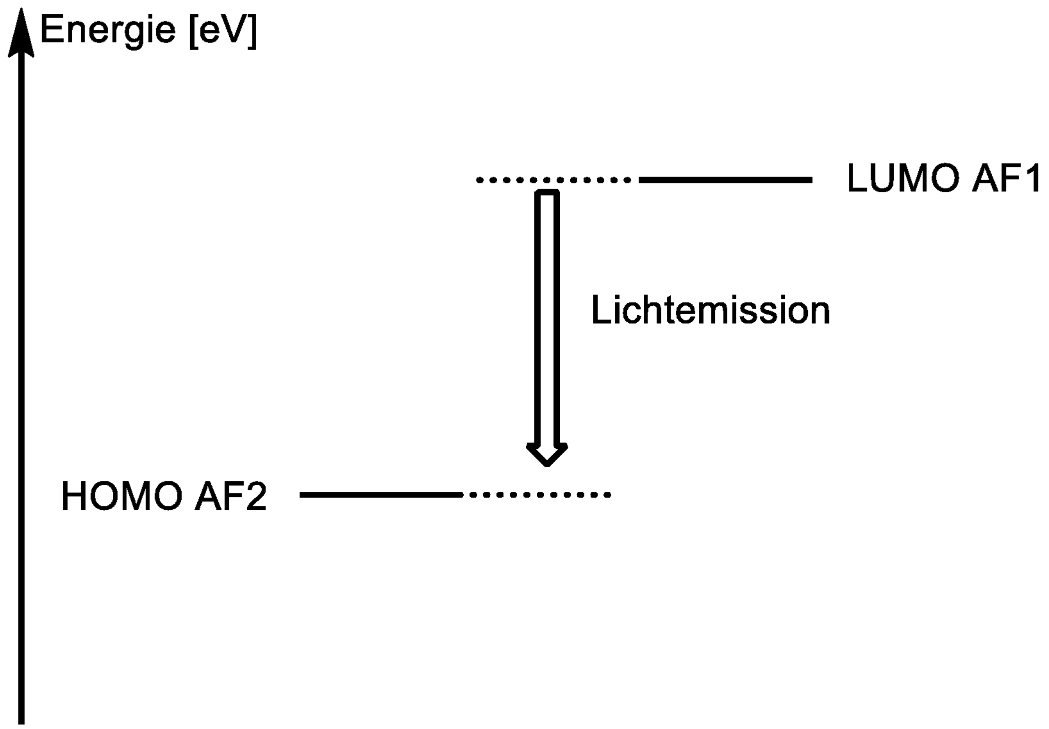

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **M. A. BALDO ; D. F. O'BRIEN ; M. E. THOMPSON ; S. R. FORREST.** *Phys. Rev. B,* 1999, vol. 60, 14422
- **KUNDU et al.** *Adv. Funct. Mater.,* 2003, vol. 13 (6), 445
- **BECKE, A. D.** *Phys. Rev.,* 1988, vol. 38, 3098-3100
- **PERDEW, J. P.** *Phys. Rev. B,* 1986, vol. 33, 8822-8827